# EUROPÄISCHE PATENTANMELDUNG

(11) **EP 0 934 935 A1**
(43) Veröffentlichungstag der Anmeldung: **11.08.1999**
(21) Anmeldenummer: 99100240.3
(22) Anmeldetag: 08.01.1999
(51) Int. Cl.: C07D 263/44, A01N 43/76, C07D 413/12, C07D 249/12

(54) **Heterocyclylsubstituierte Phenylverbindungen, Verfahren und Zwischenprodukte zu ihrer Herstellung und ihre Verwendung zur Bekämpfung von Schadpilzen und tierischen Schädlingen**

(30) Priorität: 05.02.1998 DE 19804485
(71) Anmelder: BASF AKTIENGESELLSCHAFT, 67056 Ludwigshafen (DE)
(72) Erfinder: Gypser, Andreas Dr., 68159 Mannheim (DE); Sauter, Hubert Dr., 68167 Mannheim (DE); Bayer, Herbert Dr., 68159 Mannheim (DE); Gewehr, Markus Dr., 56288 Kastellaun (DE); Grammenos, Wassilios Dr., 67063 Ludwigshafen (DE); Grote, Thomas Dr., 67105 Schifferstadt (DE); Götz, Roland Dr., 68809 Neulussheim (DE); Müller, Bernd Dr., 67227 Frankenthal (DE); Ptock, Arne Dr., 67067 Ludwigshafen (DE); Röhl, Franz Dr., 67105 Schifferstadt (DE); Harries, Volker Dr., 67227 Frankenthal (DE); Ammermann, Eberhard Dr., 64646 Heppenheim (DE); Lorenz, Gisela Dr., 67434 Neustadt (DE); Strathmann, Siegfried Dr., 67117 Limburgerhof (DE)

(57) **Zusammenfassung**

Die vorliegende Erfindung betrifft heterocyclylsubstituierte Phenylverbindungen der Formel I, 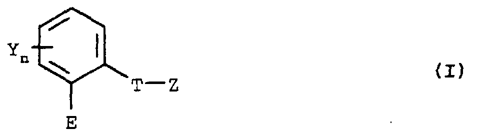 in der die Substituenten folgende Bedeutung haben:
- Y: Halogen, C₁-C₄-Alkyl, C₁-C₄-Halogenalkyl oder C₁-C₄-Alkoxy;
- n: 0, 1 oder 2, wobei die Reste Y verschieden sein können, wenn n = 2 ist;
- E: eine Gruppe A oder B, 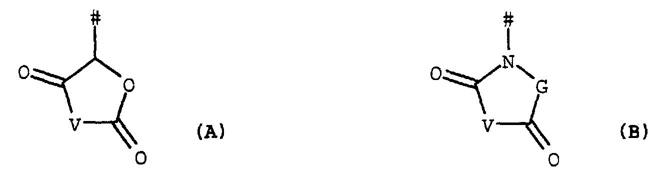 wobei # die Bindung mit dem Phenylring kennzeichnet,
und G, R^{α}, R^{β} Y, n, T und Z die in der Beschreibung genannten Bedeutungen haben.

## Beschreibung

Die vorliegende Erfindung betrifft heterocyclylsubstituierte Phenylverbindungen der Formel I, 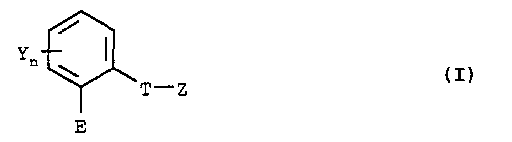 in der die Substituenten folgende Bedeutung haben:
- Y: Halogen, C₁-C₄-Alkyl, C₁-C₄-Halogenalkyl oder C₁-C₄-Alkoxy;
- n: 0, 1 oder 2, wobei die Reste Y verschieden sein können, wenn n = 2 ist;
- E: eine Gruppe A oder B, 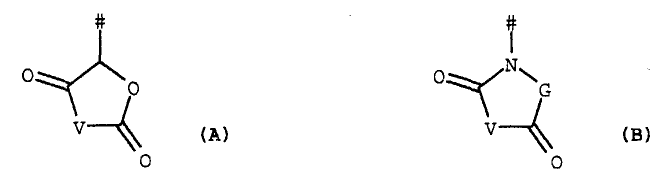 wobei # die Bindung mit dem Phenylring kennzeichnet;
- V: Sauerstoff oder N-R^{α}, und
- G: N-R^{β}, wobei
- R^{α}: C₁-C₄-Alkyl, C₁-C₄-Halogenalkyl, C₃-C₆-Cycloalkyl oder ggf. subst. Arylmethylen;
- R^{β}: Wasserstoff oder einen Rest R^{α};
- T: Sauerstoff oder Oxymethylen;
- Z: eine Gruppe X, N=CWR¹ oder N=C(R¹)-C(R²)=NOR³;
- X: ggf. subst. Heterocyclyl, ggf. subst. Aryl, ggf. subst. Hetaryl, ggf. subst. Arylmethylen oder ggf. subst. Hetarylmethylen;
- W: ggf. subst. C₁-C₆-Alkyl, ggf. subst. C₂-C₆-Alkenyl, ggf. subst. C₂-C₆-Alkinyl, ggf. subst. C₃-C₆-Cycloalkyl, ggf. subst. C₃-C₆-Cycloalkenyl, ggf. subst. Heterocyclyl, ggf. subst. Aryl oder ggf. subst. Hetaryl;
- R¹: Wasserstoff, Cyano, C₁-C₄-Alkyl, C₁-C₄-Halogenalkyl, C₁-C₄-Alkoxy, C₁-C₄-Alkoxy-C₁-C₄-alkyl, C₃-C₆-Cycloalkyl;
- R²: Wasserstoff, Cyano, Halogen, C(R^{d})=NOR³ oder W, OW, SW oder NR^{c}W, wobei
- R^{c}: Wasserstoff, C₁-C₆-Alkyl, C₂-C₆-Alkenyl oder C₂-C₆-Alkinyl und
- R^{d}: Wasserstoff oder C₁-C₆-Alkyl;
- R³: Wasserstoff, ggf. subst. C₁-C₆-Alkyl, ggf. subst. C₂-C₆-Alkenyl oder ggf. subst. C₂-C₆-Alkinyl bedeuten.

Zusätzlich betrifft die Erfindung verfahren und Zwischenprodukte zur Herstellung der Verbindungen I und die Verwendung der Verbindungen I zur Bekämpfung von Schadpilzen und tierischen Schädlingen.

4-Phenyl-2,3-dihydro-isoxazolone und 4-Phenyl-2,4-dihydro-triazolone mit orthoständigem Substituenten werden in WO-A 95/14,009 und WO-A 97/02,255 offenbart.

α-Phenylacrylsäure- und α-Phenyl-α-methoximinoessigsäurederivate mit orthoständigem Aryl- oder Hetaryl-Substituenten sind in EP-A 253 213 und EP-A 398 692, solche mit orthoständiger Oximether-Gruppierung sind in DE-A 40 20 384, solche mit Bisoximether-Gruppierung in WO-A 95/21,153 und WO-A 97/05,103 und solche mit Trisoximether-Gruppierung in WO-A 97/15,552 beschrieben.

Cyclische Amide, die in ortho-Stellung zu der Amid-Carbonylgruppe einen orthoständig substituierten Phenylring tragen, sind aus WO-A 95/14,009, WO-A 96/26,191, WO-A 96/36,229, WO-A 96/36,615, WO-A 96/36,616, WO-A 96/38,425, WO-A 97/00,612 und WO-A 97/05,120 bekannt. Cyclische Amide, die in ortho-Stellung zu der Amid-Carbonylgruppe einen substituierten Heterocyclus tragen, sind in WO-A 96/36,633 offenbart.

Die in den vorstehend genannten Schriften beschriebenen Verbindungen sind als Pflanzenschutzmittel gegen Schadpilze und z. T. gegen tierische Schädlinge geeignet.

Ihre Wirkung ist jedoch in vielen Fällen nicht zufriedenstellend. Daher lag als Aufgabe zugrunde, Verbindungen mit verbesserter Wirksamkeit zu finden.

Demgemäß wurden die substituierten Phenylverbindungen der Formel I gefunden. Weiterhin wurden Zwischenprodukte und Verfahren zur Herstellung der Verbindungen I, sowie die Verwendung der Verbindungen I und diese enthaltene Mittel zur Bekämpfung von Schadpilzen und tierischen Schädlingen gefunden. Die fungizide Wirkung ist bevorzugt.

Die Verbindungen der Formel I unterscheiden sich von den aus den oben genannten Schriften bekannten Verbindungen durch die Ausgestaltung der Gruppe E: In den Verbindungen der Formel IA ist die Gruppe E, wenn V für Sauerstoff steht, ein Dioxolandion und falls V N-R^{α} bedeutet, ein Oxazolidindion. In den Verbindungen der Formel IB ist die Gruppe E, wenn V für Sauerstoff steht, ein Oxadiazolidindion und falls V N-R^{α} bedeutet, ein Triazolidindion.

Die Verbindungen der Formel I weisen eine gegenüber den bekannten Verbindungen erhöhte Wirksamkeit gegen Schadpilze und tierische Schädlinge auf.

Die Verbindungen I können auf verschiedenen Wegen erhalten werden, wobei es für die Synthese unerheblich ist, ob zunächst die Heterocyclen E oder die Gruppierung T-Z aufgebaut wird. Zur besseren Übersichtlichkeit wird in den nachfolgenden Reaktionsbeschreibungen daher die Bezeichnung
- E^{#}: für die Gruppe A oder B, bzw. eine geeignete Vorstufe dafür und
- Z^{#}: für die Gruppierung T-Z, bzw. eine geeignete Vorstufe dafür verwendet.

Für Verbindungen der Formel IA 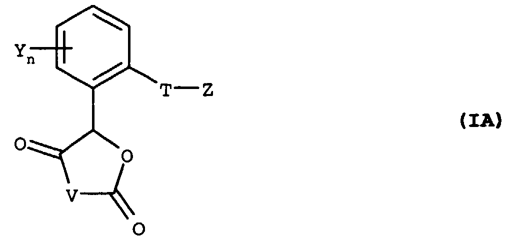 wird die Einführung der Gruppierung T-Z auf der Stufe, in der E^{#} C(OH)COOR', wobei R' für C₁-C₄-Alkyl steht, bedeutet, bevorzugt.

Für Verbindungen der Formel IB 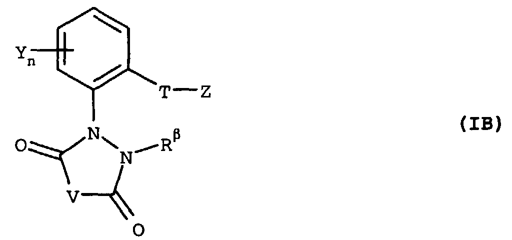 wird die Einführung der Gruppierung T-Z auf der Stufe, in der E^{#} für NH-NH₂ steht, bevorzugt.

Die Gruppierung T-Z in den Verbindungen der Formel I kann an sich analog der in WO-A 96/07,633, bzw. WO-A 97/24,317 beschriebenen Methoden erhalten werden.

Die im Folgenden beschriebene Syntheseroute ist gleichermaßen für Verbindungen der Formeln I, in denen T für Sauerstoff oder Oxymethylen steht, geeignet.

Die Oxazolidindione der Formel IAa^{#} erhält man besonders vorteilhaft dadurch, daß man einen α-Hydroxy-α-Phenylessigsäurealkylester der Formel IIA^{#} mit Phosgen oder einem Phosgenäquivalent zu Dicarbonylverbindungen der Formel IIIA^{#} acyliert, die durch Umsetzung mit primären Aminen der Formel IVa zu den Oxadiazolidindionen der Formel IAa^{#} cyclisieren.

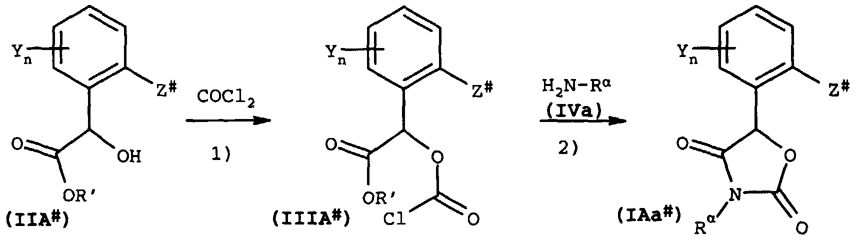

In der Formel IIA^{#} steht R' für C₁-C₄-Alkyl. Verbindungen der Formel IIA^{#} sind literaturbekannt, bzw. können nach literaturbekannten Methoden hergestellt werden [vgl. WO-A 96/07,633].
1) Die Umsetzung von IIA^{#} mit Phosgen oder einem phosgenäquivalent, wie beispielsweise Di- oder Triphosgen, erfolgt üblicherweise bei Temperaturen von -10°C bis 250°C, vorzugsweise 10°C bis 110°C, in einem inerten organischen Lösungsmittel in Gegenwart einer Base oder eines Katalysators [vgl. Chem. Ber., Bd. 72, S. 457 (1972); Chem. Soc. Rev., Bd. 3, S. 209 (1974); Angew. Chem., Bd. 107, S. 2746 (1995)].
   Geeignete Lösungsmittel sind aliphatische Kohlenwasserstoffe, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile, sowie Dimethylsulfoxid, Dimethylformamid und Dimethylacetamid, besonders bevorzugt Tetrahydrofuran, Dimethylformamid, Chlorbenzol und Toluol. Es können auch Gemische der genannten Lösungsmittel verwendet werden.
   Als Basen kommen allgemein anorganische Verbindungen, wie Alkalimetall- und Erdalkalimetallhydroxide, Alkalimetall- und Erdalkalimetalloxide, Alkalimetall- und Erdalkalimetallhydride, Alkalimetallamide, Alkalimetall- und Erdalkalimetallcarbonate, sowie Alkalimetallhydrogencarbonate, metallorganische Verbindungen, insbesondere Alkalimetallalkyle, Alkylmagnesiumhalogenide, sowie außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Triethylamin und Pyridin. Die Basen werden im allgemeinen äquimolar, im Überschuß oder gegebenenfalls als Lösungsmittel verwendet.
   Die Edukte werden im allgemeinen in äquimolaren Mengen miteinander umgesetzt. Es kann für die Ausbeute vorteilhaft sein, Phosgen oder das Phosgenäquivalent in einem Überschuß bezogen auf IIA^{#} einzusetzen.
2) Diese Umsetzung erfolgt üblicherweise bei Temperaturen von -10°C bis 250°C, vorzugsweise 0°C bis 100°C, in einem inerten organischen Lösungsmittel [vgl. Chem. Ber., Bd. 33, Seiten 455 ff. (1900)].
   Geeignete Lösungsmittel sind aliphatische Kohlenwasserstoffe, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile, Alkohole wie Methanol, Ethanol, n-Propanol, Isopropanol, n-Butanol und tert.-Butanol sowie Dimethylsulfoxid, Dimethylformamid und Dimethylacetamid, besonders bevorzugt Toluol, Tetrahydrofuran, Dimethylformamid, Chlorbenzol und Ethanol. Es können auch Gemische der genannten Lösungsmittel verwendet werden.
   Die Edukte werden im allgemeinen in äquimolaren Mengen miteinander umgesetzt. Es kann für die Ausbeute vorteilhaft sein, IVa in einem Überschuß bezogen auf IIIA^{#} einzusetzen.
   Sofern die Dicarbonylverbindungen IIIA^{#} durch Umsetzung mit IVa nicht von selbst cyclisieren, kann die Bildung der Oxadiazolidindione durch Verseifung der Estergruppe in den Verbindungen IIIA^{#} und anschließende Säurebehandlung erfolgen. 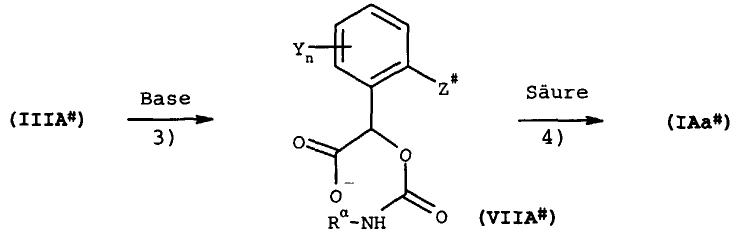
3) Die Verseifung erfolgt üblicherweise bei Temperaturen von 10°C bis 150°C, vorzugsweise 20°C bis 100°C, in einem inerten organischen Lösungsmittel in Gegenwart einer Base [vgl. Chem. Zentralblatt, S. 936 ff. (1901)].
   Geeignete Lösungsmittel sind aliphatische Kohlenwasserstoffe, aromatische Kohlenwasserstoffe, Ether wie Diethylether, Diisopropylether, tert.-Butylmethylether, Dioxan, Anisol und Tetrahydrofuran, Alkohole wie Methanol, Ethanol, n-Propanol, Isopropanol, n-Butanol und tert.-Butanol sowie Dimethylsulfoxid, Dimethylformamid und Dimethylacetamid, besonders bevorzugt Methanol, Ethanol, n.-Propanol, Diethylether und Dimethylformamid. Es können auch Gemische der genannten Lösungsmittel verwendet werden.
   Als Basen kommen allgemein anorganische Verbindungen wie Alkalimetall- und Erdalkalimetallhydroxide wie Lithiumhydroxid, Natriumhydroxid, Kaliumhydroxid und Calziumhydroxid, Alkalimetall- und Erdalkalimetalloxide, Alkalimetall- und Erdalkalimetallcarbonate sowie Alkalimetallhydrogencarbonate, in Betracht. Besonders bevorzugt werden Natrium- und Kaliumhydroxid.
   Die Edukte werden im allgemeinen in äquimolaren Mengen miteinander umgesetzt. Es kann für die Ausbeute vorteilhaft sein, die Base in einem Überschuß bezogen auf IIIA^{#} einzusetzen.
4) Die Cyclisierung erfolgt üblicherweise bei Temperaturen von 10°C bis 150°C, vorzugsweise 20°C bis 100°C, in einem inerten organischen Lösungsmittel in Gegenwart einer Säure [vgl. Chem. zentralblatt, S. 936 ff. (1901)].
   Geeignete Lösungsmittel sind aliphatische Kohlenwasserstoffe, aromatische Kohlenwasserstoffe, halogenierte Kohlenwasserstoffe, Ether wie Diethylether, Diisopropylether, tert.-Butylmethylether, Dioxan, Anisol und Tetrahydrofuran, Nitrile, Alkohole wie Methanol, Ethanol, n-Propanol, Isopropanol, n-Butanol und tert.-Butanol sowie Dimethylsulfoxid, Dimethylformamid und Dimethylacetamid, besonders bevorzugt Methanol, Ethanol, Diethylether und tert.-Butylmethylether. Es können auch Gemische der genannten Lösungsmittel verwendet werden.
   Als Säuren und saure Katalysatoren finden anorganische Säuren wie Fluorwasserstoffsäure, Salzsäure, Bromwasserstoffsäure, Schwefelsäure und Perchlorsäure, sowie organische Säuren wie Ameisensäure, Essigsäure, Propionsäure, Oxalsäure, Zitronensäure und Trifluoressigsäure Verwendung.
   Die Edukte werden im allgemeinen in äquimolaren Mengen miteinander umgesetzt. Es kann für die Ausbeute vorteilhaft sein, die Säure in einem Überschuß bezogen auf VIIA^{#} einzusetzen.
   Zwischenprodukte der Formel 1, 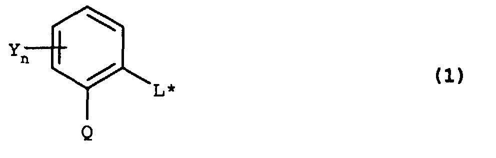 in der Q für CH(COOR')OCOCl, CH(COOH)OCOCl, CH(COOR')OCONHR^{α}, CH(COOH)OCONHR^{α}, oder eine Gruppe A und L* für CH₂L' oder eine Gruppe L', wobei L' eine nucleophil abspaltbare Gruppe bedeutet, und R' für C₁-C₄-Alkyl steht, sind neu.
   Die Triazolidindione der Formel IBa^{#} erhält man besonders vorteilhaft dadurch, daß man Phenylhydrazine der Formel IIB^{#} mit Chlorameisensäurealkylestern in Carbamate der Formel IIIB^{#} überführt, die mit Phosgen oder einem Phosgenäquivalent zu Dicarbonylverbindungen der Formel VB^{#} acyliert werden. 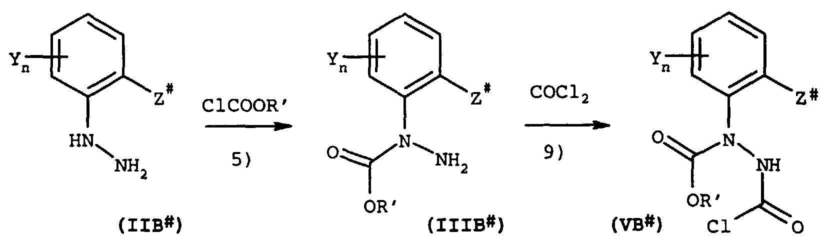
   In der Formel IIIB^{#} steht R' für C₁-C₄-Alkyl. Die Verbindungen VB^{#} werden durch Umsetzung mit primären Aminen der Formel IVa zu den Triazolidindionen der Formel IBa^{#} cyclisiert.
5) Die Umsetzung von IIB^{#} mit Chlorameisensäurealkylestern zu Carbamaten der Formel IIIB^{#} erfolgt üblicherweise bei Temperaturen von 0°C bis 200°C, vorzugsweise 20°C bis 150°C, mit niederen Chlorameisensäurealkylestern in einem inerten organischen Lösungsmittel im allgemeinen in Gegenwart einer Base oder einer Säure [vgl. Synth. Commun. Bd. 15, S. 697 (1985)].
   Geeignete Lösungsmittel sind aliphatische Kohlenwasserstoffe, aromatische Kohlenwasserstoffe, halogenierte Kohlenwasserstoffe, Ether wie Diethylether, Diisopropylether, tert.-Butylmethylether, Dioxan, Anisol und Tetrahydrofuran, Nitrile, Ketone, Alkohole wie Methanol, Ethanol, n-Propanol, Isopropanol, n-Butanol und tert.-Butanol, sowie Dimethylsulfoxid, Dimethylformamid und Dimethylacetamid, besonders bevorzugt Ethanol, Dimethylformamid, Tetrahydrofuran und Diethylether. Es können auch Gemische der genannten Lösungsmittel verwendet werden.
   Als Basen kommen allgemein anorganische Verbindungen, wie Alkalimetall- und Erdalkalimetallhydroxide wie Lithiumhydroxid, Natriumhydroxid, Kaliumhydroxid und Calziumhydroxid, Alkalimetall- und Erdalkalimetalloxide, Alkalimetall- und Erdalkalimetallhydride wie Lithiumhydrid, Natriumhydrid, Kaliumhydrid und Calziumhydrid, Alkalimetallamide, Alkalimetall- und Erdalkalimetallcarbonate wie Lithiumcarbonat, Kaliumcarbonat und Calziumcarbonat sowie Alkalimetallhydrogencarbonate wie Natriumhydrogencarbonat, metallorganische Verbindungen, insbesondere Alkalimetallalkyle, Alkylmagnesiumhalogenide sowie Alkalimetall- und Erdalkalimetallalkoholate wie Natriummethanolat, Natriumethanolat, Kaliumethanolat, Kaliumtert.-Butanolat und Dimethoxymagnesium, außerdem organische Basen, z.B. tertiäre Amine wie Trimethylamin, Triethylamin, Triisopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Triethylamin, Natriumhydrid, Natrium- und Kaliumethanolat und Natrium- und Kaliumhydroxid. Die Basen werden im allgemeinen in katalytischen Mengen eingesetzt, sie können aber auch äquimolar, im Überschuß oder gegebenenfalls als Lösungsmittel verwendet werden.
   Als Säuren und saure Katalysatoren finden anorganische Säuren, wie Fluorwasserstoffsäure, Salzsäure, Bromwasserstoffsäure, Schwefelsäure und Perchlorsäure, Lewis-Säuren wie Bortrifluorid, Aluminiumtrichlorid, Eisen-III-chlorid, Zinn-IV-chlorid, Titan-IV-chlorid und Zink-II-chlorid, sowie organische Säuren wie Ameisensäure, Essigsäure, Propionsäure, Oxalsäure, Zitronensäure und Trifluoressigsäure Verwendung. Die Säuren werden im allgemeinen in katalytischen Mengen eingesetzt, sie können aber auch äquimolar, im Überschuß oder gegebenenfalls als Lösungsmittel verwendet werden.
   Phenylhydrazine IIB^{#} und Chlorameisensäurealkylester werden im allgemeinen in äquimolaren Mengen miteinander umgesetzt. Es kann für die Ausbeute vorteilhaft sein, die Chlorameisensäurealkylester in einem Überschuß bezogen auf IIB^{#} einzusetzen.
   Phenylhydrazine IIB^{#} können nach literaturbekannten Methoden aus den entsprechenden Nitroverbindungen IIB1^{#} durch Reduktion der Nitrogruppe und Diazotierung der entstandenen Aniline IIB2^{#}, die zu den Phenylhydrazinen IIB^{#} reduziert werden, erhalten werden. 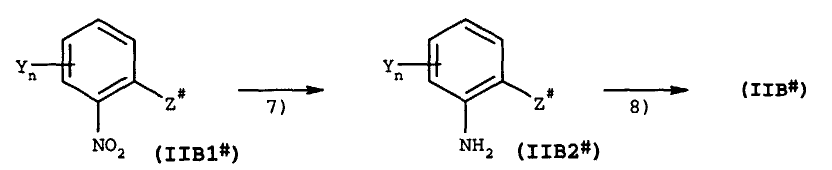
7) Die Reduktion der Nitrogruppe von IIB1^{#} kann unter allgemein üblichen Bedingungen durchgeführt werden, bevorzugt durch katalytische Hydrierung, durch Reduktion mit Eisen, Zinn oder Zink in Gegenwart einer Säure, durch Reduktion mit Alkalimetallen in Gegenwart einer Base oder durch enzymkatalysierte Reduktion [vgl. Houben-Weyl, Bd. IV/1c, 4. Aufl., S. 506ff., Thieme Verlag Stuttgart und New York (1980); ebd. Bd. IV/1d, 4. Aufl., S. 473ff. (1981); Heterocycles, Bd. 31, S. 2201 (1990)]
   Nitrobenzolderivate der Formel IIB1^{#} sind z. T. aus der Literatur bekannt [vgl. EP-A 498 396; WO-A 93/15,046; WO-A 95/14,009] oder können gemäß der zitierten Literatur hergestellt werden.
8a) Die Umsetzung von IIB2^{#} mit Nitrit, erfolgt bei Temperaturen von -10°C bis 25°C, vorzugsweise -5°C bis 10°C, in Wasser oder einem inerten organischen Lösungsmittel in Gegenwart einer Säure [vgl. Organikum, 15. Auflage 1976, S. 654ff., VEB Verlag der Wissenschaften, Berlin].
8b) Die Reduktion der Diazoverbindung kann unter allgemein üblichen Bedingungen durchgeführt werden, bevorzugt durch Reduktion mit Eisen, Zinn oder Zink oder deren Salzen in Gegenwart einer Säure oder durch Reduktion mit Alkalimetallen in Gegenwart einer Base [vgl. Houben-Weyl, Bd. IV/1c, 4. Aufl., S. 506ff., Thieme Verlag Stuttgart und New York (1980); ebd. Bd. IV/1d, 4. Aufl., S. 473ff. (1981); Heterocycles, Bd. 31, S. 2201 (1990)]. Weiterhin bevorzugt ist die Reduktion der Diazoniumsalze mit Sulfit oder Disulfit [vgl: Organikum, 15. Aufl., VEB, S. 662, 1976; J. Chem. Soc, 1927, S. 1325ff.: Chem. Ber., Bd. 55, S. 1827, 1922; Houben-Weyl, Bd. 10/2, S. 182, Thieme Verlag, Stuttgart].
9) Die Umsetzung der Carbamate IIIB^{#} mit Phosgen oder einem Phosgenäquivalent erfolgt unter den in Abschnitt 1) für die Acylierung von IIA^{#} zu den Dicarbonylverbindungen IIIA^{#} geschilderten Bedingungen.
   Die Triazolidindione der Formel IBa^{#} erhält man besonders vorteilhaft dadurch, daß man Dicarbonylverbindungen der Formel VB^{#} durch Umsetzung mit primären Aminen der Formel IVa cyclisiert. 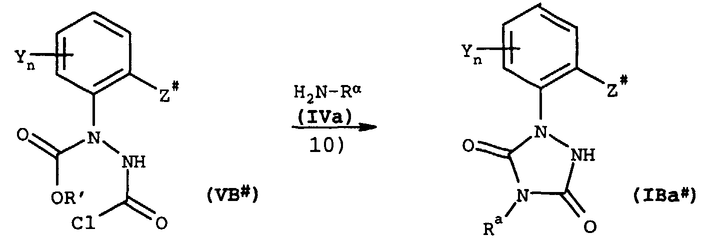
10) Die Cyclisierung von VB^{#} zu den Triazolidindionen IBa^{#} erfolgt unter den in dem Abschnitt 2) für die Cyclisierung der Dicarbonylverbindungen IIIA^{#} geschilderten Bedingungen.
   Sofern die Dicarbonylverbindungen VB^{#} durch Umsetzung mit IVa nicht von selbst cyclisieren, kann die Bildung der Oxadiazolidindione durch Verseifung der Estergruppe in den Verbindungen VIIB^{#} und anschließende Säurebehandlung erfolgen. 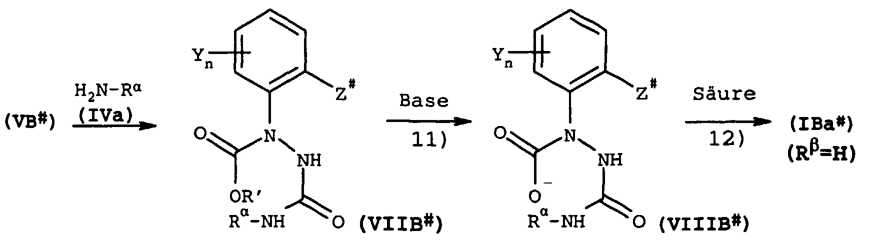
11-12) Die Cyclisierung von VB^{#} über die Zwischenstufen VIIB^{#} und VIIIB^{#} erfolgt unter den in den Abschnitten 3) und 4) für die Cyclisierung der Dicarbonylverbindungen IIIA^{#} geschilderten Bedingungen. Zwischenprodukte der Formel 2, 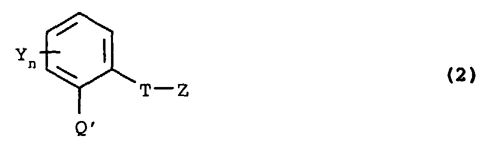 in der Q' N(NH₂)COOR', N(NHCOCl)COOR' oder N(NHCONHR^{α})COOR' bedeuten, wobei Y, n, T, Z und R^{α} die Bedeutung wie in Formel I und R' C₁-C₄-Alkyl bedeuten, sind neu.
   Verbindungen der Formel IBa^{#}, in denen R^{β} nicht für Wasserstoff steht, erhält man bevorzugt aus den entsprechenden Verbindungen IBa^{#} mit R^{β} = Wasserstoff durch Alkylierung. 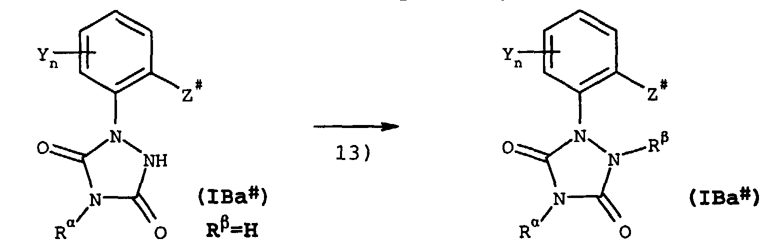
13) Die Alkylierung der Triazolidindione IBa^{#} erfolgt unter allgemein üblichen Bedingungen ggf. in Gegenwart einer Base [vgl. Synth. Commun., Bd. 18, S. 2011 (1988); J. Chem. Soc. Chem. Commun., S. 735 (1987)].
   Als Alkylierungsagentien kommen beispielsweise Alkylhalogenide, Alkylsulfonate, Alkyl-p-toluolsulfonate, Alkyl-trifluormethansulfonate, Alkohole, Ether oder Alkyl-p-bromphenylsulfonate oder die entsprechenden Benzylverbindungen, insbesondere Methyljodid oder Dimethylsulfat in Betracht.
   Die Edukte werden im allgemeinen in äquimolaren Mengen miteinander umgesetzt. Es kann für die Ausbeute vorteilhaft sein, das Alkylierungsagenz in einem Überschuß bezogen auf IBa^{#} einzusetzen.
   Phenylverbindungen der Formel I'^{#} werden bevorzugt aus den Verbindungen der Formel II'^{#} erhalten. In der Formel II'# steht L' für eine für die nucleophile aromatische Substitution übliche Abgangsgruppe, wie beispielsweise Fluor, Chlor, Brom, Nitro oder Alkyl- oder Arylsulfonate, wie Mesylat, Tosylat oder Triflat. Bevorzugte Abgangsgruppe ist Fluor. 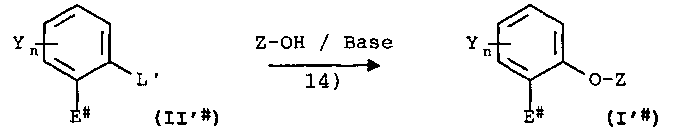
14) Diese Umsetzung erfolgt üblicherweise bei Temperaturen von -20°C bis 170°C, vorzugsweise 0°C bis 100°C, in einem inerten organischen Lösungsmittel in Gegenwart einer Base [vgl. WO-A 97/24,317; J. Chem. Soc. Perkin Trans., Bd. 1, S. 1727 (1989); Chem. Ber., Bd. 121, S. 2035 (1988)].
   Geeignete Lösungsmittel sind aromatische Kohlenwasserstoffe, halogenierte Kohlenwasserstoffe, Ether wie Diethylether, Diisopropylether, tert.-Butylmethylether, Dioxan, Anisol und Tetrahydrofuran, Nitrile, Ketone, sowie Dimethylsulfoxid, Dimethylformamid und Dimethylacetamid, besonders bevorzugt Dimethylformamid, Dimethylsulfoxid und Tetrahydrofuran. Es können auch Gemische der genannten Lösungsmittel verwendet werden.
   Als Basen kommen allgemein anorganische Verbindungen, wie Alkalimetall- und Erdalkalimetallhydroxide, Alkalimetall- und Erdalkalimetalloxide, Alkalimetall- und Erdalkalimetallhydride wie Lithiumhydrid, Natriumhydrid, Kaliumhydrid und Calziumhydrid, Alkalimetall- und Erdalkalimetallcarbonate wie Lithiumcarbonat, Kaliumcarbonat und Calziumcarbonat, sowie Alkalimetallhydrogencarbonate wie Natriumhydrogencarbonat, metallorganische Verbindungen, insbesondere Alkalimetallalkyle, Alkylmagnesiumhalogenide, sowie Alkalimetall- und Erdalkalimetallalkoholate wie Natriummethanolat, Natriumethanolat, Kaliumethanolat, Kaliumtert.-Butanolat und Dimethoxymagnesium, außerdem organische Basen, z.B. tertiäre Amine, Pyridin, substituierte Pyridine sowie bicyclische Amine in Betracht. Besonders bevorzugt werden Natriumhydrid, Kalium-tert. Butanolat und Kaliumcarbonat. Die Basen werden im allgemeinen äquimolar, im Überschuß oder gegebenenfalls als Lösungsmittel verwendet.
   Die Edukte werden im allgemeinen in äquimolaren Mengen miteinander umgesetzt. Es kann für die Ausbeute vorteilhaft sein, Z-OH in einem Überschuß bezogen auf II'^{#} einzusetzen.
   Phenylverbindungen der Formel I'^{#}, in denen Z für eine Gruppe X steht, können alternativ auch aus Phenolen der Formel IIb'^{#} erhalten werden. IIb'^{#} wird mit einem Halogenid Z-Hal zu I'^{#} umgesetzt. 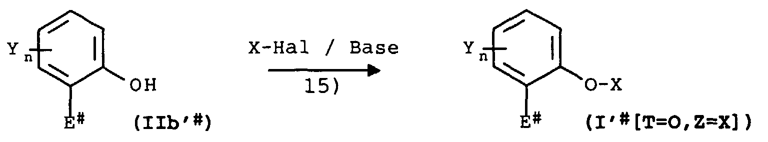
15) Diese Umsetzung erfolgt üblicherweise bei Temperaturen von -20°C bis 180°C, vorzugsweise 20°C bis 160°C, in einem inerten organischen Lösungsmittel in Gegenwart einer Base [vgl. EP-A 203 608; EP-A 242 081].
   Geeignete Lösungsmittel sind aromatische Kohlenwasserstoffe, halogenierte Kohlenwasserstoffe, Ether, Nitrile, Ketone, sowie Dimethylsulfoxid, Dimethylformamid und Dimethylacetamid, besonders bevorzugt Dimethylformamid und Dimethylsulfoxid. Es können auch Gemische der genannten Lösungsmittel verwendet werden.
   Als Basen kommen allgemein anorganische Verbindungen, wie Alkalimetall- und Erdalkalimetallhydroxide, Alkalimetall- und Erdalkalimetalloxide, Alkalimetall- und Erdalkalimetallhydride wie Lithiumhydrid, Natriumhydrid, Kaliumhydrid und Calziumhydrid, Alkalimetall- und Erdalkalimetallcarbonate wie Lithiumcarbonat, Kaliumcarbonat und Calziumcarbonat, sowie Alkalimetallhydrogencarbonate wie Natriumhydrogencarbonat, metallorganische Verbindungen, insbesondere Alkalimetallalkyle, Alkylmagnesiumhalogenide, sowie Alkalimetall- und Erdalkalimetallalkoholate wie Natriummethanolat, Natriumethanolat, Kaliumethanolat, Kalium- tert.-Butanolat und Dimethoxymagnesium, außerdem organische Basen, z.B. tertiäre Amine, Pyridin, substituierte Pyridine sowie bicyclische Amine in Betracht. Besonders bevorzugt werden Natriumhydrid, Kalium-tert. Butanolat und Kaliumcarbonat. Die Basen werden im allgemeinen äquimolar, im Überschuß oder gegebenenfalls als Lösungsmittel verwendet.
   Die Edukte werden im allgemeinen in äquimolaren Mengen miteinander umgesetzt. Es kann für die Ausbeute vorteilhaft sein, X-Hal in einem Überschuß bezogen auf IIb'^{#} einzusetzen.
   Phenylverbindungen der Formel I''^{#} werden bevorzugt aus den Benzylverbindungen der Formel II''^{#} erhalten. In der Formel II''^{#} steht L für eine nucleofuge Abgangsgruppe, wie Halogen oder Alkyl- oder Arylsulfonat, vorzugsweise Brom, Chlor, Jod, Mesylat, Tosylat oder Triflat und E^{#} für eine Gruppe A oder B oder eine Vorstufe dafür. 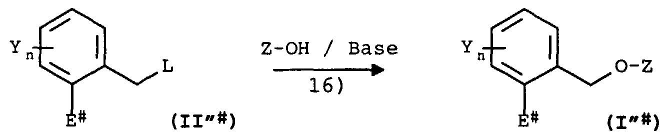
16) Diese Umsetzung erfolgt üblicherweise bei Temperaturen von 0°C bis 180°C, vorzugsweise 20°C bis 60°C, in einem inerten organischen Lösungsmittel in Gegenwart einer Base [vgl. EP-A 254 426; EP-A 463 488; WO-A 95/18,789; WO-A 95/29,896].
   Geeignete Lösungsmittel sind aliphatische Kohlenwasserstoffe, aromatische Kohlenwasserstoffe, halogenierte Kohlenwasserstoffe, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole, sowie Dimethylsulfoxid, Dimethylformamid und Dimethylacetamid, besonders bevorzugt Dimethylformamid, Tetrahydrofuran und Aceton. Es können auch Gemische der genannten Lösungsmittel verwendet werden.
   Als Basen kommen allgemein anorganische Verbindungen, wie Alkalimetall- und Erdalkalimetallhydroxide, Alkalimetall- und Erdalkalimetalloxide, Alkalimetall- und Erdalkalimetallhydride wie Lithiumhydrid, Natriumhydrid, Kaliumhydrid und Calziumhydrid, Alkalimetall- und Erdalkalimetallcarbonate wie Lithiumcarbonat, Kaliumcarbonat und Calziumcarbonat, sowie Alkalimetallhydrogencarbonate wie Natriumhydrogencarbonat, metallorganische Verbindungen, insbesondere Alkalimetallalkyle, Alkylmagnesiumhalogenide, sowie Alkalimetall- und Erdalkalimetallalkoholate wie Natriummethanolat, Natriumethanolat, Kaliumethanolat, Kalium- tert.-Butanolat und Dimethoxymagnesium, außerdem organische Basen, z.B. tertiäre Amine, Pyridin, substituierte Pyridine sowie bicyclische Amine in Betracht. Besonders bevorzugt werden Natriumhydrid, Kaliumcarbonat und Natriummethanolat. Die Basen werden im allgemeinen äquimolar, im Überschuß oder gegebenenfalls als Lösungsmittel verwendet.
   Die Edukte werden im allgemeinen in äquimolaren Mengen miteinander umgesetzt. Es kann für die Ausbeute vorteilhaft sein, Z-OH in einem Überschuß bezogen auf II''^{#} einzusetzen.
   Die für die Herstellung der Verbindungen I' benötigten Ausgangsstoffe Z-OH sind bekannt oder können gemäß der zitierten Literatur hergestellt werden.
17) Die Benzylverbindungen II''^{#} sind unter allgemein üblichen Bedingungen aus den entsprechenden Tolylverbindungen IIa''^{#} zugänglich [vgl. J. Amer. Chem. Soc., Bd. 71, S. 2137ff. (1949); ebd., Bd. 90, S. 1797ff. (1968)].

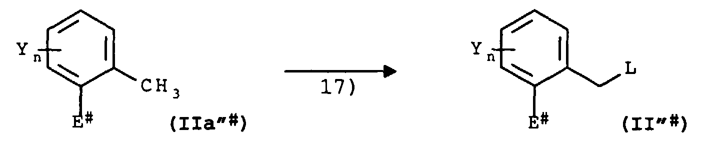

Sofern einzelne Verbindungen I nicht auf den voranstehend beschriebenen Wegen zugänglich sind, können sie durch Derivatisierung anderer Verbindungen I hergestellt werden.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischen- und Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Die Verbindungen I können bei der Herstellung aufgrund ihrer C=C- und C=N-Doppelbindungen als E/Z-Isomerengemische anfallen, die z.B. durch Kristallisation oder Chromatographie in üblicher Weise in die Einzelverbindungen getrennt werden können.

Sofern bei der Synthese Isomerengemische anfallen, ist im allgemeinen jedoch eine Trennung nicht unbedingt erforderlich, da sich die einzelnen Isomere teilweise während der Aufbereitung für die Anwendung oder bei der Anwendung (z.B. unter Licht-, Säure- oder Baseneinwirkung) ineinander umwandeln können. Entsprechende Umwandlungen können auch nach der Anwendung, beispielsweise bei der Behandlung von Pflanzen in der behandelten Pflanze oder im zu bekämpfenden Schadpilz oder tierischen Schädling erfolgen.

In Bezug auf die -N=C(R¹)-C(R²)=NOR³ Doppelbindungen werden im allgemeinen hinsichtlich ihrer Wirksamkeit die trans,trans-Isomere der Verbindungen I bevorzugt (Konfiguration bezogen auf den Rest T im Verhältnis zur C(R²)=NOR³-Gruppe, bzw. bezogen auf den Rest OR³ im Verhältnis zur T-N=C(R¹)-Gruppe).

Bei den in den vorstehenden Formeln angegebenen Definitionen der Symbole wurden Sammelbegriffe verwendet, die allgemein repräsentativ für die folgenden Substituenten stehen:
**Halogen:** Fluor, Chlor, Brom und Jod;
**Alkyl:** gesättigte, geradkettige oder verzweigte Kohlenwasserstoffreste mit 1 bis 4 oder 6 Kohlenstoffatomen, z.B. C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
**Halogenalkyl:** geradkettige oder verzweigte Alkylgruppen mit 1 bis 10 Kohlenstoffatomen (wie vorstehend genannt), wobei in diesen Gruppen teilweise oder vollständig die Wasserstoffatome durch Halogenatome wie vorstehend genannt ersetzt sein können, z.B. C₁-C₂-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
**Alkoxy:** geradkettige oder verzweigte Alkylgruppen mit 1 bis 6 Kohlenstoffatomen (wie vorstehend genannt), welche über ein Sauerstoffatom (-O-) an das Gerüst gebunden sind;
**Halogenalkoxy:** geradkettige oder verzweigte Halogenalkylgruppen mit 1 bis 6 Kohlenstoffatomen (wie vorstehend genannt), welche über ein Sauerstoffatom (-O-) an das Gerüst gebunden sind;
**Alkenyl:** ungesättigte, geradkettige oder verzweigte Kohlenwasserstoffreste mit 2 bis 4 oder 6 Kohlenstoffatomen und einer Doppelbindung in einer beliebigen Position, z.B. Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1propenyl und 1-Ethyl-2-methyl-2-propenyl;
**Halogenalkenyl:** ungesättigte, geradkettige oder verzweigte Kohlenwasserstoffreste mit 2 bis 6 Kohlenstoffatomen und einer Doppelbindung in einer beliebigen Position (wie vorstehend genannt), wobei in diesen Gruppen die Wasserstoffatome teilweise oder vollständig gegen Halogenatome wie vorstehend genannt, insbesondere Fluor, Chlor und Brom, ersetzt sein können;
**Alkenyloxy:** ungesättigte, geradkettige oder verzweigte Kohlenwasserstoffreste mit 3 bis 6 Kohlenstoffatomen und einer Doppelbindung in einer beliebigen, nicht zum Heteroatom benachbarten, Position (wie vorstehend genannt), welche über ein Sauerstoffatom (-O-) an das Gerüst gebunden sind;
**Halogenalkenyloxy:** ungesättigte, geradkettige oder verzweigte Alkenyloxygruppen mit 3 bis 6 Kohlenstoffatomen (wie vorstehend genannt), wobei in diesen Gruppen die Wasserstoffatome teilweise oder vollständig gegen Halogenatome wie vorstehend genannt, insbesondere Fluor, Chlor und Brom, ersetzt sein können;
**Alkinyl:** geradkettige oder verzweigte Kohlenwasserstoffgruppen mit 2 bis 4 oder 6 Kohlenstoffatomen und einer Dreifachbindung in einer beliebigen Position, z.B. C₂-C₆-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;
**Halogenalkinyl:** ungesättigte, geradkettige oder verzweigte Kohlenwasserstoffreste mit 2 bis 6 Kohlenstoffatomen und einer Dreifachbindung in einer beliebigen Position (wie vorstehend genannt), wobei in diesen Gruppen die Wasserstoffatome teilweise oder vollständig gegen Halogenatome wie vorstehend genannt, insbesondere Fluor, Chlor und Brom, ersetzt sein können;
**Alkinyloxy:** ungesättigte, geradkettige oder verzweigte Kohlenwasserstoffreste mit 3 bis 6 Kohlenstoffatomen und einer Dreifachbindung in einer beliebigen, nicht zum Heteroatom benachbarten, Position (wie vorstehend genannt), welche über ein Sauerstoffatom (-O-) an das Gerüst gebunden sind;
**Halogenalkinyloxy:** ungesättigte, geradkettige oder verzweigte Alkinyloxygruppen mit 3 bis 6 Kohlenstoffatomen (wie vorstehend genannt), wobei in diesen Gruppen die Wasserstoffatome teilweise oder vollständig gegen Halogenatome wie vorstehend genannt, insbesondere Fluor, Chlor und Brom, ersetzt sein können;
**Cycloalkyl:** monocyclische, gesättigte Kohlenwasserstoffgruppen mit 3 bis 6, 8, 10 oder 12 Kohlenstoffringgliedern, z.B. C₃-C₈-Cycloalkyl wie Cyclopropyl, Cyclobutyl, Cyclopentyl, Cyclohexyl, Cycloheptyl und Cyclooctyl;
**Cycloalkoxy:** monocyclische, gesättigte Kohlenwasserstoffgruppen mit 3 bis 12 Kohlenstoffringgliedern (wie vorstehend genannt), welche über ein Sauerstoffatom (-O-) an das Gerüst gebunden sind;
**gesättigter oder partiell ungesättigter cyclischer Rest, welcher neben Kohlenstoffatomen als Ringglieder Heteroatome aus der Gruppe Sauerstoff, Schwefel oder Stickstoff enthalten kann:** Cycloalkyl mit 3 bis 12 Kohlenstoffringgliedern wie vorstehend genannt oder 5- oder 6-gliedrige Heterocyclen (Heterocyclyl) enthaltend neben Kohlenstoffringgliedern ein bis drei Stickstoffatome und/oder ein Sauerstoff- oder Schwefelatom oder ein oder zwei Sauerstoff- und/oder Schwefelatome, z.B. 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,4-Dihydrofur-2-yl, 2,4-Dihydrofur-3-yl, 2,3-Dihydrothien-2-yl, 2,3-Dihydrothien-3-yl, 2,4-Dihydrothien-2-yl, 2,4-Dihydrothien-3-yl, 2,3-Pyrrolin-2-yl, 2,3-Pyrrolin-3-yl, 2,4-Pyrrolin-2-yl, 2,4-Pyrrolin-3-yl, 2,3-Isoxazolin-3-yl, 3,4-Isoxazolin-3-yl, 4,5-Isoxazolin-3-yl, 2,3-Isoxazolin-4-yl, 3,4-Isoxazolin-4-yl, 4,5-Isoxazolin-4-yl, 2,3-Isoxazolin-5-yl, 3,4-Isoxazolin-5-yl, 4,5-Isoxazolin-5-yl, 2,3-Isothiazolin-3-yl, 3,4-Isothiazolin-3-yl, 4,5-Isothiazolin-3-yl, 2,3-Isothiazolin-4-yl, 3,4-Isothiazolin-4-yl, 4,5-Isothiazolin-4-yl, 2,3-Isothiazolin-5-yl, 3,4-Isothiazolin-5-yl, 4,5-Isothiazolin-5-yl, 2,3-Dihydropyrazol-1-yl, 2,3-Dihydropyrazol-2-yl, 2,3-Dihydropyrazol-3-yl, 2,3-Dihydropyrazol-4-yl, 2,3-Dihydropyrazol-5-yl, 3,4-Dihydropyrazol-1-yl, 3,4-Dihydropyrazol-3-yl, 3,4-Dihydropyrazol-4-yl, 3,4-Dihydropyrazol-5-yl, 4,5-Dihydropyrazol-1-yl, 4,5-Dihydropyrazol-3-yl, 4,5-Dihydropyrazol-4-yl, 4,5-Dihydropyrazol5-yl, 2,3-Dihydrooxazol-2-yl, 2,3-Dihydrooxazol-3-yl, 2,3-Dihydrooxazol-4-yl, 2,3-Dihydrooxazol-5-yl, 3,4-Dihydrooxazol-2-yl, 3,4-Dihydrooxazol-3-yl, 3,4-Dihydrooxazol-4-yl, 3,4-Dihydrooxazol-5-yl, 3,4-Dihydrooxazol-2-yl, 3,4-Dihydrooxazol-3-yl, 3,4-Dihydrooxazol-4-yl, 2-Piperidinyl, 3-Piperidinyl, 4-Piperidinyl, 1,3-Dioxan-5-yl, 2-Tetrahydropyranyl, 4-Tetrahydropyranyl, 2-Tetrahydrothienyl, 3-Tetrahydropyridazinyl, 4-Tetrahydropyridazinyl, 2-Tetrahydropyrimidinyl, 4-Tetrahydropyrimidinyl, 5-Tetrahydropyrimidinyl, 2-Tetrahydropyrazinyl, 1,3,5-Tetrahydro-triazin-2-yl und 1,2,4-Tetrahydrotriazin-3-yl;
**Aryl:** ein ein- bis dreikerniges aromatisches Ringsystem enthaltend 6 bis 14 Kohlenstoffringglieder, z.B. Phenyl, Naphthyl und Anthracenyl;
**Aryloxy:** ein ein- bis dreikerniges aromatisches Ringsystem (wie vorstehend genannt), welches über ein Sauerstoffatom (-O-) an das Gerüst gebunden ist;
**Arylthio:** ein ein- bis dreikerniges aromatisches Ringsystem (wie vorstehend genannt), welches über ein Schwefelatom (-S-) an das Gerüst gebunden ist;
**Arylamino:** ein ein- bis dreikerniges aromatisches Ringsystem (wie vorstehend genannt), welches über eine Aminogruppe (-NH-) an das Gerüst gebunden ist;
**Hetaryl:** aromatisches Ringsystem, welches neben Kohlenstoffringgliedern Heteroatome aus der Gruppe Sauerstoff, Schwefel und Stickstoff enthalten kann: Aryl wie vorstehend genannt oder ein- oder zweikerniges Heteroaryl, z.B.
   - 5-galiedriges Heteroaryl, enthaltend ein bis vier Stickstoffatome oder ein bis drei Stickstoffatome und ein Schwefel- oder Sauerstoffatom: 5-Ring Heteroarylgruppen, welche neben Kohlenstoffatomen ein bis vier Stickstoffatome oder ein bis drei Stickstoffatome und ein Schwefel- oder Sauerstoffatom als Ringglieder enthalten können, z.B. 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl;
   - benzokondensiertes 5-gliedriges Heteroaryl, enthaltend ein bis drei Stickstoffatome oder ein Stickstoffatom und ein Sauerstoff- oder Schwefelatom: 5-Ring Heteroarylgruppen, welche neben Kohlenstoffatomen ein bis vier Stickstoffatome oder ein bis drei Stickstoffatome und ein Schwefel- oder Sauerstoffatom als Ringglieder enthalten können, und in welchen zwei benachbarte Kohlenstoffringglieder oder ein Stickstoff- und ein benachbartes Kohlenstoffringglied durch eine Buta-1,3-dien-1,4-diylgruppe verbrückt sein können;
   - über Stickstoff gebundenes 5-gliedriges Heteroaryl, enthaltend ein bis vier Stickstoffatome, oder über Stickstoff gebundenes benzokondensiertes 5-gliedriges Heteroaryl, enthaltend ein bis drei Stickstoffatome: 5-Ring Heteroarylgruppen, welche neben Kohlenstoffatomen ein bis vier Stickstoffatome bzw. ein bis drei Stickstoffatome als Ringglieder enthalten können, und in welchen zwei benachbarte Kohlenstoffringglieder oder ein Stickstoff- und ein benachbartes Kohlenstoffringglied durch eine Buta-1,3-dien-1,4-diylgruppe verbrückt sein können, wobei diese Ringe über eines der Stickstoffringglieder an das Gerüst gebunden sind;
   - 6-gliedriges Heteroaryl, enthaltend ein bis drei bzw. ein bis vier Stickstoffatome: 6-Ring Heteroarylgruppen, welche neben Kohlenstoffatomen ein bis drei bzw. ein bis vier Stickstoffatome als Ringglieder enthalten können, z.B. 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl;
      Der Zusatz *"ggf. subst."* in Bezug auf *Alkyl-, Alkenyl- und Alkinylgruppen* soll zum Ausdruck bringen, daß diese Gruppen partiell oder vollständig halogeniert sein können *[d.h. die Wasserstoffatome dieser Gruppen können teilweise oder vollständig durch gleiche oder verschiedene Halogenatome wie vorstehend genannt (vorzugsweise Fluor, Chlor oder Brom) ersetzt sein]* und/oder einen bis drei (vorzugsweise einen) der folgenden Reste tragen können:
      - Cyano, Nitro, Hydroxy, Alkoxy, Halogenalkoxy, Alkoxycarbonyl, Alkylcarbonyloxy, wobei die Alkylgruppen in diesen Resten vorzugsweise 1 bis 6 Kohlenstoffatome, insbesondere 1 bis 4 Kohlenstoffatome enthalten;
      - unsubstituiertes oder durch übliche Gruppen substituiertes Cycloalkyl, Cycloalkoxy, Heterocyclyl, Heterocyclyloxy, wobei die cyclischen Systeme 3 bis 12 Ringglieder, vorzugsweise 2 bis 8 Ringglieder, insbesondere 3 bis 6 Ringglieder enthalten und die Alkylgruppen in diesen Resten vorzugsweise 1 bis 6 Kohlenstoffatome, insbesondere 1 bis 4 Kohlenstoffatome enthalten;
      - unsubstituiertes oder durch übliche Gruppen substituiertes Aryl, Aryloxy, Arylalkoxy, Arylalkyl, Hetaryl, Hetaryloxy, Hetarylthio, Hetarylamino, Hetarylalkoxy, Hetarylalkyl, wobei die Arylreste vorzugsweise 6 bis 10 Ringglieder, insbesondere 6 Ringglieder (Phenyl) enthalten, die Hetarylreste insbesondere 5 oder 6 Ringglieder enthalten und die Alkylgruppen in diesen Resten vorzugsweise 1 bis 6 Kohlenstoffatome, insbesondere 1 bis 4 Kohlenstoffatome enthalten.
      Der Zusatz *"ggf. subst"* in Bezug auf die *cyclischen (gesättigten, ungesättigten oder aromatischen) Gruppen* soll zum Ausdruck bringen, daß diese Gruppen partiell oder vollständig halogeniert sein können *[d.h. die Wasserstoffatome dieser Gruppen können teilweise oder vollständig durch gleiche oder verschiedene Halogenatome wie vorstehend genannt (vorzugsweise Fluor, Chlor oder Brom, insbesondere Fluor oder Chlor) ersetzt sein]* und/oder einen bis vier (insbesondere einen bis drei) der folgenden Reste tragen können:
      - Cyano, Nitro, Hydroxy, Mercapto, Amino, Carboxyl, Aminocarbonyl, Aminothiocarbonyl, Alkyl, Haloalkyl, Alkenyl, Alkenyloxy, Alkinyloxy, Alkoxy, Halogenalkoxy, Alkylthio, Alkylamino, Dialkylamino, Formyl, Alkylcarbonyl, Alkylsulfonyl, Alkylsulfoxyl, Alkoxycarbonyl, Alkylcarbonyloxy, Alkylaminocarbonyl, Dialkylaminocarbonyl, Alkylaminothiocarbonyl, Dialkylaminothiocarbonyl, wobei die Alkylgruppen in diesen Resten vorzugsweise 1 bis 6 Kohlenstoffatome, insbesondere 1 bis 4 Kohlenstoffatome enthalten und die genannten Alkenyl- oder Alkinylgruppen in diesen Resten 2 bis 8, vorzugsweise 2 bis 6, insbesondere 2 bis 4 Kohlenstoffatome enthalten;
      und/oder einen bis drei (insbesondere einen) der folgenden Reste:
      - unsubstituiertes oder substituiertes Cycloalkyl, Cycloalkoxy, Heterocyclyl, Heterocyclyloxy, wobei die cyclischen Systeme 3 bis 12 Ringglieder, vorzugsweise 2 bis 8 Ringglieder, insbesondere 3 bis 6 Ringglieder enthalten und die Alkylgruppen in diesen Resten vorzugsweise 1 bis 6 Kohlenstoffatome, insbesondere 1 bis 4 Kohlenstoffatome enthalten;
      - unsubstituiertes oder substituiertes Aryl, Aryloxy, Arylthio, Arylalkoxy, Arylalkyl, Hetaryl, Hetaryloxy, Hetarylthio, wobei die Arylreste vorzugsweise 6 bis 10 Ringglieder, insbesondere 6 Ringglieder (Phenyl) enthalten, die Hetarylreste insbesondere 5 oder 6 Ringglieder enthalten und die Alkylgruppen in diesen Resten vorzugsweise 1 bis 6 Kohlenstoffatome, insbesondere 1 bis 4 Kohlenstoffatome enthalten, die vorstehend genannten cyclischen Gruppen ihrerseits partiell oder vollständig halogeniert sein und/oder 1 bis 3 der folgenden Substituenten tragen können:
      - Cyano, Nitro, Hydroxy, C₁-C₆-Alkyl, C₁-C₆-Halogenalkyl, C₁-C₆-Alkylcarbonyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₂-C₆-Alkenyl, C₂-C₆-Alkenyloxy, C₃-c₆-Alkinyloxy und C₁-C₄-Alkylendioxy, welches halogeniert sein kann,
      und/oder einen oder zwei (insbesondere einen) der folgenden Reste tragen kann:
      - C(=NOR^{d})-Γ₁-R^{d'}, wobei R^{d} für Wasserstoff oder C₁-C₆-Alkyl, Γ für Sauerstoff, Schwefel oder NR^{d} steht und 1 gleich 0 oder 1 ist; und/oder bei denen zwei benachbarte C-Atome der cyclischen Systeme eine Oxy-C₁-C₃-alkylenoxy, Oxy-C₂-C₄-alkenylen-, oder Butadiendiylgruppe tragen können, wobei diese Brücken ihrerseits partiell oder vollständig halogeniert sein können.

Unter *üblichen Gruppen* sind insbesondere die folgenden Substituenten zu verstehen: Cyano, Halogen, Nitro, Hydroxy, C₁-C₄-Alkyl, C₁-C₄-Halogenalkyl, C₁-C₄-Alkoxy und C₁-C₄-Halogenalkoxy.

Im Hinblick auf ihre bestimmungsgemäße Verwendung der Phenylverbindungen der Formel I sind die folgenden Bedeutungen der Substituenten, und zwar jeweils für sich allein oder in Kombination, besonders bevorzugt:

Verbindungen der Formel I, in denen T Oxymethylen bedeutet, sind besonders bevorzugt.

Daneben besonders bevorzugt sind Verbindungen der Formel I, in denen n = null (0) ist.

Gleichermaßen besonders bevorzugt sind Verbindungen I, in denen Z für N=C(R¹)-C(R²)=NOR³ steht.

Daneben werden Verbindungen I besonders bevorzugt, in denen Z für N=CWR¹ steht.

Außerdem werden Verbindungen I besonders bevorzugt, in denen W für gegebenenfalls substituiertes Phenyl steht.

Insbesondere werden auch Verbindungen I bevorzugt, in denen Z für eine Gruppe X steht.

Außerdem werden Verbindungen I besonders bevorzugt, in denen R² für eine Gruppe W steht.

Gleichermaßen besonders bevorzugt sind Verbindungen I, in denen R² für eine Gruppe W, die über Sauerstoff gebunden ist, steht.

Daneben werden Verbindungen I besonders bevorzugt, in denen X für ggf. subst. Aryl oder ggf. subst. Hetaryl steht.

Desweiteren werden Verbindungen I besonders bevorzugt, in denen R¹ für Methyl oder Ethyl steht.

Außerdem werden Verbindungen I besonders bevorzugt, in denen R³ für C₁-C₃-Alkyl, C₃-C₅-Alkenyl oder C₃-C₅-Alkinyl steht.

Daneben werden Verbindungen I besonders bevorzugt, in denen R³ für Methyl, Allyl oder Propargyl steht.

Weiterhin besonders bevorzugt sind Verbindungen der Formel I, in denen T Sauerstoff oder Oxymethylen, Z eine Gruppe X, N=CWR¹ oder N=C(R¹)-C(R²)=NOR³ bedeutet, wobei
- X: Heterocyclyl, welches vollständig oder partiell halogeniert sein und/oder 1 bis 3 der folgenden Reste tragen kann:
Cyano, C₁-C₄-Alkyl, C₁-C₄-Halogenalkyl oder C₁-C₄-Alkoxy; Aryl, Hetaryl, Arylmethylen oder Hetarylmethylen, wobei die cyclischen Reste partiell oder vollständig halogeniert sein und/oder eine bis drei der folgenden Gruppen tragen können:
Cyano, Nitro, Hydroxy, Mercapto, Amino, Carboxyl, Aminocarbonyl, Aminothiocarbonyl, C₁-C₆-Alkyl, C₁-C₆-Halogenalkyl, C₁-C₆-Alkylcarbonyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Arylthio, Heteroaryl, Heteroaryloxy, Heteroarylthio, C₃-C₆-Alkinyloxy, C₁-C₄-Alkylendioxy, welches halogeniert sein kann, oder C(=NOR^{d})-Γ₁-R^{d'}, wobei
   R^{d} für Wasserstoff oder C₁-C₆-Alkyl;
   Γ für Sauerstoff, Schwefel oder NR^{d} steht;
   1 gleich 0 oder 1 ist und
   die cyclischen Gruppen ihrerseits partiell oder vollständig halogeniert sein und/oder 1 bis 3 der folgenden Substituenten tragen können: Cyano, Nitro, Hydroxy, C₁-C₆-Alkyl, C₁-C₆-Halogenalkyl, C₁-C₆-Alkylcarbonyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₂-C₆-Alkenyl, C₂-C₆-Alkenyloxy, C₃-C₆-Alkinyloxy und C₁-C₄-Alkylendioxy, welches halogeniert sein kann, steht,
- W: für C₁-C₆-Alkyl, C₂-C₆-Alkenyl oder C₂-C₆-Alkinyl,
wobei diese Gruppen vollständig oder partiell halogeniert sein und/oder 1 bis 3 der folgenden Reste tragen können:
Cyano, C₁-C₆-Alkoxy, C₁-C₆-Halogenalkoxy, C₁-C₆-Alkoxycarbonyl, C₃-C₆-Cycloalkyl, Heterocyclyl, Aryl oder Hetaryl, wobei
die cyclischen Gruppen ihrerseits partiell oder vollständig halogeniert sein und/oder 1 bis 3 der folgenden Reste tragen können:
   Cyano, Nitro, C₁-C₄-Alkyl, C₁-C₄-Halogenalkyl, C₁-C₄-Alkoxy, C₁-C₄-Halogenalkoxy; oder
   für C₃-C₆-Cycloalkyl, C₃-C₆-Cycloalkenyl oder Heterocyclyl, wobei diese Gruppen vollständig oder partiell halogeniert sein und/oder 1 bis 3 der folgenden Reste tragen können:
Cyano, C₁-C₄-Alkyl, C₁-C₄-Halogenalkyl oder C₁-C₄-Alkoxy; oder
für Aryl oder Heteroaryl,
wobei diese Reste partiell oder vollständig halogeniert sein und/oder eine bis drei der folgenden Gruppen tragen können:
Cyano, Nitro, Hydroxy, Mercapto, Amino, Carboxyl, Aminocarbonyl, Aminothiocarbonyl, C₁-C₆-Alkyl, C₁-C₆-Halogenalkyl, C₁-C₆-Alkylcarbonyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Arylthio, Heteroaryl, Heteroaryloxy, Heteroarylthio, C₃-C₆-Alkinyloxy, C₁-C₄-Alkylendioxy, welches halogeniert sein kann, oder C(=NOR^{d})-Γ₁-R^{d'}, wobei

R^{d} für Wasserstoff oder C₁-C₆-Alkyl;
Γ für Sauerstoff, Schwefel oder NR^{d} steht;
1 gleich 0 oder 1 ist und
die cyclischen Gruppen ihrerseits partiell oder vollständig halogeniert sein und/oder 1 bis 3 der folgenden Substituenten tragen können:
Cyano, Nitro, Hydroxy, C₁-C₆-Alkyl, C₁-C₆-Halogenalkyl, C₁-C₆-Alkylcarbonyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₂-C₆-Alkenyl, C₂-C₆-Alkenyloxy, C₃-C₆-Alkinyloxy und C₁-C₄-Alkylendioxy, welches halogeniert sein kann, steht;
- R²: Wasserstoff, Cyano, Halogen, C(R^{d})=NOR³ oder W, OW, SW oder NR^{c}W, wobei
- R^{c}: Wasserstoff, C₁-C₆-Alkyl, C₂-C₆-Alkenyl oder C₂-C₆-Alkinyl;
- R^{d}: Wasserstoff oder C₁-C₄-Alkyl; und
- R³: Wasserstoff, C₁-C₆-Alkyl, C₁-C₆-Cyanoalkyl, C₂-C₆-Alkenyl, C₂-C₆-Alkinyl, C₁-C₄-Alkoxy-C₁-C₆-alkyl oder C₃-C₆-Cycloalkyl-C₁-C₄-alkyl, wobei
diese Gruppen partiell oder vollständig halogeniert sein können und die Cycloalkylgruppen zusätzlich 1 bis 3 C₁-C₄-Alkylreste tragen können; bedeuten.

Die besonders bevorzugten Ausführungsformen der Zwischenprodukte in Bezug auf die Variablen entsprechen denen der Reste T, Z, W, R^{α}, R^{β},Yₙ, R¹, R² und R³ der Formel I.

Darüber hinaus sind Verbindungen der Formel IA besonders bevorzugt, in denen R^{α} C₁-C₄-Alkyl oder C₁-C₄-Halogenalkyl bedeutet.

Insbesondere werden Verbindungen IA bevorzugt, in denen R^{α} für Methyl steht.

Gleichermaßen besonders bevorzugt sind Verbindungen IA, in denen R^{α} für Trifluormethyl steht.

Insbesondere werden Verbindungen IA.1' bevorzugt, in denen R^{α} für Methyl, Ethyl, Trifluormethyl oder Cyclopropyl und Z für gegebenenfalls substituiertes Phenyl, Pyridinyl, Pyrimidinyl, Chinazolinyl, Furanyl, Thienyl, Pyrrolyl, Benzofuranyl, Benzothiophenyl, Indolyl, Pyrazolyl, Isoxazolyl, Isothiazolyl, Oxazolyl, Thiazolyl, Imidazolyl, Triazolyl, Tetrazolyl, Thiadiazolyl, Benzoxazolyl, Benzisoxazolyl, Benzthiazolyl, Benzisothiazolyl oder Indazolyl steht.

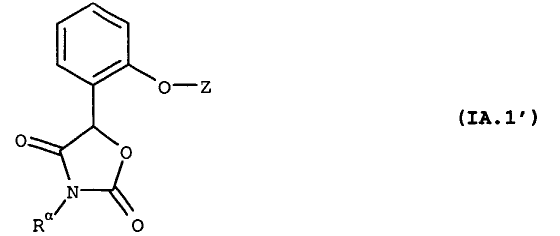

Daneben werden Verbindungen IA.1' besonders bevorzugt, in denen Z für durch Halogen, Methyl, Trifluormethyl oder Methoxy substituiertes Phenyl steht.

Außerdem werden Verbindungen IA.1'' bevorzugt, in denen R^{α} für Methyl, Ethyl, Trifluormethyl oder Cyclopropyl und Z für gegebenenfalls substituiertes Phenyl, Naphthyl, Pyridinyl, Pyrimidinyl, Chinolyl, Triazolyl, Pyrazinyl, Thienyl, Chinoxalinyl, Benzoxazolyl, Benzthiazolyl oder Pyrazolyl steht.

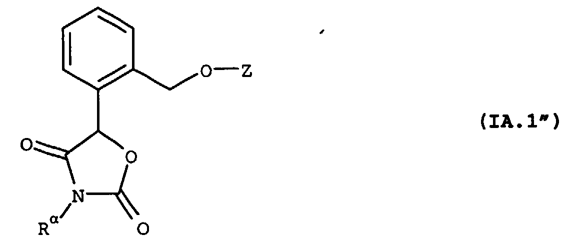

Weiterhin werden Verbindungen IA.1'' besonders bevorzugt, in denen Z für durch Halogen, Methyl, Trifluormethyl oder C(=NOR^{d})R^{d'} substituiertes Phenyl steht.

Gleichermaßen bevorzugt sind Verbindungen IA.2', in denen R^{α} für Methyl, Ethyl, Trifluormethyl oder Cyclopropyl und Z für gegebenenfalls substituiertes Phenyl, Naphthyl, Pyridinyl, Pyrimidinyl, Chinolyl, Pyrazinyl, Thienyl, Chinoxalinyl, Benzoxazolyl, Benzthiazolyl oder Pyrazolyl steht.

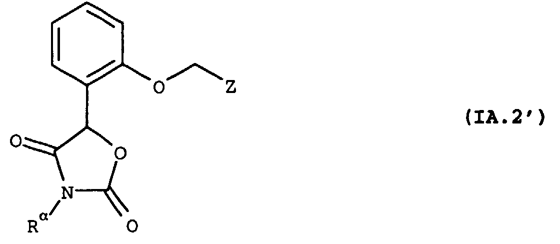

Darüber hinaus werden Verbindungen IA.2' besonders bevorzugt, in denen Z für durch Halogen, Methyl, Trifluormethyl oder C(=NOR^{d})R^{d'} substituiertes Phenyl steht.

Gleichermaßen besonders bevorzugt sind Verbindungen IA.3', in denen R^{α} für Methyl, Ethyl, Trifluormethyl oder Cyclopropyl, W für ggf. subst. Phenyl, Naphthyl, Anthryl, Benzyl, Phenylethyl, Phenylpropyl, Pyridinyl, Pyrimidinyl, Thienyl, Furyl, Thiazolyl, Benzothiazolyl, Dioxanyl, C₃-C₆-Cycloalkyl, sowie C₁-C₄-Alkyl, Allyl, Propargyl oder Trifluormethyl und R¹ für Methyl oder Methoxy steht.

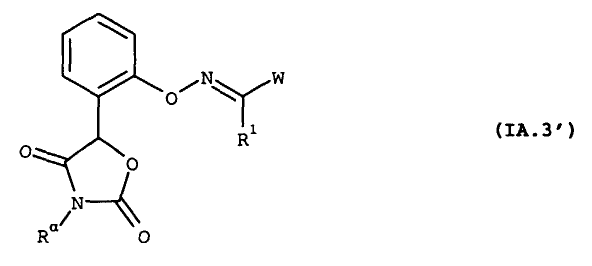

Daneben werden Verbindungen IA.3'' bevorzugt, in denen R^{α} für Methyl, Ethyl, Trifluormethyl oder Cyclopropyl, W für ggf. subst. Phenyl, Naphthyl, Anthryl, Benzyl, Phenylethyl, Phenylpropyl, Pyridinyl, Pyrimidinyl, Thienyl, Furyl, Thiazolyl, Benzothiazolyl, Dioxanyl, C₃-C₆-Cycloalkyl, sowie C₁-C₄-Alkyl, Allyl, Propargyl oder Trifluormethyl und R¹ für Methyl oder Methoxy steht.

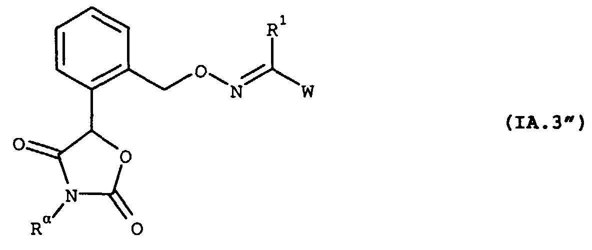

Desweiteren werden Verbindungen IA.4' bevorzugt, in denen R^{α} für Methyl, Ethyl, Trifluormethyl oder Cyclopropyl, R¹ für Methyl oder Methoxy, R² für Halogen, Cyano, C(=NOR^{d})R^{d'}, ggf. subst. C₁-C₄-Alkyl, C₁-C₄-Cycloalkyl, C₁-C₄-Halogenalkyl, C₁-C₄-Alkoxy, C₁-C₄-Alkylthio, C₁-C₄-Cycloalkoxy, C₁-C₄-Alkylamino, C₂-C₄-Alkenyl oder Phenyl und R³ für Wasserstoff, Propargyl oder Allyl oder ggf. subst. C₁-C₄-Alkyl steht.

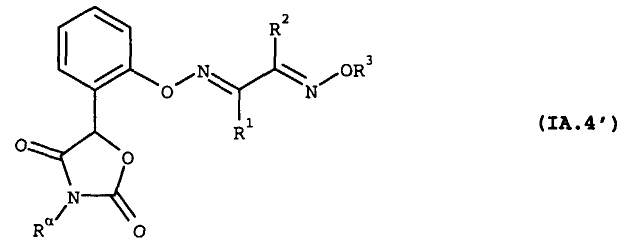

Außerdem werden Verbindungen IA.4'' bevorzugt, in denen R^{α} für Methyl, Ethyl, Trifluormethyl oder Cyclopropyl, R¹ für Methyl oder Methoxy, R² für Halogen, Cyano, C(=NOR^{d})R^{d'}, ggf. subst. C₁-C₄-Alkyl, C₁-C₄-Cycloalkyl, C₁-C₄-Halogenalkyl, C₁-C₄-Alkoxy, C₁-C₄-Alkylthio, C₁-C₄-Cycloalkoxy, C₁-C₄-Alkylamino, C₂-C₄-Alkenyl oder Phenyl und R³ für Wasserstoff, Propargyl oder Allyl oder ggf. subst. C₁-C₄-Alkyl steht.

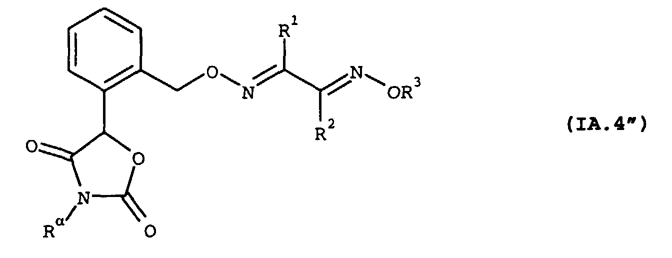

Desweiteren werden Verbindungen IA.5' bevorzugt, in denen R^{α} für Methyl, Ethyl, Trifluormethyl oder Cyclopropyl, W für ggf. subst. Phenyl, Naphthyl, Anthryl, Benzyl, Phenylethyl, Phenylpropyl, Pyridinyl, Pyrimidinyl, Thienyl, Furyl, Thiazolyl, Benzothiazolyl, Dioxanyl, C₃-C₆-Cycloalkyl, sowie C₁-C₄-Alkyl, Allyl, Propargyl oder Trifluormethyl steht.

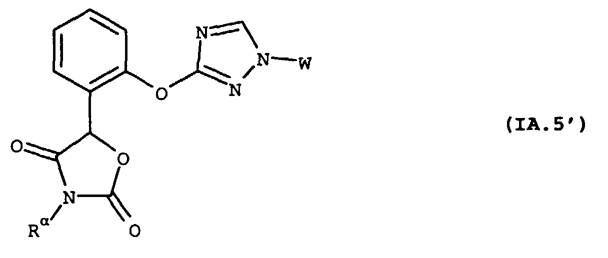

Insbesondere werden Verbindungen IA.5'' bevorzugt, in denen R^{α} für Methyl, Ethyl, Trifluormethyl oder Cyclopropyl, W für ggf. subst. Phenyl, Naphthyl, Anthryl, Benzyl, Phenylethyl, Phenylpropyl, Pyridinyl, Pyrimidinyl, Thienyl, Furyl, Thiazolyl, Benzothiazolyl, Dioxanyl, C₃-C₆-Cycloalkyl, sowie C₁-C₄-Alkyl, Allyl, Propargyl oder Trifluormethyl steht.

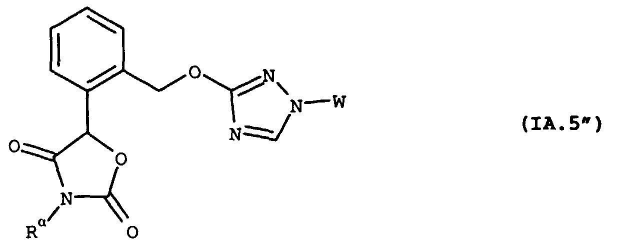

Außerdem werden Verbindungen IA.6' bevorzugt, in denen R^{α} für Methyl, Ethyl, Trifluormethyl oder Cyclopropyl, W für ggf. subst. Phenyl, Naphthyl, Anthryl, Benzyl, Phenylethyl, Phenylpropyl, Pyridinyl, Pyrimidinyl, Thienyl, Furyl, Thiazolyl, Benzothiazolyl, Dioxanyl, C₃-C₆-Cycloalkyl, sowie C₁-C₄-Alkyl, Allyl, Propargyl oder Trifluormethyl steht.

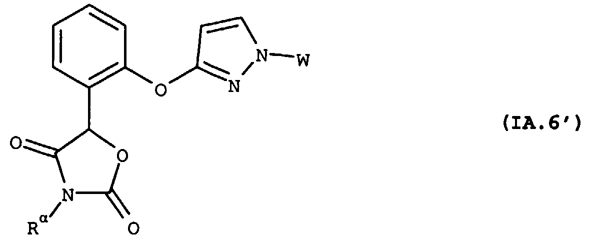

Gleichermaßen bevorzugt sind Verbindungen IA.6'', in denen R^{α} für Methyl, Ethyl, Trifluormethyl oder Cyclopropyl, W für ggf. subst. Phenyl, Naphthyl, Anthryl, Benzyl, Phenylethyl, Phenylpropyl, Pyridinyl, Pyrimidinyl, Thienyl, Furyl, Thiazolyl, Benzothiazolyl, Dioxanyl, C₃-C₆-Cycloalkyl, sowie C₁-C₄-Alkyl, Allyl, Propargyl oder Trifluormethyl steht.

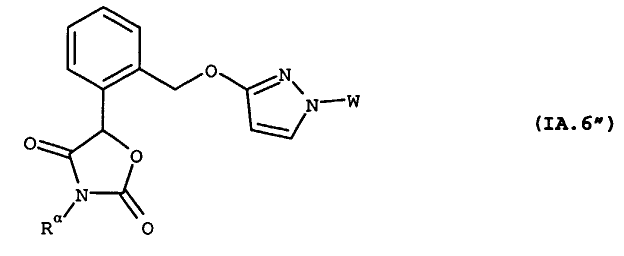

Daneben werden Verbindungen IA.7' bevorzugt, in denen R^{α} für Methyl, Ethyl, Trifluormethyl oder Cyclopropyl, W für ggf. subst. Phenyl, Naphthyl, Anthryl, Benzyl, Phenylethyl, Phenylpropyl, Pyridinyl, Pyrimidinyl, Thienyl, Furyl, Thiazolyl, Benzothiazolyl, Dioxanyl, C₃-C₆-Cycloalkyl, sowie C₁-C₄-Alkyl, Allyl, Propargyl oder Trifluormethyl steht.

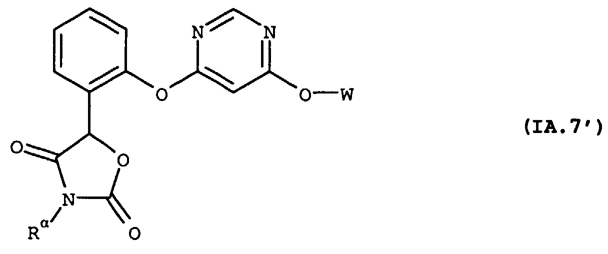

Außerdem werden auch Verbindungen IA.8' bevorzugt, in denen R^{α} für Methyl, Ethyl, Trifluormethyl oder Cyclopropyl, T für Stickstoff oder Kohlenstoff und W für ggf. subst. Phenyl, Naphthyl, Anthryl, Benzyl, Phenylethyl, Phenylpropyl, Pyridinyl, Pyrimidinyl, Thienyl, Furyl, Thiazolyl, Benzothiazolyl, Dioxanyl, C₃-C₆-Cycloalkyl, sowie C₁-C₄-Alkyl, Allyl, Propargyl oder Trifluormethyl steht.

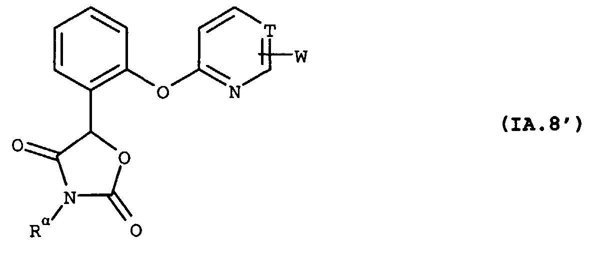

Insbesondere werden Verbindungen IB bevorzugt, in denen R^{α} für Methyl, Ethyl, Trifluormethyl oder Cyclopropyl steht.

Daneben werden Verbindungen IB bevorzugt, in denen R^{β} für Wasserstoff, Methyl, Ethyl, Trifluormethyl oder Cyclopropyl steht.

Desweiteren werden Verbindungen IB besonders bevorzugt, in denen R^{β} für Benzyl steht.
Gleichermaßen besonders bevorzugt sind Verbindungen IB, in denen n null ist und R^{α} und R^{β} für C₁-C₄-Alkyl stehen.

Insbesondere werden Verbindungen IB.1' bevorzugt, in denen R^{α} für Methyl, Ethyl, Trifluormethyl oder Cyclopropyl, R^{β} für Wasserstoff, Methyl, Ethyl, Trifluormethyl, Cyclopropyl oder Benzyl und Z für gegebenenfalls substituiertes Phenyl, Pyridinyl, Pyrimidinyl, Chinazolinyl, Furanyl, Thienyl, Pyrrolyl, Benzofuranyl, Benzothiophenyl, Indolyl, Pyrazolyl, Isoxazolyl, Isothiazolyl, Oxazolyl, Thiazolyl, Imidazolyl, Triazolyl, Tetrazolyl, Thiadiazolyl, Benzoxazolyl, Benzisoxazolyl, Benzthiazolyl, Benzisothiazolyl oder Indazolyl steht.

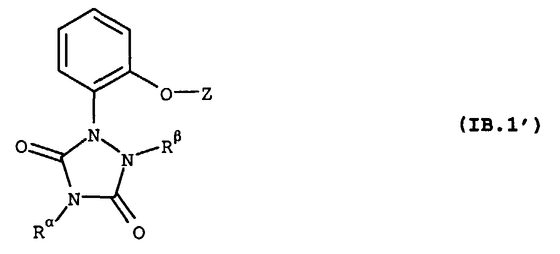

Daneben werden Verbindungen IB.1' besonders bevorzugt, in denen Z für durch Halogen, Methyl, Trifluormethyl oder Methoxy substituiertes Phenyl steht.

Außerdem werden Verbindungen IB.1'' bevorzugt, in denen R^{α} für Methyl, Ethyl, Trifluormethyl oder Cyclopropyl, R^{β} für Wasserstoff, Methyl, Ethyl, Trifluormethyl, Cyclopropyl oder Benzyl und Z für gegebenenfalls substituiertes Phenyl, Naphthyl, Pyridinyl, Pyrimidinyl, Chinolyl, Triazolyl, Pyrazinyl, Thienyl, Chinoxalinyl, Benzoxazolyl, Benzthiazolyl oder Pyrazolyl steht.

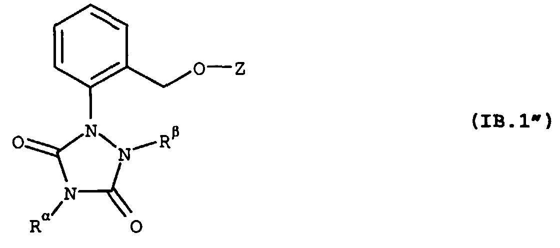

Weiterhin werden Verbindungen IB.1'' besonders bevorzugt, in denen Z für durch Halogen, Methyl, Trifluormethyl oder C(=NOR^{d})R^{d'} substituiertes Phenyl steht.

Gleichermaßen bevorzugt sind Verbindungen IB.2', in denen R^{α} für Methyl, Ethyl, Trifluormethyl oder Cyclopropyl, R^{β} für Wasserstoff, Methyl, Ethyl, Trifluormethyl, Cyclopropyl oder Benzyl und Z für gegebenenfalls substituiertes Phenyl, Naphthyl, Pyridinyl, Pyrimidinyl, Chinolyl, Pyrazinyl, Thienyl, Chinoxalinyl, Benzoxazolyl, Benzthiazolyl oder Pyrazolyl steht.

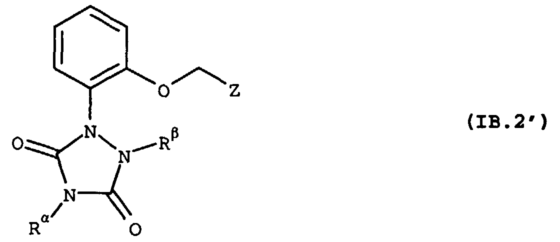

Darüber hinaus werden Verbindungen IB.2' besonders bevorzugt, in denen Z für durch Halogen, Methyl, Trifluormethyl oder C(=NOR^{d})R^{d'} substituiertes Phenyl steht.

Gleichermaßen besonders bevorzugt sind Verbindungen IB.3', in denen R^{α} für Methyl, Ethyl, Trifluormethyl oder Cyclopropyl, R^{β} für Wasserstoff, Methyl, Ethyl, Trifluormethyl, Cyclopropyl oder Benzyl, W für ggf. subst. Phenyl, Naphthyl, Anthryl, Benzyl, Phenylethyl, Phenylpropyl, Pyridinyl, Pyrimidinyl, Thienyl, Furyl, Thiazolyl, Benzothiazolyl, Dioxanyl, C₃-C₆-Cycloalkyl, sowie C₁-C₄-Alkyl, Allyl, Propargyl oder Trifluormethyl und R¹ für Methyl oder Methoxy steht.

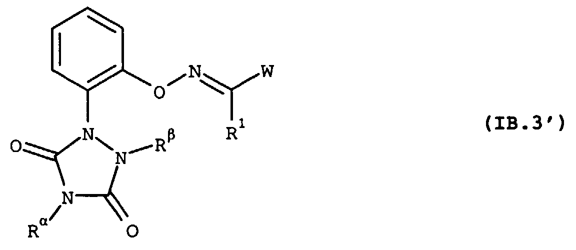

Daneben werden Verbindungen IB.3'' bevorzugt, in denen R^{α} für Methyl, Ethyl, Trifluormethyl oder Cyclopropyl, R^{β} für Wasserstoff, Methyl, Ethyl, Trifluormethyl, Cyclopropyl oder Benzyl, W für ggf. subst. Phenyl, Naphthyl, Anthryl, Benzyl, Phenylethyl, Phenylpropyl, Pyridinyl, Pyrimidinyl, Thienyl, Furyl, Thiazolyl, Benzothiazolyl, Dioxanyl, C₃-C₆-Cycloalkyl, sowie C₁-C₄-Alkyl, Allyl, Propargyl oder Trifluormethyl und R¹ für Methyl oder Methoxy steht.

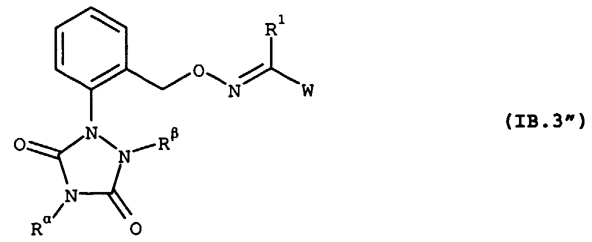

Desweiteren werden Verbindungen IB.4' bevorzugt, in denen R^{α} für Methyl, Ethyl, Trifluormethyl oder Cyclopropyl, R^{β} für Wasserstoff, Methyl, Ethyl, Trifluormethyl, Cyclopropyl oder Benzyl, R¹ für Methyl oder Methoxy, R² für Halogen, Cyano, C(=NOR^{d})R^{d'}, ggf. subst. C₁-C₄-Alkyl, C₁-C₄-Cycloalkyl, C₁-C₄-Halogenalkyl, C₁-C₄-Alkoxy, C₁-C₄-Alkylthio, C₁-C₄-Cycloalkoxy, C₁-C₄-Alkylamino, C₂-C₄-Alkenyl oder Phenyl und R³ für Wasserstoff, Propargyl oder Allyl oder ggf. subst. C₁-C₄-Alkyl steht.

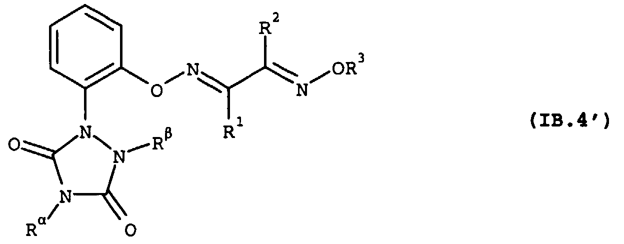

Außerdem werden Verbindungen IB.4'' bevorzugt, in denen R^{α} für Methyl, Ethyl, Trifluormethyl oder Cyclopropyl, R^{β} für Wasserstoff, Methyl, Ethyl, Trifluormethyl, Cyclopropyl oder Benzyl, R¹ für Methyl oder Methoxy, R² für Halogen, Cyano, C(=NOR^{d})R^{d'}, ggf. subst. C₁-C₄-Alkyl, C₁-C₄-Cycloalkyl, C₁-C₄-Halogenalkyl, C₁-C₄-Alkoxy, C₁-C₄-Alkylthio, C₁-C₄-Cycloalkoxy, C₁-C₄-Alkylamino, C₂-C₄-Alkenyl oder Phenyl und R³ für Wasserstoff, Propargyl oder Allyl oder ggf. subst. C₁-C₄-Alkyl steht.

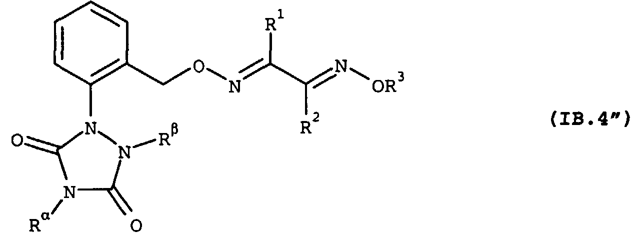

Desweiteren werden Verbindungen IB.5' bevorzugt, in denen R^{α} für Methyl, Ethyl, Trifluormethyl oder Cyclopropyl, R^{β} für Wasserstoff, Methyl, Ethyl, Trifluormethyl, Cyclopropyl oder Benzyl, W für ggf. subst. Phenyl, Naphthyl, Anthryl, Benzyl, Phenylethyl, Phenylpropyl, Pyridinyl, Pyrimidinyl, Thienyl, Furyl, Thiazolyl, Benzothiazolyl, Dioxanyl, C₃-C₆-Cycloalkyl, sowie C₁-C₄-Alkyl, Allyl, Propargyl oder Trifluormethyl steht.

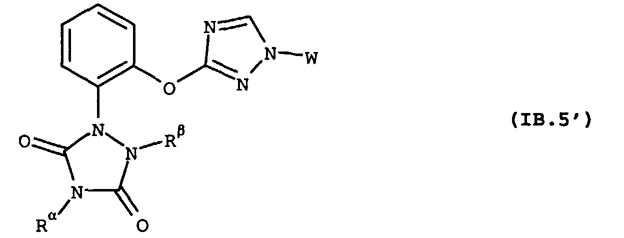

Insbesondere werden Verbindungen IB.5'' bevorzugt, in denen R^{α} für Methyl, Ethyl, Trifluormethyl oder Cyclopropyl, R^{β} für Wasserstoff, Methyl, Ethyl, Trifluormethyl, Cyclopropyl oder Benzyl, W für ggf. subst. Phenyl, Naphthyl, Anthryl, Benzyl, Phenylethyl, Phenylpropyl, Pyridinyl, Pyrimidinyl, Thienyl, Furyl, Thiazolyl, Benzothiazolyl, Dioxanyl, C₃-C₆-Cycloalkyl, sowie C₁-C₄-Alkyl, Allyl, Propargyl oder Trifluormethyl steht.

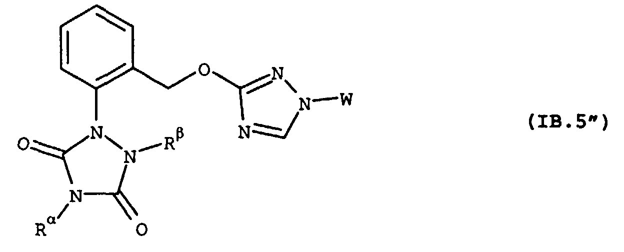

Außerdem werden Verbindungen IB.6' bevorzugt, in denen R^{α} für Methyl, Ethyl, Trifluormethyl oder Cyclopropyl, R^{β} für Wasserstoff, Methyl, Ethyl, Trifluormethyl, Cyclopropyl oder Benzyl, W für ggf. subst. Phenyl, Naphthyl, Anthryl, Benzyl, Phenylethyl, Phenylpropyl, Pyridinyl, Pyrimidinyl, Thienyl, Furyl, Thiazolyl, Benzothiazolyl, Dioxanyl, C₃-C₆-Cycloalkyl, sowie C₁-C₄-Alkyl, Allyl, Propargyl oder Trifluormethyl steht.

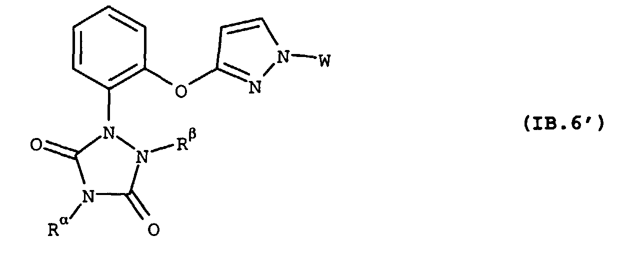

Gleichermaßen bevorzugt sind Verbindungen IB.6'', in denen R^{α} für Methyl, Ethyl, Trifluormethyl oder Cyclopropyl, R^{β} für Wasserstoff, Methyl, Ethyl, Trifluormethyl, Cyclopropyl oder Benzyl, W für ggf. subst. Phenyl, Naphthyl, Anthryl, Benzyl, Phenylethyl, Phenylpropyl, pyridinyl, Pyrimidinyl, Thienyl, Furyl, Thiazolyl, Benzothiazolyl, Dioxanyl, C₃-C₆-Cycloalkyl, sowie C₁-C₄-Alkyl, Allyl, Propargyl oder Trifluormethyl steht.

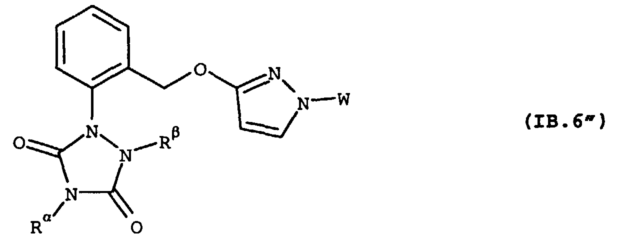

Daneben werden Verbindungen IB.7' bevorzugt, in denen R^{α} für Methyl, Ethyl, Trifluormethyl oder Cyclopropyl, R^{β} für Wasserstoff, Methyl, Ethyl, Trifluornethyl, Cyclopropyl oder Benzyl, W für ggf. subst. Phenyl, Naphthyl, Anthryl, Benzyl, Phenylethyl, Phenylpropyl, Pyridinyl, Pyrimidinyl, Thienyl, Furyl, Thiazolyl, Benzothiazolyl, Dioxanyl, C₃-C₆-Cycloalkyl, sowie C₁-C₄-Alkyl, Allyl, Propargyl oder Trifluormethyl steht.

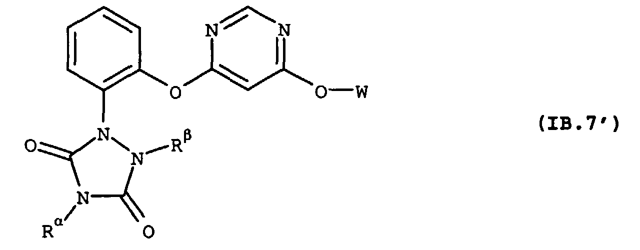

Außerdem werden auch Verbindungen IB.8' bevorzugt, in denen R^{α} für Methyl, Ethyl, Trifluormethyl oder Cyclopropyl, R^{β} für Wasserstoff, Methyl, Ethyl, Trifluormethyl, Cyclopropyl oder Benzyl, T für Stickstoff oder Kohlenstoff und W für ggf. subst. Phenyl, Naphthyl, Anthryl, Benzyl, Phenylethyl, Phenylpropyl, Pyridinyl, Pyrimidinyl, Thienyl, Furyl, Thiazolyl, Benzothiazol, Dioxanyl, C₃-C₆-Cycloalkyl, sowie C₁-C₄-Alkyl, Allyl, Propargyl oder Trifluormethyl steht.

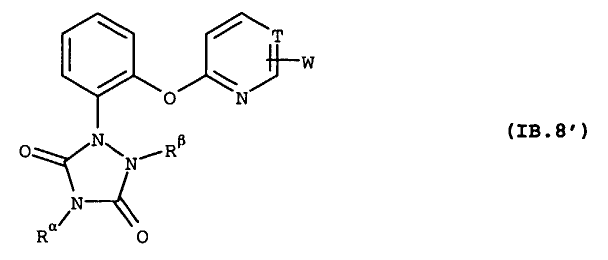

Insbesondere sind im Hinblick auf ihre Verwendung die in den folgenden Tabellen zusammengestellten Verbindungen I bevorzugt. Die in den Tabellen für einen Substituenten genannten Gruppen stellen außerdem für sich betrachtet, unabhängig von der Kombination, in der sie genannt sind, eine besonders bevorzugte Ausgestaltung des betreffenden Substituenten dar.
Tabelle 1
   Verbindungen der allgemeinen Formel IA.1', in denen R^{α} für Methyl steht und Z für eine Verbindung jeweils einer Zeile der Tabelle A entspricht
Tabelle 2
   Verbindungen der allgemeinen Formel IA.1'', in denen R^{α} für Methyl steht und Z für eine Verbindung jeweils einer Zeile der Tabelle B entspricht
Tabelle 3
   Verbindungen der allgemeinen Formel IA.2', in denen R^{α} für Methyl steht, und Z für eine Verbindung jeweils einer Zeile der Tabelle B entspricht
Tabelle 4
   Verbindungen der allgemeinen Formel IA.3', in denen R^{α} für Methyl steht, R¹ Methyl bedeutet und W für eine Verbindung jeweils einer Zeile der Tabelle C entspricht
Tabelle 5
   Verbindungen der allgemeinen Formel IA.3'', in denen R^{α} für Methyl steht, R¹ Methyl bedeutet und W für eine Verbindung jeweils einer Zeile der Tabelle C entspricht
Tabelle 6
   Verbindungen der allgemeinen Formel IA.4', in denen R^{α} für Methyl, R¹ für Methyl steht und die Kombination der Reste R² und R³ für eine Verbindung jeweils einer Zeile der Tabelle D entspricht
Tabelle 7
   Verbindungen der allgemeinen Formel IA.4', in denen R^{α} für Methyl, R¹ für Methoxy steht und die Kombination der Reste R² und R³ für eine Verbindung jeweils einer Zeile der Tabelle D entspricht
Tabelle 8
   Verbindungen der allgemeinen Formel IA.4'', in denen R^{α} für Methyl, R¹ für Methyl steht und die Kombination der Reste R² und R³ für eine Verbindung jeweils einer Zeile der Tabelle D entspricht
Tabelle 9
   Verbindungen der allgemeinen Formel IA.4'', in denen R^{α} für Methyl, R¹ für Methoxy steht und die Kombination der Reste R² und R³ für eine Verbindung jeweils einer Zeile der Tabelle D entspricht
Tabelle 10
   Verbindungen der allgemeinen Formel IA.5'', in denen R^{α} für Methyl steht und W für eine Verbindung jeweils einer Zeile der Tabelle C entspricht
Tabelle 11
   Verbindungen der allgemeinen Formel IA.6', in denen R^{α} für Methyl steht und W für eine Verbindung jeweils einer Zeile der Tabelle C entspricht
Tabelle 12
   Verbindungen der allgemeinen Formel IA.6'', in denen R^{α} für Methyl steht und W für eine Verbindung jeweils einer Zeile der Tabelle C entspricht
Tabelle 13
   Verbindungen der allgemeinen Formel IA.7', in denen R^{α} für Methyl steht und W für eine Verbindung jeweils einer Zeile der Tabelle C entspricht
Tabelle 14
   Verbindungen der allgemeinen Formel IA.1', in denen R^{α} für Ethyl steht und Z für eine Verbindung jeweils einer Zeile der Tabelle A entspricht
Tabelle 15
   Verbindungen der allgemeinen Formel IA.1'', in denen R^{α} für Ethyl steht und Z für eine Verbindung jeweils einer Zeile der Tabelle B entspricht
Tabelle 16
   Verbindungen der allgemeinen Formel IA.2', in denen R^{α} für Ethyl steht, und Z für eine Verbindung jeweils einer Zeile der Tabelle B entspricht
Tabelle 17
   Verbindungen der allgemeinen Formel IA.3', in denen R^{α} für Ethyl steht, R¹ Methyl bedeutet und W für eine Verbindung jeweils einer Zeile der Tabelle C entspricht
Tabelle 18
   Verbindungen der allgemeinen Formel IA.3'', in denen R^{α} für Ethyl steht, R¹ Methyl bedeutet und W für eine Verbindung jeweils einer Zeile der Tabelle C entspricht
Tabelle 19
   Verbindungen der allgemeinen Formel IA.4', in denen R^{α} für Ethyl, R¹ für Methyl steht und die Kombination der Reste R² und R³ für eine Verbindung jeweils einer Zeile der Tabelle D entspricht
Tabelle 20
   Verbindungen der allgemeinen Formel IA.4', in denen R^{α} für Ethyl, R¹ für Methoxy steht und die Kombination der Reste R² und R³ für eine Verbindung jeweils einer Zeile der Tabelle D entspricht
Tabelle 21
   Verbindungen der allgemeinen Formel IA.4'', in denen R^{α} für Ethyl, R¹ für Methyl steht und die Kombination der Reste R² und R³ für eine Verbindung jeweils einer Zeile der Tabelle D entspricht
Tabelle 22
   Verbindungen der allgemeinen Formel IA.4'', in denen R^{α} für Ethyl, R¹ für Methoxy steht und die Kombination der Reste R² und R³ für eine Verbindung jeweils einer Zeile der Tabelle D entspricht
Tabelle 23
   Verbindungen der allgemeinen Formel IA.5'', in denen R^{α} für Ethyl steht und W für eine Verbindung jeweils einer Zeile der Tabelle C entspricht
Tabelle 24
   Verbindungen der allgemeinen Formel IA.6', in denen R^{α} für Ethyl steht und W für eine Verbindung jeweils einer Zeile der Tabelle C entspricht
Tabelle 25
   Verbindungen der allgemeinen Formel IA.6'', in denen R^{α} für Ethyl steht und W für eine Verbindung jeweils einer Zeile der Tabelle C entspricht
Tabelle 26
   Verbindungen der allgemeinen Formel IA.7', in denen R^{α} für Ethyl steht und W für eine Verbindung jeweils einer Zeile der Tabelle C entspricht
Tabelle 27
   Verbindungen der allgemeinen Formel IA.1', in denen R^{α} für Trifluormethyl steht und Z für eine Verbindung jeweils einer Zeile der Tabelle A entspricht
Tabelle 28
   Verbindungen der allgemeinen Formel IA.1'', in denen R^{α} für Trifluormethyl steht und Z für eine Verbindung jeweils einer Zeile der Tabelle B entspricht
Tabelle 29
   Verbindungen der allgemeinen Formel IA.2', in denen R^{α} für Trifluormethyl steht, und Z für eine Verbindung jeweils einer Zeile der Tabelle B entspricht
Tabelle 30
   Verbindungen der allgemeinen Formel IA.3', in denen R^{α} für Trifluormethyl steht, R¹ Methyl bedeutet und W für eine Verbindung jeweils einer Zeile der Tabelle C entspricht
Tabelle 31
   Verbindungen der allgemeinen Formel IA.3'', in denen R^{α} für Trifluormethyl steht, R¹ Methyl bedeutet und W für eine Verbindung jeweils einer Zeile der Tabelle C entspricht
Tabelle 32
   Verbindungen der allgemeinen Formel IA.4', in denen R^{α} für Trifluormethyl, R¹ für Methyl steht und die Kombination der Reste R² und R³ für eine Verbindung jeweils einer Zeile der Tabelle D entspricht
Tabelle 33
   Verbindungen der allgemeinen Formel IA.4', in denen R^{α} für Trifluormethyl, R¹ für Methoxy steht und die Kombination der Reste R² und R³ für eine Verbindung jeweils einer Zeile der Tabelle D entspricht
Tabelle 34
   Verbindungen der allgemeinen Formel IA.4'', in denen R^{α} für Trifluormethyl, R¹ für Methyl steht und die Kombination der Reste R² und R³ für eine Verbindung jeweils einer Zeile der Tabelle D entspricht
Tabelle 35
   Verbindungen der allgemeinen Formel IA.4'', in denen R^{α} für Trifluormethyl, R¹ für Methoxy steht und die Kombination der Reste R² und R³ für eine Verbindung jeweils einer Zeile der Tabelle D entspricht
Tabelle 36
   Verbindungen der allgemeinen Formel IA.5'', in denen R^{α} für Trifluormethyl steht und W für eine Verbindung jeweils einer Zeile der Tabelle C entspricht
Tabelle 37
   Verbindungen der allgemeinen Formel IA.6', in denen R^{α} für Trifluormethyl steht und W für eine Verbindung jeweils einer Zeile der Tabelle C entspricht
Tabelle 38
   Verbindungen der allgemeinen Formel IA.6'', in denen R^{α} für Trifluormethyl steht und W für eine Verbindung jeweils einer Zeile der Tabelle C entspricht
Tabelle 39
   Verbindungen der allgemeinen Formel IA.7', in denen R^{α} für Trifluormethyl steht und W für eine Verbindung jeweils einer Zeile der Tabelle C entspricht
Tabelle 40
   Verbindungen der allgemeinen Formel IA.1', in denen R^{α} für Cyclopropyl steht und Z für eine Verbindung jeweils einer Zeile der Tabelle A entspricht
Tabelle 41
   Verbindungen der allgemeinen Formel IA.1'', in denen R^{α} für Cyclopropyl steht und Z für eine Verbindung jeweils einer Zeile der Tabelle B entspricht
Tabelle 42
   Verbindungen der allgemeinen Formel IA.2', in denen R^{α} für Cyclopropyl steht, und Z für eine Verbindung jeweils einer Zeile der Tabelle B entspricht
Tabelle 43
   Verbindungen der allgemeinen Formel IA.3', in denen R^{α} für Cyclopropyl steht, R¹ Methyl bedeutet und W für eine Verbindung jeweils einer Zeile der Tabelle C entspricht
Tabelle 44
   Verbindungen der allgemeinen Formel IA.3'', in denen R^{α} für Cyclopropyl steht, R¹ Methyl bedeutet und W für eine Verbindung jeweils einer Zeile der Tabelle C entspricht
Tabelle 45
   Verbindungen der allgemeinen Formel IA.4', in denen R^{α} für Cyclopropyl, R¹ für Methyl steht und die Kombination der Reste R² und R³ für eine Verbindung jeweils einer Zeile der Tabelle D entspricht
Tabelle 46
   Verbindungen der allgemeinen Formel IA.4', in denen R^{α} für Cyclopropyl, R¹ für Methoxy steht und die Kombination der Reste R² und R³ für eine Verbindung jeweils einer Zeile der Tabelle D entspricht
Tabelle 47
   Verbindungen der allgemeinen Formel IA.4'', in denen R^{α} für Cyclopropyl, R¹ für Methyl steht und die Kombination der Reste R² und R³ für eine Verbindung jeweils einer Zeile der Tabelle D entspricht
Tabelle 48
   Verbindungen der allgemeinen Formel IA.4'', in denen R^{α} für Cyclopropyl, R¹ für Methoxy steht und die Kombination der Reste R² und R³ für eine Verbindung jeweils einer Zeile der Tabelle D entspricht
Tabelle 49
   Verbindungen der allgemeinen Formel IA.5'', in denen R^{α} für Cyclopropyl steht und W für eine Verbindung jeweils einer Zeile der Tabelle C entspricht
Tabelle 50
   Verbindungen der allgemeinen Formel IA.6', in denen R^{α} für Cyclopropyl steht und W für eine Verbindung jeweils einer Zeile der Tabelle C entspricht
Tabelle 51
   Verbindungen der allgemeinen Formel IA.6'', in denen R^{α} für Cyclopropyl steht und W für eine Verbindung jeweils einer Zeile der Tabelle C entspricht
Tabelle 52
   Verbindungen der allgemeinen Formel IA.7', in denen R^{α} für Cyclopropyl steht und W für eine Verbindung jeweils einer Zeile der Tabelle C entspricht
Tabelle 53
   Verbindungen der allgemeinen Formel IB.1', in denen R^{α} für Methyl und R^{β} für Wasserstoff stehen und Z für eine Verbindung jeweils einer Zeile der Tabelle A entspricht
Tabelle 54
   Verbindungen der allgemeinen Formel IB.1'', in denen R^{α} für Methyl und R^{β} für Wasserstoff stehen und Z für eine Verbindung jeweils einer Zeile der Tabelle B entspricht
Tabelle 55
   Verbindungen der allgemeinen Formel IB.2', in denen R^{α} für Methyl und R^{β} für Wasserstoff stehen und Z für eine Verbindung jeweils einer Zeile der Tabelle B entspricht
Tabelle 56
   Verbindungen der allgemeinen Formel IB.3', in denen R^{α} für Methyl und R^{β} für Wasserstoff stehen, R¹ Methyl bedeutet und W für eine Verbindung jeweils einer Zeile der Tabelle C entspricht
Tabelle 57
   Verbindungen der allgemeinen Formel IB.3'', in denen R^{α} für Methyl und R^{β} für Wasserstoff stehen, R¹ Methyl bedeutet und W für eine Verbindung jeweils einer Zeile der Tabelle C entspricht
Tabelle 58
   Verbindungen der allgemeinen Formel IB.4', in denen R^{α} für Methyl und R^{β} für Wasserstoff und R¹ für Methyl stehen und die Kombination der Reste R² und R³ für eine Verbindung jeweils einer Zeile der Tabelle D entspricht
Tabelle 59
   Verbindungen der allgemeinen Formel IB.4', in denen R^{α} für Methyl und R^{β} für Wasserstoff, R¹ für Methoxy steht und die Kombination der Reste R² und R³ für eine Verbindung jeweils einer Zeile der Tabelle D entspricht
Tabelle 60
   Verbindungen der allgemeinen Formel IB.4'', in denen R^{α} für Methyl und R^{β} für Wasserstoff und R¹ für Methyl stehen und die Kombination der Reste R² und R³ für eine Verbindung jeweils einer Zeile der Tabelle D entspricht
Tabelle 61
   Verbindungen der allgemeinen Formel IB.4'', in denen R^{α} für Methyl und R^{β} für Wasserstoff, R¹ für Methoxy steht und die Kombination der Reste R² und R³ für eine Verbindung jeweils einer Zeile der Tabelle D entspricht
Tabelle 62
   Verbindungen der allgemeinen Formel IB.5'', in denen R^{α} für Methyl und R^{β} für Wasserstoff steht und W für eine Verbindung jeweils einer Zeile der Tabelle C entspricht
Tabelle 63
   Verbindungen der allgemeinen Formel IB.6', in denen R^{α} für Methyl und R^{β} für Wasserstoff steht und W für eine Verbindung jeweils einer Zeile der Tabelle C entspricht
Tabelle 64
   Verbindungen der allgemeinen Formel IB.6'', in denen R^{α} für Methyl und R^{β} für Wasserstoff steht und W für eine Verbindung jeweils einer Zeile der Tabelle C entspricht
Tabelle 65
   Verbindungen der allgemeinen Formel IB.7', in denen R^{α} für Methyl und R^{β} für Wasserstoff steht und W für eine Verbindung jeweils einer Zeile der Tabelle C entspricht
Tabelle 66
   Verbindungen der allgemeinen Formel IB.1', in denen R^{α} und R^{β} für Methyl stehen und Z für eine Verbindung jeweils einer Zeile der Tabelle A entspricht
Tabelle 67
   Verbindungen der allgemeinen Formel IB.1'', in denen R^{α} und R^{β} für Methyl stehen und Z für eine Verbindung jeweils einer Zeile der Tabelle B entspricht
Tabelle 68
   Verbindungen der allgemeinen Formel IB.2', in denen R^{α} und R^{β} für Methyl stehen und Z für eine Verbindung jeweils einer Zeile der Tabelle B entspricht
Tabelle 69
   Verbindungen der allgemeinen Formel IB.3', in denen R^{α} und R^{β} für Methyl stehen, R¹ Methyl bedeutet und W für eine Verbindung jeweils einer Zeile der Tabelle C entspricht
Tabelle 70
   Verbindungen der allgemeinen Formel IB.3'', in denen R^{α} und R^{β} für Methyl stehen, R¹ Methyl bedeutet und W für eine Verbindung jeweils einer Zeile der Tabelle C entspricht
Tabelle 71
   Verbindungen der allgemeinen Formel IB.4', in denen R^{α}, R^{β} und R¹ für Methyl stehen und die Kombination der Reste R² und R³ für eine Verbindung jeweils einer Zeile der Tabelle D entspricht
Tabelle 72
   Verbindungen der allgemeinen Formel IB.4', in denen R^{α} und R^{β} für Methyl, R¹ für Methoxy steht und die Kombination der Reste R² und R³ für eine Verbindung jeweils einer Zeile der Tabelle D entspricht
Tabelle 73
   Verbindungen der allgemeinen Formel IB.4'', in denen R^{α}, R^{β} und R¹ für Methyl stehen und die Kombination der Reste R² und R³ für eine Verbindung jeweils einer Zeile der Tabelle D entspricht
Tabelle 74
   Verbindungen der allgemeinen Formel IB.4'', in denen R^{α} und R^{β} für Methyl, R¹ für Methoxy steht und die Kombination der Reste R² und R³ für eine Verbindung jeweils einer Zeile der Tabelle D entspricht
Tabelle 75
   Verbindungen der allgemeinen Formel IB.5'', in denen R^{α} und R^{β} für Methyl steht und W für eine Verbindung jeweils einer Zeile der Tabelle C entspricht
Tabelle 76
   Verbindungen der allgemeinen Formel IB.6', in denen R^{α} und R^{β} für Methyl steht und W für eine Verbindung jeweils einer Zeile der Tabelle C entspricht
Tabelle 77
   Verbindungen der allgemeinen Formel IB.6'', in denen R^{α} und R^{β} für Methyl steht und W für eine Verbindung jeweils einer Zeile der Tabelle C entspricht
Tabelle 78
   Verbindungen der allgemeinen Formel IB.7', in denen R^{α} und R^{β} für Methyl steht und W für eine Verbindung jeweils einer Zeile der Tabelle C entspricht
Tabelle 79
   Verbindungen der allgemeinen Formel IB.1', in denen R^{α} für Methyl, R^{β} für Ethyl steht und Z für eine Verbindung jeweils einer Zeile der Tabelle A entspricht
Tabelle 80
   Verbindungen der allgemeinen Formel IB.1'', in denen R^{α} für Methyl, R^{β} für Ethyl steht und Z für eine Verbindung jeweils einer Zeile der Tabelle B entspricht
Tabelle 81
   Verbindungen der allgemeinen Formel IB.2', in denen R^{α} für Methyl, R^{β} für Ethyl steht, und Z für eine Verbindung jeweils einer Zeile der Tabelle B entspricht
Tabelle 82
   Verbindungen der allgemeinen Formel IB.3', in denen R^{α} für Methyl, R^{β} für Ethyl steht, R¹ Methyl bedeutet und W für eine Verbindung jeweils einer Zeile der Tabelle C entspricht
Tabelle 83
   Verbindungen der allgemeinen Formel IB.3'', in denen R^{α} für Methyl, R^{β} für Ethyl steht, R¹ Methyl bedeutet und W für eine Verbindung jeweils einer Zeile der Tabelle C entspricht
Tabelle 84
   Verbindungen der allgemeinen Formel IB.4', in denen R^{α} für Methyl, R^{β} für Ethyl, R¹ für Methyl steht und die Kombination der Reste R² und R³ für eine Verbindung jeweils einer Zeile der Tabelle D entspricht
Tabelle 85
   Verbindungen der allgemeinen Formel IB.4', in denen R^{α} für Methyl, R^{β} für Ethyl, R¹ für Methoxy steht und die Kombination der Reste R² und R³ für eine Verbindung jeweils einer Zeile der Tabelle D entspricht
Tabelle 86
   Verbindungen der allgemeinen Formel IB.4'', in denen R^{α} für Methyl, R^{β} für Ethyl, R¹ für Methyl steht und die Kombination der Reste R² und R³ für eine Verbindung jeweils einer Zeile der Tabelle D entspricht
Tabelle 87
   Verbindungen der allgemeinen Formel IB.4'', in denen R^{α} für Methyl, R^{β} für Ethyl, R¹ für Methoxy steht und die Kombination der Reste R² und R³ für eine Verbindung jeweils einer Zeile der Tabelle D entspricht
Tabelle 88
   Verbindungen der allgemeinen Formel IB.5'', in denen R^{α} für Methyl, R^{β} für Ethyl steht und W für eine Verbindung jeweils einer Zeile der Tabelle C entspricht
Tabelle 89
   Verbindungen der allgemeinen Formel IB.6', in denen R^{α} für Methyl, R^{β} für Ethyl steht und W für eine Verbindung jeweils einer Zeile der Tabelle C entspricht
Tabelle 90
   Verbindungen der allgemeinen Formel IB.6'', in denen R^{α} für Methyl, R^{β} für Ethyl steht und W für eine Verbindung jeweils einer Zeile der Tabelle C entspricht
Tabelle 91
   Verbindungen der allgemeinen Formel IB.7', in denen R^{α} für Methyl, R^{β} für Ethyl steht und W für eine Verbindung jeweils einer Zeile der Tabelle C entspricht
Tabelle 92
   Verbindungen der allgemeinen Formel IB.1', in denen R^{α} für Methyl, R^{β} für Trifluormethyl steht und Z für eine Verbindung jeweils einer Zeile der Tabelle A entspricht
Tabelle 93
   Verbindungen der allgemeinen Formel IB.1'', in denen R^{α} für Methyl, R^{β} für Trifluormethyl steht und Z für eine Verbindung jeweils einer Zeile der Tabelle B entspricht
Tabelle 94
   Verbindungen der allgemeinen Formel IB.2', in denen R^{α} für Methyl, R^{β} für Trifluormethyl steht, und Z für eine Verbindung jeweils einer Zeile der Tabelle B entspricht
Tabelle 95
   Verbindungen der allgemeinen Formel IB.3', in denen R^{α} für Methyl, R^{β} für Trifluormethyl steht, R¹ Methyl bedeutet und W für eine Verbindung jeweils einer Zeile der Tabelle C entspricht
Tabelle 96
   Verbindungen der allgemeinen Formel IB.3'', in denen R^{α} für Methyl, R^{β} für Trifluormethyl steht, R¹ Methyl bedeutet und W für eine Verbindung jeweils einer Zeile der Tabelle C entspricht
Tabelle 97
   Verbindungen der allgemeinen Formel IB.4', in denen R^{α} für Methyl, R^{β} für Trifluormethyl, R¹ für Methyl steht und die Kombination der Reste R² und R³ für eine Verbindung jeweils einer Zeile der Tabelle D entspricht
Tabelle 98
   Verbindungen der allgemeinen Formel IB.4', in denen R^{α} für Methyl, R^{β} für Trifluormethyl, R¹ für Methoxy steht und die Kombination der Reste R² und R³ für eine Verbindung jeweils einer Zeile der Tabelle D entspricht
Tabelle 99
   Verbindungen der allgemeinen Formel IB.4'', in denen R^{α} für Methyl, R^{β} für Trifluormethyl, R¹ für Methyl steht und die Kombination der Reste R² und R³ für eine Verbindung jeweils einer Zeile der Tabelle D entspricht
Tabelle 100
   Verbindungen der allgemeinen Formel IB.4'', in denen R^{α} für Methyl, R^{β} für Trifluormethyl, R¹ für Methoxy steht und die Kombination der Reste R² und R³ für eine Verbindung jeweils einer Zeile der Tabelle D entspricht
Tabelle 101
   Verbindungen der allgemeinen Formel IB.5'', in denen R^{α} für Methyl, R^{β} für Trifluormethyl steht und W für eine Verbindung jeweils einer Zeile der Tabelle C entspricht
Tabelle 102
   Verbindungen der allgemeinen Formel IB.6', in denen R^{α} für Methyl, R^{β} für Trifluormethyl steht und W für eine Verbindung jeweils einer Zeile der Tabelle C entspricht
Tabelle 103
   Verbindungen der allgemeinen Formel IB.6'', in denen R^{α} für Methyl, R^{β} für Trifluormethyl steht und W für eine Verbindung jeweils einer Zeile der Tabelle C entspricht
Tabelle 104
   Verbindungen der allgemeinen Formel IB.7', in denen R^{α} für Methyl, R^{β} für Trifluormethyl steht und W für eine Verbindung jeweils einer Zeile der Tabelle C entspricht
Tabelle 105
   Verbindungen der allgemeinen Formel IB.1', in denen R^{α} für Methyl, R^{β} für Cyclopropyl steht und Z für eine Verbindung jeweils einer Zeile der Tabelle A entspricht
Tabelle 106
   Verbindungen der allgemeinen Formel IB.1'', in denen R^{α} für Methyl, R^{β} für Cyclopropyl steht und Z für eine Verbindung jeweils einer Zeile der Tabelle B entspricht
Tabelle 107
   Verbindungen der allgemeinen Formel IB.2', in denen R^{α} für Methyl, R^{β} für Cyclopropyl steht, und Z für eine Verbindung jeweils einer Zeile der Tabelle B entspricht
Tabelle 108
   Verbindungen der allgemeinen Formel IB.3', in denen R^{α} für Methyl, R^{β} für Cyclopropyl steht, R¹ Methyl bedeutet und W für eine Verbindung jeweils einer Zeile der Tabelle C entspricht
Tabelle 109
   Verbindungen der allgemeinen Formel IB.3'', in denen R^{α} für Methyl, R^{β} für Cyclopropyl steht, R¹ Methyl bedeutet und W für eine Verbindung jeweils einer Zeile der Tabelle C entspricht
Tabelle 110
   Verbindungen der allgemeinen Formel IB.4', in denen R^{α} für Methyl, R^{β} für Cyclopropyl, R¹ für Methyl steht und die Kombination der Reste R² und R³ für eine Verbindung jeweils einer Zeile der Tabelle D entspricht
Tabelle 111
   Verbindungen der allgemeinen Formel IB.4', in denen R^{α} für Methyl, R^{β} für Cyclopropyl, R¹ für Methoxy steht und die Kombination der Reste R² und R³ für eine Verbindung jeweils einer Zeile der Tabelle D entspricht
Tabelle 112
   Verbindungen der allgemeinen Formel IB.4'', in denen R^{α} für Methyl, R^{β} für Cyclopropyl, R¹ für Methyl steht und die Kombination der Reste R² und R³ für eine Verbindung jeweils einer Zeile der Tabelle D entspricht
Tabelle 113
   Verbindungen der allgemeinen Formel IB.4'', in denen R^{α} für Methyl, R^{β} für Cyclopropyl, R¹ für Methoxy steht und die Kombination der Reste R² und R³ für eine Verbindung jeweils einer Zeile der Tabelle D entspricht
Tabelle 114
   Verbindungen der allgemeinen Formel IB.5'', in denen R^{α} für Methyl, R^{β} für Cyclopropyl steht und W für eine Verbindung jeweils einer Zeile der Tabelle C entspricht
Tabelle 115
   Verbindungen der allgemeinen Formel IB.6', in denen R^{α} für Methyl, R^{β} für Cyclopropyl steht und W für eine Verbindung jeweils einer Zeile der Tabelle C entspricht
Tabelle 116
   Verbindungen der allgemeinen Formel IB.6'', in denen R^{α} für Methyl, R^{β} für Cyclopropyl steht und W für eine Verbindung jeweils einer Zeile der Tabelle C entspricht
Tabelle 117
   Verbindungen der allgemeinen Formel IB.7', in denen R^{α} für Methyl, R^{β} für Cyclopropyl steht und W für eine Verbindung jeweils einer Zeile der Tabelle C entspricht
Tabelle 118
   Verbindungen der allgemeinen Formel IB.1', in denen R^{α} für Methyl, R^{β} für Benzyl steht und Z für eine Verbindung jeweils einer Zeile der Tabelle A entspricht
Tabelle 119
   Verbindungen der allgemeinen Formel IB.1'', in denen R^{α} für Methyl, R^{β} für Benzyl steht und Z für eine Verbindung jeweils einer Zeile der Tabelle B entspricht
Tabelle 120
   Verbindungen der allgemeinen Formel IB.2', in denen R^{α} für Methyl, R^{β} für Benzyl steht, und Z für eine Verbindung jeweils einer Zeile der Tabelle B entspricht
Tabelle 121
   Verbindungen der allgemeinen Formel IB.3', in denen R^{α} für Methyl, R^{β} für Benzyl steht, R¹ Methyl bedeutet und W für eine Verbindung jeweils einer Zeile der Tabelle C entspricht
Tabelle 122
   Verbindungen der allgemeinen Formel IB.3'', in denen R^{α} für Methyl, R^{β} für Benzyl steht, R¹ Methyl bedeutet und W für eine Verbindung jeweils einer Zeile der Tabelle C entspricht
Tabelle 123
   Verbindungen der allgemeinen Formel IB.4', in denen R^{α} für Methyl, R^{β} für Benzyl, R¹ für Methyl steht und die Kombination der Reste R² und R³ für eine Verbindung jeweils einer Zeile der Tabelle D entspricht
Tabelle 124
   Verbindungen der allgemeinen Formel IB.4', in denen R^{α} für Methyl, R^{β} für Benzyl, R¹ für Methoxy steht und die Kombination der Reste R² und R³ für eine Verbindung jeweils einer Zeile der Tabelle D entspricht
Tabelle 125
   Verbindungen der allgemeinen Formel IB.4'', in denen R^{α} für Methyl, R^{β} für Benzyl, R¹ für Methyl steht und die Kombination der Reste R² und R³ für eine Verbindung jeweils einer Zeile der Tabelle D entspricht
Tabelle 126
   Verbindungen der allgemeinen Formel IB.4'', in denen R^{α} für Methyl, R^{β} für Benzyl, R¹ für Methoxy steht und die Kombination der Reste R² und R³ für eine Verbindung jeweils einer Zeile der Tabelle D entspricht
Tabelle 127
   Verbindungen der allgemeinen Formel IB.5'', in denen R^{α} für Methyl, R^{β} für Benzyl steht und W für eine Verbindung jeweils einer Zeile der Tabelle C entspricht
Tabelle 128
   Verbindungen der allgemeinen Formel IB.6', in denen R^{α} für Methyl, R^{β} für Benzyl steht und W für eine Verbindung jeweils einer Zeile der Tabelle C entspricht
Tabelle 129
   Verbindungen der allgemeinen Formel IB.6'', in denen R^{α} für Methyl, R^{β} für Benzyl steht und W für eine Verbindung jeweils einer Zeile der Tabelle C entspricht
Tabelle 130
   Verbindungen der allgemeinen Formel IB.7', in denen R^{α} für Methyl, R^{β} für Benzyl steht und W für eine Verbindung jeweils einer Zeile der Tabelle C entspricht
Tabelle 131
   Verbindungen der allgemeinen Formel IB.1', in denen R^{α} für Ethyl und R^{β} für Wasserstoff stehen und Z für eine Verbindung jeweils einer Zeile der Tabelle A entspricht
Tabelle 132
   Verbindungen der allgemeinen Formel IB.1'', in denen R^{α} für Ethyl und R^{β} für Wasserstoff stehen und Z für eine Verbindung jeweils einer Zeile der Tabelle B entspricht
Tabelle 133
   Verbindungen der allgemeinen Formel IB.2', in denen R^{α} für Ethyl und R^{β} für Wasserstoff stehen und Z für eine Verbindung jeweils einer Zeile der Tabelle B entspricht
Tabelle 134
   Verbindungen der allgemeinen Formel IB.3', in denen R^{α} für Ethyl und R^{β} für Wasserstoff stehen, R¹ Methyl bedeutet und W für eine Verbindung jeweils einer Zeile der Tabelle C entspricht
Tabelle 135
   Verbindungen der allgemeinen Formel IB.3'', in denen R^{α} für Ethyl und R^{β} für Wasserstoff stehen, R¹ Methyl bedeutet und W für eine Verbindung jeweils einer Zeile der Tabelle C entspricht
Tabelle 136
   Verbindungen der allgemeinen Formel IB.4', in denen R^{α} für Ethyl und R^{β} für Wasserstoff und R¹ für Methyl stehen und die Kombination der Reste R² und R³ für eine Verbindung jeweils einer Zeile der Tabelle D entspricht
Tabelle 137
   Verbindungen der allgemeinen Formel IB.4', in denen R^{α} für Ethyl und R^{β} für Wasserstoff, R¹ für Methoxy steht und die Kombination der Reste R² und R³ für eine Verbindung jeweils einer Zeile der Tabelle D entspricht
Tabelle 138
   Verbindungen der allgemeinen Formel IB.4'', in denen R^{α} für Ethyl und R^{β} für Wasserstoff und R¹ für Methyl stehen und die Kombination der Reste R² und R³ für eine Verbindung jeweils einer Zeile der Tabelle D entspricht
Tabelle 139
   Verbindungen der allgemeinen Formel IB.4'', in denen R^{α} für Ethyl und R^{β} für Wasserstoff, R¹ für Methoxy steht und die Kombination der Reste R² und R³ für eine Verbindung jeweils einer Zeile der Tabelle D entspricht
Tabelle 140
   Verbindungen der allgemeinen Formel IB.5'', in denen R^{α} für Ethyl und R^{β} für Wasserstoff steht und W für eine Verbindung jeweils einer Zeile der Tabelle C entspricht
Tabelle 141
   Verbindungen der allgemeinen Formel IB.6', in denen R^{α} für Ethyl und R^{β} für Wasserstoff steht und W für eine Verbindung jeweils einer Zeile der Tabelle C entspricht
Tabelle 142
   Verbindungen der allgemeinen Formel IB.6'', in denen R^{α} für Ethyl und R^{β} für Wasserstoff steht und W für eine Verbindung jeweils einer Zeile der Tabelle C entspricht
Tabelle 143
   Verbindungen der allgemeinen Formel IB.7', in denen R^{α} für Ethyl und R^{β} für Wasserstoff steht und W für eine Verbindung jeweils einer Zeile der Tabelle C entspricht
Tabelle 144
   Verbindungen der allgemeinen Formel IB.1', in denen R^{α} für Ethyl, R^{β} für Methyl stehen und Z für eine Verbindung jeweils einer Zeile der Tabelle A entspricht
Tabelle 145
   Verbindungen der allgemeinen Formel IB.1'', in denen R^{α} für Ethyl, R^{β} für Methyl stehen und Z für eine Verbindung jeweils einer Zeile der Tabelle B entspricht
Tabelle 146
   Verbindungen der allgemeinen Formel IB.2', in denen R^{α} für Ethyl, R^{β} für Methyl stehen und Z für eine Verbindung jeweils einer Zeile der Tabelle B entspricht
Tabelle 147
   Verbindungen der allgemeinen Formel IB.3', in denen R^{α} für Ethyl, R^{β} für Methyl stehen, R¹ Methyl bedeutet und W für eine Verbindung jeweils einer Zeile der Tabelle C entspricht
Tabelle 148
   Verbindungen der allgemeinen Formel IB.3'', in denen R^{α} für Ethyl, R^{β} für Methyl stehen, R¹ Methyl bedeutet und W für eine Verbindung jeweils einer Zeile der Tabelle C entspricht
Tabelle 149
   Verbindungen der allgemeinen Formel IB.4', in denen R^{α} für Ethyl, R^{β} und R¹ für Methyl stehen und die Kombination der Reste R² und R³ für eine Verbindung jeweils einer Zeile der Tabelle D entspricht
Tabelle 150
   Verbindungen der allgemeinen Formel IB.4', in denen R^{α} für Ethyl, R^{β} für Methyl, R¹ für Methoxy steht und die Kombination der Reste R² und R³ für eine Verbindung jeweils einer Zeile der Tabelle D entspricht
Tabelle 151
   Verbindungen der allgemeinen Formel IB.4'', in denen R^{α} für Ethyl, R^{β} und R¹ für Methyl stehen und die Kombination der Reste R² und R³ für eine Verbindung jeweils einer Zeile der Tabelle D entspricht
Tabelle 152
   Verbindungen der allgemeinen Formel IB.4'', in denen R^{α} für Ethyl, R^{β} für Methyl, R¹ für Methoxy steht und die Kombination der Reste R² und R³ für eine Verbindung jeweils einer Zeile der Tabelle D entspricht
Tabelle 153
   Verbindungen der allgemeinen Formel IB.5'', in denen R^{α} für Ethyl, R^{β} für Methyl steht und W für eine Verbindung jeweils einer Zeile der Tabelle C entspricht
Tabelle 154
   Verbindungen der allgemeinen Formel IB.6', in denen R^{α} für Ethyl, R^{β} für Methyl steht und W für eine Verbindung jeweils einer Zeile der Tabelle C entspricht
Tabelle 155
   Verbindungen der allgemeinen Formel IB.6'', in denen R^{α} für Ethyl, R^{β} für Methyl steht und W für eine Verbindung jeweils einer Zeile der Tabelle C entspricht
Tabelle 156
   Verbindungen der allgemeinen Formel IB.7', in denen R^{α} für Ethyl, R^{β} für Methyl steht und W für eine Verbindung jeweils einer Zeile der Tabelle C entspricht
Tabelle 157
   Verbindungen der allgemeinen Formel IB.1', in denen R^{α} und R^{β} für Ethyl stehen und Z für eine Verbindung jeweils einer Zeile der Tabelle A entspricht
Tabelle 158
   Verbindungen der allgemeinen Formel IB.1'', in denen R^{α} und R^{β} für Ethyl stehen und Z für eine Verbindung jeweils einer Zeile der Tabelle B entspricht
Tabelle 159
   Verbindungen der allgemeinen Formel IB.2', in denen R^{α} und R^{β} für Ethyl stehen und Z für eine Verbindung jeweils einer Zeile der Tabelle B entspricht
Tabelle 160
   Verbindungen der allgemeinen Formel IB.3', in denen R^{α} und R^{β} für Ethyl stehen, R¹ Methyl bedeutet und W für eine Verbindung jeweils einer Zeile der Tabelle C entspricht
Tabelle 161
   Verbindungen der allgemeinen Formel IB.3'', in denen R^{α} und R^{β} für Ethyl stehen, R¹ Methyl bedeutet und W für eine Verbindung jeweils einer Zeile der Tabelle C entspricht
Tabelle 162
   Verbindungen der allgemeinen Formel IB.4', in denen R^{α} und R^{β} für Ethyl stehen, R¹ für Methyl steht und die Kombination der Reste R² und R³ für eine Verbindung jeweils einer Zeile der Tabelle D entspricht
Tabelle 163
   Verbindungen der allgemeinen Formel IB.4', in denen R^{α} und R^{β} für Ethyl stehen, R¹ für Methoxy steht und die Kombination der Reste R² und R³ für eine Verbindung jeweils einer Zeile der Tabelle D entspricht
Tabelle 164
   Verbindungen der allgemeinen Formel IB.4'', in denen R^{α} und R^{β} für Ethyl stehen, R¹ für Methyl steht und die Kombination der Reste R² und R³ für eine Verbindung jeweils einer Zeile der Tabelle D entspricht
Tabelle 165
   Verbindungen der allgemeinen Formel IB.4'', in denen R^{α} und R^{β} für Ethyl stehen, R¹ für Methoxy steht und die Kombination der Reste R² und R³ für eine Verbindung jeweils einer Zeile der Tabelle D entspricht
Tabelle 166
   Verbindungen der allgemeinen Formel IB.5'', in denen R^{α} und R^{β} für Ethyl stehen und W für eine Verbindung jeweils einer Zeile der Tabelle C entspricht
Tabelle 167
   Verbindungen der allgemeinen Formel IB.6', in denen R^{α} und R^{β} für Ethyl stehen und W für eine Verbindung jeweils einer Zeile der Tabelle C entspricht
Tabelle 168
   Verbindungen der allgemeinen Formel IB.6'', in denen R^{α} und R^{β} für Ethyl stehen und W für eine Verbindung jeweils einer Zeile der Tabelle C entspricht
Tabelle 169
   Verbindungen der allgemeinen Formel IB.7', in denen R^{α} und R^{β} für Ethyl stehen und W für eine Verbindung jeweils einer Zeile der Tabelle C entspricht
Tabelle 170
   Verbindungen der allgemeinen Formel IB.1', in denen R^{α} für Ethyl, R^{β} für Trifluormethyl steht und Z für eine Verbindung jeweils einer Zeile der Tabelle A entspricht
Tabelle 171
   Verbindungen der allgemeinen Formel IB.1'', in denen R^{α} für Ethyl, R^{β} für Trifluormethyl steht und Z für eine Verbindung jeweils einer Zeile der Tabelle B entspricht
Tabelle 172
   Verbindungen der allgemeinen Formel IB.2', in denen R^{α} für Ethyl, R^{β} für Trifluormethyl steht, und Z für eine Verbindung jeweils einer Zeile der Tabelle B entspricht
Tabelle 173
   Verbindungen der allgemeinen Formel IB.3', in denen R^{α} für Ethyl, R^{β} für Trifluormethyl steht, R¹ Methyl bedeutet und W für eine Verbindung jeweils einer Zeile der Tabelle C entspricht
Tabelle 174
   Verbindungen der allgemeinen Formel IB.3'', in denen R^{α} für Ethyl, R^{β} für Trifluormethyl steht, R¹ Methyl bedeutet und W für eine Verbindung jeweils einer Zeile der Tabelle C entspricht
Tabelle 175
   Verbindungen der allgemeinen Formel IB.4', in denen R^{α} für Ethyl, R^{β} für Trifluormethyl, R¹ für Methyl steht und die Kombination der Reste R² und R³ für eine Verbindung jeweils einer Zeile der Tabelle D entspricht
Tabelle 176
   Verbindungen der allgemeinen Formel IB.4', in denen R^{α} für Ethyl, R^{β} für Trifluormethyl, R¹ für Methoxy steht und die Kombination der Reste R² und R³ für eine Verbindung jeweils einer Zeile der Tabelle D entspricht
Tabelle 177
   Verbindungen der allgemeinen Formel IB.4'', in denen R^{α} für Ethyl, R^{β} für Trifluormethyl, R¹ für Methyl steht und die Kombination der Reste R² und R³ für eine Verbindung jeweils einer Zeile der Tabelle D entspricht
Tabelle 178
   Verbindungen der allgemeinen Formel IB.4'', in denen R^{α} für Ethyl, R^{β} für Trifluormethyl, R¹ für Methoxy steht und die Kombination der Reste R² und R³ für eine Verbindung jeweils einer Zeile der Tabelle D entspricht
Tabelle 179
   Verbindungen der allgemeinen Formel IB.5'', in denen R^{α} für Ethyl, R^{β} für Trifluormethyl steht und W für eine Verbindung jeweils einer Zeile der Tabelle C entspricht
Tabelle 180
   Verbindungen der allgemeinen Formel IB.6', in denen R^{α} für Ethyl, R^{β} für Trifluormethyl steht und W für eine Verbindung jeweils einer Zeile der Tabelle C entspricht
Tabelle 181
   Verbindungen der allgemeinen Formel IB.6'', in denen R^{α} für Ethyl, R^{β} für Trifluormethyl steht und W für eine Verbindung jeweils einer Zeile der Tabelle C entspricht
Tabelle 182
   Verbindungen der allgemeinen Formel IB.7', in denen R^{α} für Ethyl, R^{β} für Trifluormethyl steht und W für eine Verbindung jeweils einer Zeile der Tabelle C entspricht
Tabelle 183
   Verbindungen der allgemeinen Formel IB.1', in denen R^{α} für Ethyl, R^{β} für Cyclopropyl steht und Z für eine Verbindung jeweils einer Zeile der Tabelle A entspricht
Tabelle 184
   Verbindungen der allgemeinen Formel IB.1'', in denen R^{α} für Ethyl, R^{β} für Cyclopropyl steht und Z für eine Verbindung jeweils einer Zeile der Tabelle B entspricht
Tabelle 185
   Verbindungen der allgemeinen Formel IB.2', in denen R^{α} für Ethyl, R^{β} für Cyclopropyl steht, und Z für eine Verbindung jeweils einer Zeile der Tabelle B entspricht
Tabelle 186
   Verbindungen der allgemeinen Formel IB.3', in denen R^{α} für Ethyl, R^{β} für Cyclopropyl steht, R¹ Methyl bedeutet und W für eine Verbindung jeweils einer Zeile der Tabelle C entspricht
Tabelle 187
   Verbindungen der allgemeinen Formel IB.3'', in denen R^{α} für Ethyl, R^{β} für Cyclopropyl steht, R¹ Methyl bedeutet und W für eine Verbindung jeweils einer Zeile der Tabelle C entspricht
Tabelle 188
   Verbindungen der allgemeinen Formel IB.4', in denen R^{α} für Ethyl, R^{β} für Cyclopropyl, R¹ für Methyl steht und die Kombination der Reste R² und R³ für eine Verbindung jeweils einer Zeile der Tabelle D entspricht
Tabelle 189
   Verbindungen der allgemeinen Formel IB.4', in denen R^{α} für Ethyl, R^{β} für Cyclopropyl, R¹ für Methoxy steht und die Kombination der Reste R² und R³ für eine Verbindung jeweils einer Zeile der Tabelle D entspricht
Tabelle 190
   Verbindungen der allgemeinen Formel IB.4'', in denen R^{α} für Ethyl, R^{β} für Cyclopropyl, R¹ für Methyl steht und die Kombination der Reste R² und R³ für eine Verbindung jeweils einer Zeile der Tabelle D entspricht
Tabelle 191
   Verbindungen der allgemeinen Formel IB.4'', in denen R^{α} für Ethyl, R^{β} für Cyclopropyl, R¹ für Methoxy steht und die Kombination der Reste R² und R³ für eine Verbindung jeweils einer Zeile der Tabelle D entspricht
Tabelle 192
   Verbindungen der allgemeinen Formel IB.5'', in denen R^{α} für Ethyl, R^{β} für Cyclopropyl steht und W für eine Verbindung jeweils einer Zeile der Tabelle C entspricht
Tabelle 193
   Verbindungen der allgemeinen Formel IB.6', in denen R^{α} für Ethyl, R^{β} für Cyclopropyl steht und W für eine Verbindung jeweils einer Zeile der Tabelle C entspricht
Tabelle 194
   Verbindungen der allgemeinen Formel IB.6'', in denen R^{α} für Ethyl, R^{β} für Cyclopropyl steht und W für eine Verbindung jeweils einer Zeile der Tabelle C entspricht
Tabelle 195
   Verbindungen der allgemeinen Formel IB.7', in denen R^{α} für Ethyl, R^{β} für Cyclopropyl steht und W für eine Verbindung jeweils einer Zeile der Tabelle C entspricht
Tabelle 196
   Verbindungen der allgemeinen Formel IB.1', in denen R^{α} für Ethyl, R^{β} für Benzyl steht und Z für eine Verbindung jeweils einer Zeile der Tabelle A entspricht
Tabelle 197
   Verbindungen der allgemeinen Formel IB.1'', in denen R^{α} für Ethyl, R^{β} für Benzyl steht und Z für eine Verbindung jeweils einer Zeile der Tabelle B entspricht
Tabelle 198
   Verbindungen der allgemeinen Formel IB.2', in denen R^{α} für Ethyl, R^{β} für Benzyl steht, und Z für eine Verbindung jeweils einer Zeile der Tabelle B entspricht
Tabelle 199
   Verbindungen der allgemeinen Formel IB.3', in denen R^{α} für Ethyl, R^{β} für Benzyl steht, R¹ Methyl bedeutet und W für eine Verbindung jeweils einer Zeile der Tabelle C entspricht
Tabelle 200
   Verbindungen der allgemeinen Formel IB.3'', in denen R^{α} für Ethyl, R^{β} für Benzyl steht, R¹ Methyl bedeutet und W für eine Verbindung jeweils einer Zeile der Tabelle C entspricht
Tabelle 201
   Verbindungen der allgemeinen Formel IB.4', in denen R^{α} für Ethyl, R^{β} für Benzyl, R¹ für Methyl steht und die Kombination der Reste R² und R³ für eine Verbindung jeweils einer Zeile der Tabelle D entspricht
Tabelle 202
   Verbindungen der allgemeinen Formel IB.4', in denen R^{α} für Ethyl, R^{β} für Benzyl, R¹ für Methoxy steht und die Kombination der Reste R² und R³ für eine Verbindung jeweils einer Zeile der Tabelle D entspricht
Tabelle 203
   Verbindungen der allgemeinen Formel IB.4'', in denen R^{α} für Ethyl, R^{β} für Benzyl, R¹ für Methyl steht und die Kombination der Reste R² und R³ für eine Verbindung jeweils einer Zeile der Tabelle D entspricht
Tabelle 204
   Verbindungen der allgemeinen Formel IB.4'', in denen R^{α} für Ethyl, R^{β} für Benzyl, R¹ für Methoxy steht und die Kombination der Reste R² und R³ für eine Verbindung jeweils einer Zeile der Tabelle D entspricht
Tabelle 205
   Verbindungen der allgemeinen Formel IB.5'', in denen R^{α} für Ethyl, R^{β} für Benzyl steht und W für eine Verbindung jeweils einer Zeile der Tabelle C entspricht
Tabelle 206
   Verbindungen der allgemeinen Formel IB.6', in denen R^{α} für Ethyl, R^{β} für Benzyl steht und W für eine Verbindung jeweils einer Zeile der Tabelle C entspricht
Tabelle 207
   Verbindungen der allgemeinen Formel IB.6'', in denen R^{α} für Ethyl, R^{β} für Benzyl steht und W für eine Verbindung jeweils einer Zeile der Tabelle C entspricht
Tabelle 208
   Verbindungen der allgemeinen Formel IB.7', in denen R^{α} für Ethyl, R^{β} für Benzyl steht und W für eine Verbindung jeweils einer Zeile der Tabelle C entspricht
Tabelle 209
   Verbindungen der allgemeinen Formel IB.1', in denen R^{α} für Trifluormethyl und R^{β} für Wasserstoff stehen und Z für eine Verbindung jeweils einer Zeile der Tabelle A entspricht
Tabelle 210
   Verbindungen der allgemeinen Formel IB.1'', in denen R^{α} für Triflu ormethyl und R^{β} für Wasserstoff stehen und Z für eine Verbindung jeweils einer Zeile der Tabelle B entspricht
Tabelle 211
   Verbindungen der allgemeinen Formel IB.2, in denen R^{α} für Trifluormethyl und R^{β} für Wasserstoff stehen und Z für eine Verbindung jeweils einer Zeile der Tabelle B entspricht
Tabelle 212
   Verbindungen der allgemeinen Formel IB.3', in denen R^{α} für Trifluormethyl und R^{β} für Wasserstoff stehen, R¹ Methyl bedeutet und W für eine Verbindung jeweils einer Zeile der Tabelle C entspricht
Tabelle 213
   Verbindungen der allgemeinen Formel IB.3'', in denen R^{α} für Trifluormethyl und R^{β} für Wasserstoff stehen, R¹ Methyl bedeutet und W für eine Verbindung jeweils einer Zeile der Tabelle C entspricht
Tabelle 214
   Verbindungen der allgemeinen Formel IB.4', in denen R^{α} für Trifluormethyl und R^{β} für Wasserstoff und R¹ für Methyl stehen und die Kombination der Reste R² und R³ für eine Verbindung jeweils einer Zeile der Tabelle D entspricht
Tabelle 215
   Verbindungen der allgemeinen Formel IB.4', in denen R^{α} für Trifluormethyl und R^{β} für Wasserstoff, R¹ für Methoxy steht und die Kombination der Reste R² und R³ für eine Verbindung jeweils einer Zeile der Tabelle D entspricht
Tabelle 216
   Verbindungen der allgemeinen Formel IB.4'', in denen R^{α} für Trifluormethyl und R^{β} für Wasserstoff und R¹ für Methyl stehen und die Kombination der Reste R² und R³ für eine Verbindung jeweils einer Zeile der Tabelle D entspricht
Tabelle 217
   Verbindungen der allgemeinen Formel IB.4'', in denen R^{α} für Trifluormethyl und R^{β} für Wasserstoff, R¹ für Methoxy steht und die Kombination der Reste R² und R³ für eine Verbindung jeweils einer Zeile der Tabelle D entspricht
Tabelle 218
   Verbindungen der allgemeinen Formel IB.5'', in denen R^{α} für Trifluormethyl und R^{β} für Wasserstoff steht und W für eine Verbindung jeweils einer Zeile der Tabelle C entspricht
Tabelle 219
   Verbindungen der allgemeinen Formel IB.6', in denen R^{α} für Trifluormethyl und R^{β} für Wasserstoff steht und W für eine Verbindung jeweils einer Zeile der Tabelle C entspricht
Tabelle 220
   Verbindungen der allgemeinen Formel IB.6'', in denen R^{α} für Trifluormethyl und R^{β} für Wasserstoff steht und W für eine Verbindung jeweils einer Zeile der Tabelle C entspricht
Tabelle 221
   Verbindungen der allgemeinen Formel IB.7', in denen R^{α} für Trifluormethyl und R^{β} für Wasserstoff steht und W für eine Verbindung jeweils einer Zeile der Tabelle C entspricht
Tabelle 222
   Verbindungen der allgemeinen Formel IB.1', in denen R^{α} für Trifluormethyl, R^{β} für Methyl stehen und Z für eine Verbindung jeweils einer Zeile der Tabelle A entspricht
Tabelle 223
   Verbindungen der allgemeinen Formel IB.1'', in denen R^{α} für Trifluormethyl, R^{β} für Methyl stehen und Z für eine Verbindung jeweils einer Zeile der Tabelle B entspricht
Tabelle 224
   Verbindungen der allgemeinen Formel IB.2', in denen R^{α} für Trifluormethyl, R^{β} für Methyl stehen und Z für eine Verbindung jeweils einer Zeile der Tabelle B entspricht
Tabelle 225
   Verbindungen der allgemeinen Formel IB.3', in denen R^{α} für Trifluormethyl, R^{β} für Methyl stehen, R¹ Methyl bedeutet und W für eine Verbindung jeweils einer Zeile der Tabelle C entspricht
Tabelle 226
   Verbindungen der allgemeinen Formel IB.3'', in denen R^{α} für Trifluormethyl, R^{β} für Methyl stehen, R¹ Methyl bedeutet und W für eine Verbindung jeweils einer Zeile der Tabelle C entspricht
Tabelle 227
   Verbindungen der allgemeinen Formel IB.4', in denen R^{α} für Trifluormethyl, R^{β} und R¹ für Methyl stehen und die Kombination der Reste R² und R³ für eine Verbindung jeweils einer Zeile der Tabelle D entspricht
Tabelle 228
   Verbindungen der allgemeinen Formel IB.4', in denen R^{α} für Trifluormethyl, R^{β} für Methyl, R¹ für Methoxy steht und die Kombination der Reste R² und R³ für eine Verbindung jeweils einer Zeile der Tabelle D entspricht
Tabelle 229
   Verbindungen der allgemeinen Formel IB.4'', in denen R^{α} für Trifluormethyl, R^{β} und R¹ für Methyl stehen und die Kombination der Reste R² und R³ für eine Verbindung jeweils einer Zeile der Tabelle D entspricht
Tabelle 230
   Verbindungen der allgemeinen Formel IB.4'', in denen R^{α} für Trifluormethyl, R^{β} für Methyl, R¹ für Methoxy steht und die Kombination der Reste R² und R³ für eine Verbindung jeweils einer Zeile der Tabelle D entspricht
Tabelle 231
   Verbindungen der allgemeinen Formel IB.5'', in denen R^{α} für Trifluormethyl, R^{β} für Methyl steht und W für eine Verbindung jeweils einer Zeile der Tabelle C entspricht
Tabelle 232
   Verbindungen der allgemeinen Formel IB.6', in denen R^{α} für Trifluormethyl, R^{β} für Methyl steht und W für eine Verbindung jeweils einer Zeile der Tabelle C entspricht
Tabelle 233
   Verbindungen der allgemeinen Formel IB.6'', in denen R^{α} für Trifluormethyl, R^{β} für Methyl steht und W für eine Verbindung jeweils einer Zeile der Tabelle C entspricht
Tabelle 234
   Verbindungen der allgemeinen Formel IB.7', in denen R^{α} für Trifluormethyl, R^{β} für Methyl steht und W für eine Verbindung jeweils einer Zeile der Tabelle C entspricht
Tabelle 235
   Verbindungen der allgemeinen Formel IB.1', in denen R^{α} und R^{β} für Ethyl stehen und Z für eine Verbindung jeweils einer Zeile der Tabelle A entspricht
Tabelle 236
   Verbindungen der allgemeinen Formel IB.1'', in denen R^{α} für Trifluormethyl und R^{β} für Ethyl steht und Z für eine Verbindung jeweils einer Zeile der Tabelle B entspricht
Tabelle 237
   Verbindungen der allgemeinen Formel IB.2', in denen R^{α} für Trifluormethyl und R^{β} für Ethyl steht und Z für eine Verbindung jeweils einer Zeile der Tabelle B entspricht
Tabelle 238
   Verbindungen der allgemeinen Formel IB.3', in denen R^{α} für Trifluormethyl und R^{β} für Ethyl steht, R¹ Methyl bedeutet und W für eine Verbindung jeweils einer Zeile der Tabelle C entspricht
Tabelle 239
   Verbindungen der allgemeinen Formel IB.3'', in denen R^{α} für Trifluormethyl und R^{β} für Ethyl steht, R¹ Methyl bedeutet und W für eine Verbindung jeweils einer Zeile der Tabelle C entspricht
Tabelle 240
   Verbindungen der allgemeinen Formel IB.4', in denen R^{α} für Trifluormethyl und R^{β} für Ethyl steht, R¹ für Methyl steht und die Kombination der Reste R² und R³ für eine Verbindung jeweils einer Zeile der Tabelle D entspricht
Tabelle 241
   Verbindungen der allgemeinen Formel IB.4', in denen R^{α} für Trifluormethyl und R^{β} für Ethyl steht, R¹ für Methoxy steht und die Kombination der Reste R² und R³ für eine Verbindung jeweils einer Zeile der Tabelle D entspricht
Tabelle 242
   Verbindungen der allgemeinen Formel IB.4'', in denen R^{α} für Trifluormethyl und R^{β} für Ethyl steht, R¹ für Methyl steht und die Kombination der Reste R² und R³ für eine Verbindung jeweils einer Zeile der Tabelle D entspricht
Tabelle 243
   Verbindungen der allgemeinen Formel IB.4'', in denen R^{α} für Trifluormethyl und R^{β} für Ethyl steht, R¹ für Methoxy steht und die Kombination der Reste R² und R³ für eine Verbindung jeweils einer Zeile der Tabelle D entspricht
Tabelle 244
   Verbindungen der allgemeinen Formel IB.5'', in denen R^{α} für Trifluormethyl und R^{β} für Ethyl steht und W für eine Verbindung jeweils einer Zeile der Tabelle C entspricht
Tabelle 245
   Verbindungen der allgemeinen Formel IB.6', in denen R^{α} für Trifluormethyl und R^{β} für Ethyl steht und W für eine Verbindung jeweils einer Zeile der Tabelle C entspricht
Tabelle 246
   Verbindungen der allgemeinen Formel IB.6'', in denen R^{α} für Trifluormethyl und R^{β} für Ethyl steht und W für eine Verbindung jeweils einer Zeile der Tabelle C entspricht
Tabelle 247
   Verbindungen der allgemeinen Formel IB.7', in denen R^{α} für Trifluormethyl und R^{β} für Ethyl steht und W für eine Verbindung jeweils einer Zeile der Tabelle C entspricht
Tabelle 248
   Verbindungen der allgemeinen Formel IB.1', in denen R^{α} und R^{β} für Trifluormethyl stehen und Z für eine Verbindung jeweils einer Zeile der Tabelle A entspricht
Tabelle 249
   Verbindungen der allgemeinen Formel IB.1'', in denen R^{α} und R^{β} für Trifluormethyl stehen und Z für eine Verbindung jeweils einer Zeile der Tabelle B entspricht
Tabelle 250
   Verbindungen der allgemeinen Formel IB.2', in denen R^{α} und R^{β} für Trifluormethyl stehen, und Z für eine Verbindung jeweils einer Zeile der Tabelle B entspricht
Tabelle 251
   Verbindungen der allgemeinen Formel IB.3', in denen R^{α} und R^{β} für Trifluormethyl stehen, R¹ Methyl bedeutet und W für eine Verbindung jeweils einer Zeile der Tabelle C entspricht
Tabelle 252
   Verbindungen der allgemeinen Formel IB.3'', in denen R^{α} und R^{β} für Trifluormethyl stehen, R¹ Methyl bedeutet und W für eine Verbindung jeweils einer Zeile der Tabelle C entspricht
Tabelle 253
   Verbindungen der allgemeinen Formel IB.4', in denen R^{α} und R^{β} für Trifluormethyl stehen, R¹ für Methyl steht und die Kombination der Reste R² und R³ für eine Verbindung jeweils einer Zeile der Tabelle D entspricht
Tabelle 254
   Verbindungen der allgemeinen Formel IB.4', in denen R^{α} und R^{β} für Trifluormethyl stehen, R¹ für Methoxy steht und die Kombination der Reste R² und R³ für eine Verbindung jeweils einer Zeile der Tabelle D entspricht
Tabelle 255
   Verbindungen der allgemeinen Formel IB.4'', in denen R^{α} und R^{β} für Trifluormethyl stehen, R¹ für Methyl steht und die Kombination der Reste R² und R³ für eine Verbindung jeweils einer Zeile der Tabelle D entspricht
Tabelle 256
   Verbindungen der allgemeinen Formel IB.4'', in denen R^{α} und R^{β} für Trifluormethyl stehen, R¹ für Methoxy steht und die Kombination der Reste R² und R³ für eine Verbindung jeweils einer Zeile der Tabelle D entspricht
Tabelle 257
   Verbindungen der allgemeinen Formel IB.5'', in denen R^{α} und R^{β} für Trifluormethyl stehen und W für eine Verbindung jeweils einer Zeile der Tabelle C entspricht
Tabelle 258
   Verbindungen der allgemeinen Formel IB.6', in denen R^{α} und R^{β} für Trifluormethyl stehen und W für eine Verbindung jeweils einer Zeile der Tabelle C entspricht
Tabelle 259
   Verbindungen der allgemeinen Formel IB.6'', in denen R^{α} und R^{β} für Trifluormethyl stehen und W für eine Verbindung jeweils einer Zeile der Tabelle C entspricht
Tabelle 260
   Verbindungen der allgemeinen Formel IB.7', in denen R^{α} und R^{β} für Trifluormethyl stehen und W für eine Verbindung jeweils einer Zeile der Tabelle C entspricht
Tabelle 261
   Verbindungen der allgemeinen Formel IB.1', in denen R^{α} und R^{β} für Trifluormethyl stehen und Z für eine Verbindung jeweils einer Zeile der Tabelle A entspricht
Tabelle 262
   Verbindungen der allgemeinen Formel IB.1'', in denen R^{α} für Trifluormethyl, R^{β} für Cyclopropyl steht und Z für eine Verbindung jeweils einer Zeile der Tabelle B entspricht
Tabelle 263
   Verbindungen der allgemeinen Formel IB.2', in denen R^{α} für Trifluormethyl, R^{β} für Cyclopropyl steht, und Z für eine Verbindung jeweils einer Zeile der Tabelle B entspricht
Tabelle 264
   Verbindungen der allgemeinen Formel IB.3', in denen R^{α} für Trifluormethyl, R^{β} für Cyclopropyl steht, R¹ Methyl bedeutet und W für eine Verbindung jeweils einer Zeile der Tabelle C entspricht
Tabelle 265
   Verbindungen der allgemeinen Formel IB.3'', in denen R^{α} für Trifluormethyl, R^{β} für Cyclopropyl steht, R¹ Methyl bedeutet und W für eine Verbindung jeweils einer Zeile der Tabelle C entspricht
Tabelle 266
   Verbindungen der allgemeinen Formel IB.4', in denen R^{α} für Trifluormethyl, R^{β} für Cyclopropyl, R¹ für Methyl steht und die Kombination der Reste R² und R³ für eine Verbindung jeweils einer Zeile der Tabelle D entspricht
Tabelle 267
   Verbindungen der allgemeinen Formel IB.4', in denen R^{α} für Trifluormethyl, R^{β} für Cyclopropyl, R¹ für Methoxy steht und die Kombination der Reste R² und R³ für eine Verbindung jeweils einer Zeile der Tabelle D entspricht
Tabelle 268
   Verbindungen der allgemeinen Formel IB.4'', in denen R^{α} für Trifluormethyl, R^{β} für Cyclopropyl, R¹ für Methyl steht und die Kombination der Reste R² und R³ für eine Verbindung jeweils einer Zeile der Tabelle D entspricht
Tabelle 269
   Verbindungen der allgemeinen Formel IB.4'', in denen R^{α} für Trifluormethyl, R^{β} für Cyclopropyl, R¹ für Methoxy steht und die Kombination der Reste R² und R³ für eine Verbindung jeweils einer Zeile der Tabelle D entspricht
Tabelle 270
   Verbindungen der allgemeinen Formel IB.5'', in denen R^{α} für Trifluormethyl, R^{β} für Cyclopropyl steht und W für eine Verbindung jeweils einer Zeile der Tabelle C entspricht
Tabelle 271
   Verbindungen der allgemeinen Formel IB.6', in denen R^{α} für Trifluormethyl, R^{β} für Cyclopropyl steht und W für eine Verbindung jeweils einer Zeile der Tabelle C entspricht
Tabelle 272
   Verbindungen der allgemeinen Formel IB.6'', in denen R^{α} für Trifluormethyl, R^{β} für Cyclopropyl steht und W für eine Verbindung jeweils einer Zeile der Tabelle C entspricht
Tabelle 273
   Verbindungen der allgemeinen Formel IB.7', in denen R^{α} für Trifluormethyl, R^{β} für Cyclopropyl steht und W für eine Verbindung jeweils einer Zeile der Tabelle C entspricht
Tabelle 274
   Verbindungen der allgemeinen Formel IB.1', in denen R^{α} für Trifluormethyl, R^{β} für Benzyl steht und Z für eine Verbindung jeweils einer Zeile der Tabelle A entspricht
Tabelle 275
   Verbindungen der allgemeinen Formel IB.1'', in denen R^{α} für Trifluormethyl, R^{β} für Benzyl steht und Z für eine Verbindung jeweils einer Zeile der Tabelle B entspricht
Tabelle 276
   Verbindungen der allgemeinen Formel 10.2', in denen R^{α} für Trifluormethyl, R^{β} für Benzyl steht, und Z für eine Verbindung jeweils einer Zeile der Tabelle B entspricht
Tabelle 277
   Verbindungen der allgemeinen Formel IB.3', in denen R^{α} für Trifluormethyl, R^{β} für Benzyl steht, R¹ Methyl bedeutet und W für eine Verbindung jeweils einer Zeile der Tabelle C entspricht
Tabelle 278
   Verbindungen der allgemeinen Formel IB.3'', in denen R^{α} für Trifluormethyl, R^{β} für Benzyl steht, R¹ Methyl bedeutet und W für eine Verbindung jeweils einer Zeile der Tabelle C entspricht
Tabelle 279
   Verbindungen der allgemeinen Formel IB.4', in denen R^{α} für Trifluormethyl, R^{β} für Benzyl, R¹ für Methyl steht und die Kombination der Reste R² und R³ für eine Verbindung jeweils einer Zeile der Tabelle D entspricht
Tabelle 280
   Verbindungen der allgemeinen Formel IB.4', in denen R^{α} für Trifluormethyl, R^{β} für Benzyl, R¹ für Methoxy steht und die Kombination der Reste R² und R³ für eine Verbindung jeweils einer Zeile der Tabelle D entspricht
Tabelle 281
   Verbindungen der allgemeinen Formel IB.4'', in denen R^{α} für Trifluormethyl, R^{β} für Benzyl, R¹ für Methyl steht und die Kombination der Reste R² und R³ für eine Verbindung jeweils einer Zeile der Tabelle D entspricht
Tabelle 282
   Verbindungen der allgemeinen Formel IB.4'', in denen R^{α} für Trifluormethyl, R^{β} für Benzyl, R¹ für Methoxy steht und die Kombination der Reste R² und R³ für eine Verbindung jeweils einer Zeile der Tabelle D entspricht
Tabelle 283
   Verbindungen der allgemeinen Formel IB.5'', in denen R^{α} für Trifluormethyl, R^{β} für Benzyl steht und W für eine Verbindung jeweils einer Zeile der Tabelle C entspricht
Tabelle 284
   Verbindungen der allgemeinen Formel IB.6', in denen R^{α} für Trifluormethyl, R^{β} für Benzyl steht und W für eine Verbindung jeweils einer Zeile der Tabelle C entspricht
Tabelle 285
   Verbindungen der allgemeinen Formel IB.6'', in denen R^{α} für Trifluormethyl, R^{β} für Benzyl steht und W für eine Verbindung jeweils einer Zeile der Tabelle C entspricht
Tabelle 286
   Verbindungen der allgemeinen Formel IB.7', in denen R^{α} für Trifluormethyl, R^{β} für Benzyl steht und W für eine Verbindung jeweils einer Zeile der Tabelle C entspricht
Tabelle 287
   Verbindungen der allgemeinen Formel IB.1', in denen R^{α} für Cyclopropyl und R^{β} für Wasserstoff stehen und Z für eine Verbindung jeweils einer Zeile der Tabelle A entspricht
Tabelle 288
   Verbindungen der allgemeinen Formel IB.1'', in denen R^{α} für Cyclopropyl und R^{β} für Wasserstoff stehen und Z für eine Verbindung jeweils einer Zeile der Tabelle B entspricht
Tabelle 289
   Verbindungen der allgemeinen Formel IB.2', in denen R^{α} für Cyclopropyl und R^{β} für Wasserstoff stehen und Z für eine Verbindung jeweils einer Zeile der Tabelle B entspricht
Tabelle 290
   Verbindungen der allgemeinen Formel IB.3', in denen R^{α} für Cyclopropyl und R^{β} für Wasserstoff stehen, R¹ Methyl bedeutet und W für eine Verbindung jeweils einer Zeile der Tabelle C entspricht
Tabelle 291
   Verbindungen der allgemeinen Formel IB.3'', in denen R^{α} für Cyclopropyl und R^{β} für Wasserstoff stehen, R¹ Methyl bedeutet und W für eine Verbindung jeweils einer Zeile der Tabelle C entspricht
Tabelle 292
   Verbindungen der allgemeinen Formel IB.4', in denen R^{α} für Cyclopropyl und R^{β} für Wasserstoff und R¹ für Methyl stehen und die Kombination der Reste R² und R³ für eine Verbindung jeweils einer Zeile der Tabelle D entspricht
Tabelle 293
   Verbindungen der allgemeinen Formel IB.4', in denen R^{α} für Cyclopropyl und R^{β} für Wasserstoff, R¹ für Methoxy steht und die Kombination der Reste R² und R³ für eine Verbindung jeweils einer Zeile der Tabelle D entspricht
Tabelle 294
   Verbindungen der allgemeinen Formel IB.4'', in denen R^{α} für Cyclopropyl und R^{β} für Wasserstoff und R¹ für Methyl stehen und die Kombination der Reste R² und R³ für eine Verbindung jeweils einer Zeile der Tabelle D entspricht
Tabelle 295
   Verbindungen der allgemeinen Formel IB.4'', in denen R^{α} für Cyclopropyl und R^{β} für Wasserstoff, R¹ für Methoxy steht und die Kombination der Reste R² und R³ für eine Verbindung jeweils einer Zeile der Tabelle D entspricht
Tabelle 296
   Verbindungen der allgemeinen Formel IB.5'', in denen R^{α} für Cyclopropyl und R^{β} für Wasserstoff steht und W für eine Verbindung jeweils einer Zeile der Tabelle C entspricht
Tabelle 297
   Verbindungen der allgemeinen Formel IB.6', in denen R^{α} für Cyclopropyl und R^{β} für Wasserstoff steht und W für eine Verbindung jeweils einer Zeile der Tabelle C entspricht
Tabelle 298
   Verbindungen der allgemeinen Formel IB.6'', in denen R^{α} für Cyclopropyl und R^{β} für Wasserstoff steht und W für eine Verbindung jeweils einer Zeile der Tabelle C entspricht
Tabelle 299
   Verbindungen der allgemeinen Formel IB.7', in denen R^{α} für Cyclopropyl und R^{β} für Wasserstoff steht und W für eine Verbindung jeweils einer Zeile der Tabelle C entspricht
Tabelle 300
   Verbindungen der allgemeinen Formel IB.1', in denen R^{α} für Cyclopropyl, R^{β} für Methyl stehen und Z für eine Verbindung jeweils einer Zeile der Tabelle A entspricht
Tabelle 301
   Verbindungen der allgemeinen Formel IB.1'', in denen R^{α} für Cyclopropyl, R^{β} für Methyl stehen und Z für eine Verbindung jeweils einer Zeile der Tabelle B entspricht
Tabelle 302
   Verbindungen der allgemeinen Formel IB.2', in denen R^{α} für Cyclopropyl, R^{β} für Methyl stehen und Z für eine Verbindung jeweils einer Zeile der Tabelle B entspricht
Tabelle 303
   Verbindungen der allgemeinen Formel IB.3', in denen R^{α} für Cyclopropyl, R^{β} für Methyl stehen, R¹ Methyl bedeutet und W für eine Verbindung jeweils einer Zeile der Tabelle C entspricht
Tabelle 304
   Verbindungen der allgemeinen Formel IB.3'', in denen R^{α} für Cyclopropyl, R^{β} für Methyl stehen, R¹ Methyl bedeutet und W für eine Verbindung jeweils einer Zeile der Tabelle C entspricht
Tabelle 305
   Verbindungen der allgemeinen Formel IB.4', in denen R^{α} für Cyclopropyl, R^{β} und R¹ für Methyl stehen und die Kombination der Reste R² und R³ für eine Verbindung jeweils einer Zeile der Tabelle D entspricht
Tabelle 306
   Verbindungen der allgemeinen Formel IB.4', in denen R^{α} für Cyclopropyl, R^{β} für Methyl, R¹ für Methoxy steht und die Kombination der Reste R² und R³ für eine Verbindung jeweils einer Zeile der Tabelle D entspricht
Tabelle 307
   Verbindungen der allgemeinen Formel IB.4'', in denen R^{α} für Cyclopropyl, R^{β} und R¹ für Methyl stehen und die Kombination der Reste R² und R³ für eine Verbindung jeweils einer Zeile der Tabelle D entspricht
Tabelle 308
   Verbindungen der allgemeinen Formel IB.4'', in denen R^{α} für Cyclopropyl, R^{β} für Methyl, R¹ für Methoxy steht und die Kombination der Reste R² und R³ für eine Verbindung jeweils einer Zeile der Tabelle D entspricht
Tabelle 309
   Verbindungen der allgemeinen Formel IB.5'', in denen R^{α} für Cyclopropyl, R^{β} für Methyl steht und W für eine Verbindung jeweils einer Zeile der Tabelle C entspricht
Tabelle 310
   Verbindungen der allgemeinen Formel IB.6', in denen R^{α} für Cyclopropyl, R^{β} für Methyl steht und W für eine Verbindung jeweils einer Zeile der Tabelle C entspricht
Tabelle 311
   Verbindungen der allgemeinen Formel IB.6'', in denen R^{α} für Cyclopropyl, R^{β} für Methyl steht und W für eine Verbindung jeweils einer Zeile der Tabelle C entspricht
Tabelle 312
   Verbindungen der allgemeinen Formel IB.7', in denen R^{α} für Cyclopropyl, R^{β} für Methyl steht und W für eine Verbindung jeweils einer Zeile der Tabelle C entspricht
Tabelle 313
   Verbindungen der allgemeinen Formel IB.1', in denen R^{α} und R^{β} für Ethyl stehen und Z für eine Verbindung jeweils einer Zeile der Tabelle A entspricht
Tabelle 314
   Verbindungen der allgemeinen Formel IB.1'', in denen R^{α} für Cyclopropyl und R^{β} für Ethyl steht und Z für eine Verbindung jeweils einer Zeile der Tabelle B entspricht
Tabelle 315 Verbindungen der allgemeinen Formel IB.2', in denen R^{α} für Cyclo
   propyl und R^{β} für Ethyl steht und Z für eine Verbindung jeweils einer Zeile der Tabelle B entspricht
Tabelle 316
   Verbindungen der allgemeinen Formel IB.3', in denen R^{α} für Cyclopropyl und R^{β} für Ethyl steht, R¹ Methyl bedeutet und W für eine Verbindung jeweils einer Zeile der Tabelle C entspricht
Tabelle 317
   Verbindungen der allgemeinen Formel IB.3'', in denen R^{α} für Cyclopropyl und R^{β} für Ethyl steht, R¹ Methyl bedeutet und W für eine Verbindung jeweils einer Zeile der Tabelle C entspricht
Tabelle 318
   Verbindungen der allgemeinen Formel IB.4', in denen R^{α} für Cyclopropyl und R^{β} für Ethyl steht, R¹ für Methyl steht und die Kombination der Reste R² und R³ für eine Verbindung jeweils einer Zeile der Tabelle D entspricht
Tabelle 319
   Verbindungen der allgemeinen Formel IB.4', in denen R^{α} für Cyclopropyl und R^{β} für Ethyl steht, R¹ für Methoxy steht und die Kombination der Reste R² und R³ für eine Verbindung jeweils einer Zeile der Tabelle D entspricht
Tabelle 320
   Verbindungen der allgemeinen Formel IB.4'', in denen R^{α} für Cyclopropyl und R^{β} für Ethyl steht, R¹ für Methyl steht und die Kombination der Reste R² und R³ für eine Verbindung jeweils einer Zeile der Tabelle D entspricht
Tabelle 321
   Verbindungen der allgemeinen Formel IB.4'', in denen R^{α} für Cyclopropyl und R^{β} für Ethyl steht, R¹ für Methoxy steht und die Kombination der Reste R² und R³ für eine Verbindung jeweils einer Zeile der Tabelle D entspricht
Tabelle 322
   Verbindungen der allgemeinen Formel IB.5'', in denen R^{α} für Cyclopropyl und R^{β} für Ethyl steht und W für eine Verbindung jeweils einer Zeile der Tabelle C entspricht
Tabelle 323
   Verbindungen der allgemeinen Formel IB.6', in denen R^{α} für Cyclopropyl und R^{β} für Ethyl steht und W für eine Verbindung jeweils einer Zeile der Tabelle C entspricht
Tabelle 324
   Verbindungen der allgemeinen Formel IB.6'', in denen R^{α} für Cyclopropyl und R^{β} für Ethyl steht und W für eine Verbindung jeweils einer Zeile der Tabelle C entspricht
Tabelle 325
   Verbindungen der allgemeinen Formel IB.7', in denen R^{α} für Cyclopropyl und R^{β} für Ethyl steht und W für eine Verbindung jeweils einer Zeile der Tabelle C entspricht
Tabelle 326
   Verbindungen der allgemeinen Formel IB.1', in denen R^{α} für Cyclopropyl und R^{β} für Trifluormethyl stehen und Z für eine Verbindung jeweils einer Zeile der Tabelle A entspricht
Tabelle 327
   Verbindungen der allgemeinen Formel IB.1'', in denen R^{α} für Cyclopropyl und R^{β} für Trifluormethyl stehen und Z für eine Verbindung jeweils einer Zeile der Tabelle B entspricht
Tabelle 328
   Verbindungen der allgemeinen Formel IB.2', in denen R^{α} für Cyclopropyl und R^{β} für Trifluormethyl stehen, und Z für eine Verbindung jeweils einer Zeile der Tabelle B entspricht
Tabelle 329
   Verbindungen der allgemeinen Formel Ig. 3', in denen R^{α} für Cyclopropyl und R^{β} für Trifluormethyl stehen, R¹ Methyl bedeutet und W für eine Verbindung jeweils einer Zeile der Tabelle C entspricht
Tabelle 330
   Verbindungen der allgemeinen Formel IB.3'', in denen R^{α} für Cyclopropyl und R^{β} für Trifluormethyl stehen, R¹ Methyl bedeutet und W für eine Verbindung jeweils einer Zeile der Tabelle C entspricht
Tabelle 331
   Verbindungen der allgemeinen Formel IB.4', in denen R^{α} für Cyclopropyl und R^{β} für Trifluormethyl stehen, R¹ für Methyl steht und Die Kombination der Reste R² und R³ für eine Verbindung jeweils einer Zeile der Tabelle D entspricht
Tabelle 332
   Verbindungen der allgemeinen Formel IB.4', in denen R^{α} für Cyclopropyl und R^{β} für Trifluormethyl stehen, R¹ für Methoxy steht und die Kombination der Reste R² und R³ für eine Verbindung jeweils einer Zeile der Tabelle D entspricht
Tabelle 333
   Verbindungen der allgemeinen Formel IB.4'', in denen R^{α} für Cyclopropyl und R^{β} für Trifluormethyl stehen, R¹ für Methyl steht und die Kombination der Reste R² und R³ für eine Verbindung jeweils einer Zeile der Tabelle D entspricht
Tabelle 334
   Verbindungen der allgemeinen Formel IB.4'', in denen R^{α} für Cyclopropyl und R^{β} für Trifluormethyl stehen, R¹ für Methoxy steht und die Kombination der Reste R² und R³ für eine Verbindung jeweils einer Zeile der Tabelle D entspricht
Tabelle 335
   Verbindungen der allgemeinen Formel IB.5'', in denen R^{α} für Cyclopropyl und R^{β} für Trifluormethyl stehen und W für eine Verbindung jeweils einer Zeile der Tabelle C entspricht
Tabelle 336
   Verbindungen der allgemeinen Formel IB.6', in denen R^{α} für Cyclopropyl und R^{β} für Trifluormethyl stehen und W für eine Verbindung jeweils einer Zeile der Tabelle C entspricht
Tabelle 337
   Verbindungen der allgemeinen Formel IB.6'', in denen R^{α} für Cyclopropyl und R^{β} für Trifluormethyl stehen und W für eine Verbindung jeweils einer Zeile der Tabelle C entspricht
Tabelle 338
   Verbindungen der allgemeinen Formel IB.7', in denen R^{α} für Cyclopropyl und R^{β} für Trifluormethyl stehen und W für eine Verbindung jeweils einer Zeile der Tabelle C entspricht
Tabelle 339
   Verbindungen der allgemeinen Formel IB.1', in denen R^{α} für Cyclopropyl und R^{β} für Trifluormethyl stehen und Z für eine Verbindung jeweils einer Zeile der Tabelle A entspricht
Tabelle 340
   Verbindungen der allgemeinen Formel IB.1'', in denen R^{α} für Cyclopropyl, R^{β} für Cyclopropyl steht und Z für eine Verbindung jeweils einer Zeile der Tabelle B entspricht
Tabelle 341
   Verbindungen der allgemeinen Formel IB.2', in denen R^{α} für Cyclopropyl, R^{β} für Cyclopropyl steht, und Z für eine Verbindung jeweils einer Zeile der Tabelle B entspricht
Tabelle 342
   Verbindungen der allgemeinen Formel IB.3', in denen R^{α} für Cyclopropyl, R^{β} für Cyclopropyl steht, R¹ Methyl bedeutet und W für eine Verbindung jeweils einer Zeile der Tabelle C entspricht
Tabelle 343
   Verbindungen der allgemeinen Formel IB.3'', in denen R^{α} für Cyclopropyl, R^{β} für Cyclopropyl steht, R¹ Methyl bedeutet und W für eine Verbindung jeweils einer Zeile der Tabelle C entspricht
Tabelle 344
   Verbindungen der allgemeinen Formel IB.4', in denen R^{α} für Cyclopropyl, R^{β} für Cyclopropyl, R¹ für Methyl steht und die Kombination der Reste R² und R³ für eine Verbindung jeweils einer Zeile der Tabelle D entspricht
Tabelle 345
   Verbindungen der allgemeinen Formel IB.4', in denen R^{α} für Cyclopropyl, R^{β} für Cyclopropyl, R¹ für Methoxy steht und die Kombination der Reste R² und R³ für eine Verbindung jeweils einer Zeile der Tabelle D entspricht
Tabelle 346
   Verbindungen der allgemeinen Formel IB.4'', in denen R^{α} für Cyclopropyl, R^{β} für Cyclopropyl, R¹ für Methyl steht und die Kombination der Reste R² und R³ für eine Verbindung jeweils einer Zeile der Tabelle D entspricht
Tabelle 347
   Verbindungen der allgemeinen Formel IB.4'', in denen R^{α} für Cyclopropyl, R^{β} für Cyclopropyl, R¹ für Methoxy steht und die Kombination der Reste R² und R³ für eine Verbindung jeweils einer Zeile der Tabelle D entspricht
Tabelle 348
   Verbindungen der allgemeinen Formel IB.5'', in denen R^{α} für Cyclopropyl, R^{β} für Cyclopropyl steht und W für eine Verbindung jeweils einer Zeile der Tabelle C entspricht
Tabelle 349
   Verbindungen der allgemeinen Formel IB.6', in denen R^{α} für Cyclopropyl, R^{β} für Cyclopropyl steht und W für eine Verbindung jeweils einer Zeile der Tabelle C entspricht
Tabelle 350
   Verbindungen der allgemeinen Formel IB.6'', in denen R^{α} für Cyclopropyl, R^{β} für Cyclopropyl steht und W für eine Verbindung jeweils einer Zeile der Tabelle C entspricht
Tabelle 351
   Verbindungen der allgemeinen Formel IB.7', in denen R^{α} für Cyclopropyl, R^{β} für Cyclopropyl steht und W für eine Verbindung jeweils einer Zeile der Tabelle C entspricht
Tabelle 352
   Verbindungen der allgemeinen Formel IB.1', in denen R^{α} für Cyclopropyl, R^{β} für Benzyl steht und Z für eine Verbindung jeweils einer Zeile der Tabelle A entspricht
Tabelle 353
   Verbindungen der allgemeinen Formel IB.1'', in denen R^{α} für Cyclopropyl, R^{β} für Benzyl steht und Z für eine Verbindung jeweils einer Zeile der Tabelle B entspricht
Tabelle 354
   Verbindungen der allgemeinen Formel IB.2', in denen R^{α} für Cyclopropyl, R^{β} für Benzyl steht, und Z für eine Verbindung jeweils einer Zeile der Tabelle B entspricht
Tabelle 355
   Verbindungen der allgemeinen Formel IB.3', in denen R^{α} für Cyclopropyl, R^{β} für Benzyl steht, R¹ Methyl bedeutet und W für eine Verbindung jeweils einer Zeile der Tabelle C entspricht
Tabelle 356
   Verbindungen der allgemeinen Formel IB.3'', in denen R^{α} für Cyclopropyl, R^{β} für Benzyl steht, R¹ Methyl bedeutet und H für eine Verbindung jeweils einer Zeile der Tabelle C entspricht
Tabelle 357
   Verbindungen der allgemeinen Formel IB.4', in denen R^{α} für Cyclopropyl, R^{β} für Benzyl, R¹ für Methyl steht und die Kombination der Reste R² und R³ für eine Verbindung jeweils einer Zeile der Tabelle D entspricht
Tabelle 358
   Verbindungen der allgemeinen Formel IB.4', in denen R^{α} für Cyclopropyl, R^{β} für Benzyl, R¹ für Methoxy steht und die Kombination der Reste R² und R³ für eine Verbindung jeweils einer Zeile der Tabelle D entspricht
Tabelle 359
   Verbindungen der allgemeinen Formel IB.4'', in denen R^{α} für Cyclopropyl, R^{β} für Benzyl, R¹ für Methyl steht und die Kombination der Reste R² und R³ für eine Verbindung jeweils einer Zeile der Tabelle D entspricht
Tabelle 360
   Verbindungen der allgemeinen Formel IB.4'', in denen R^{α} für Cyclopropyl, R^{β} für Benzyl, R¹ für Methoxy steht und die Kombination der Reste R² und R³ für eine Verbindung jeweils einer Zeile der Tabelle D entspricht
Tabelle 361
   Verbindungen der allgemeinen Formel IB.5'', in denen R^{α} für Cyclopropyl, R^{β} für Benzyl steht und W für eine Verbindung jeweils einer Zeile der Tabelle C entspricht
Tabelle 362
   Verbindungen der allgemeinen Formel IB.6', in denen R^{α} für Cyclopropyl, R^{β} für Benzyl steht und W für eine Verbindung jeweils einer Zeile der Tabelle C entspricht
Tabelle 363
   Verbindungen der allgemeinen Formel IB.6'', in denen R^{α} für Cyclopropyl, R^{β} für Benzyl steht und W für eine Verbindung jeweils einer Zeile der Tabelle C entspricht
Tabelle 364
   Verbindungen der allgemeinen Formel IB.7', in denen R^{α} für Cyclopropyl, R^{β} für Benzyl steht und W für eine Verbindung jeweils einer Zeile der Tabelle C entspricht

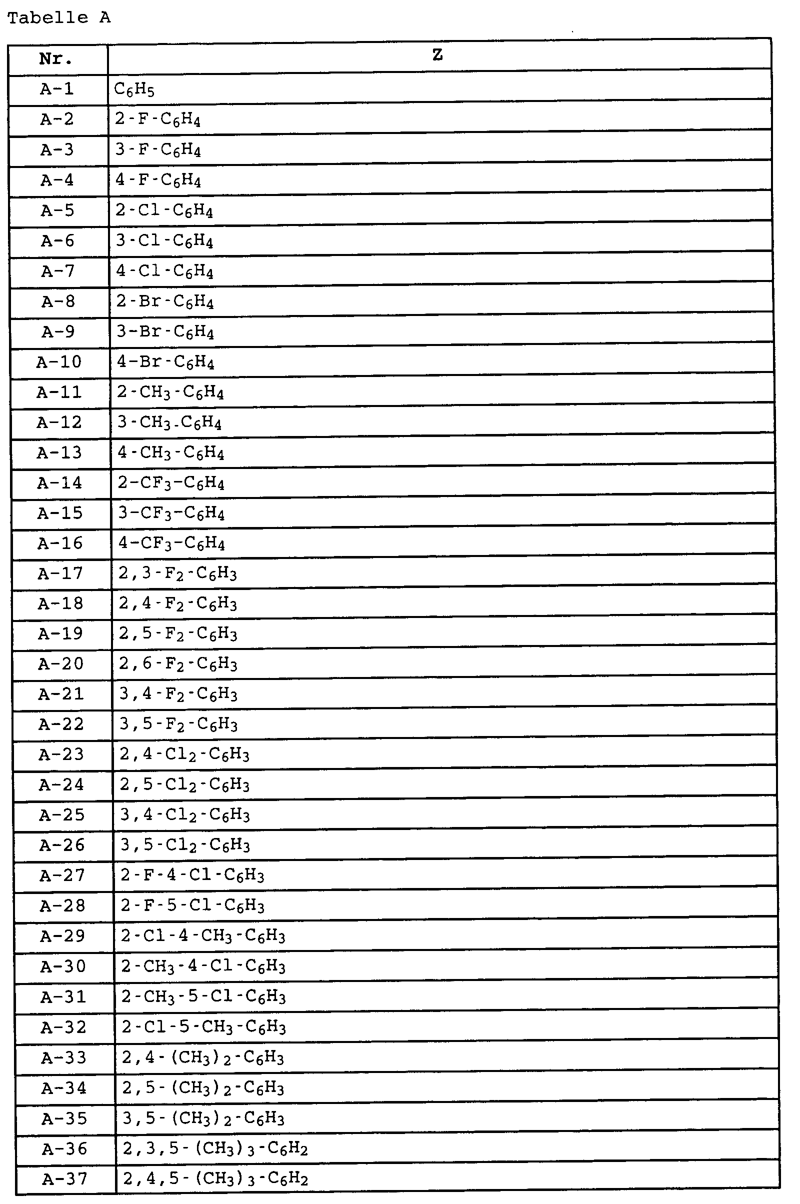

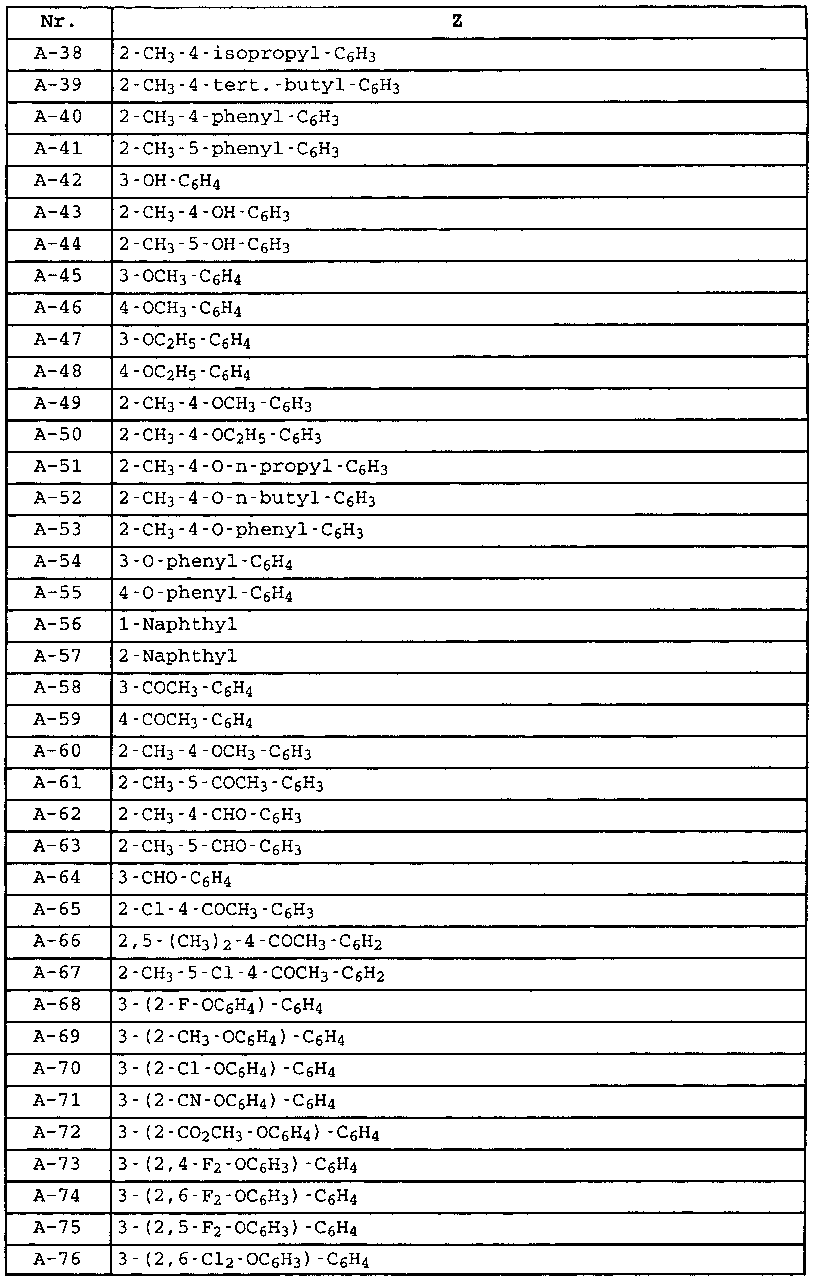

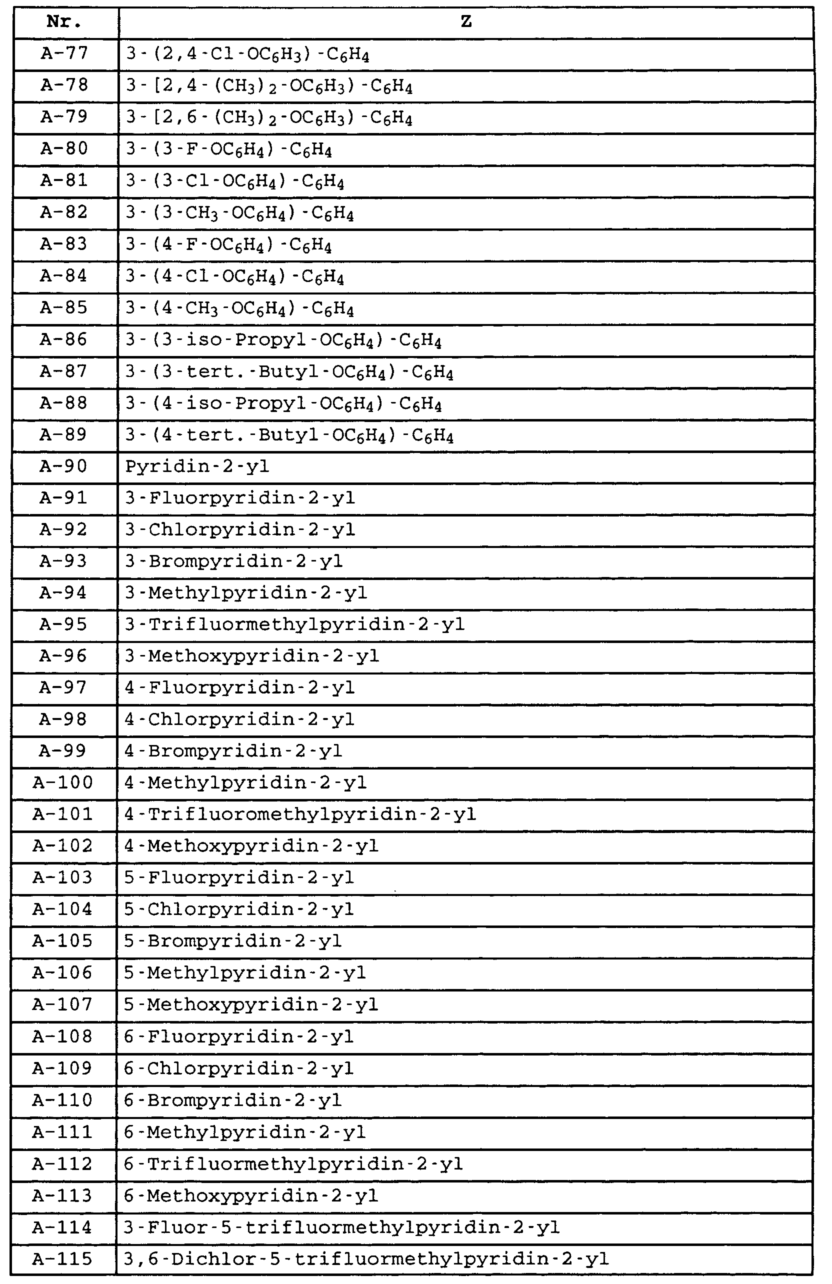

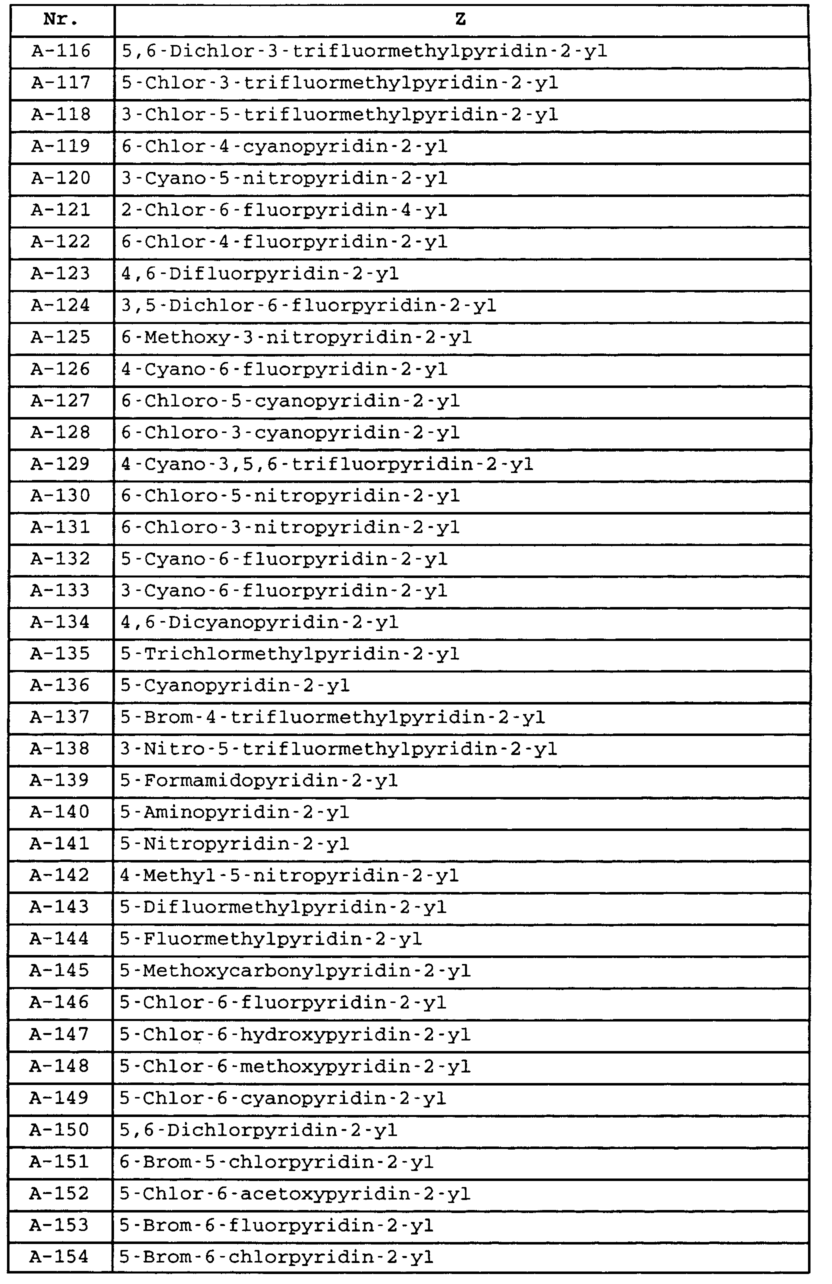

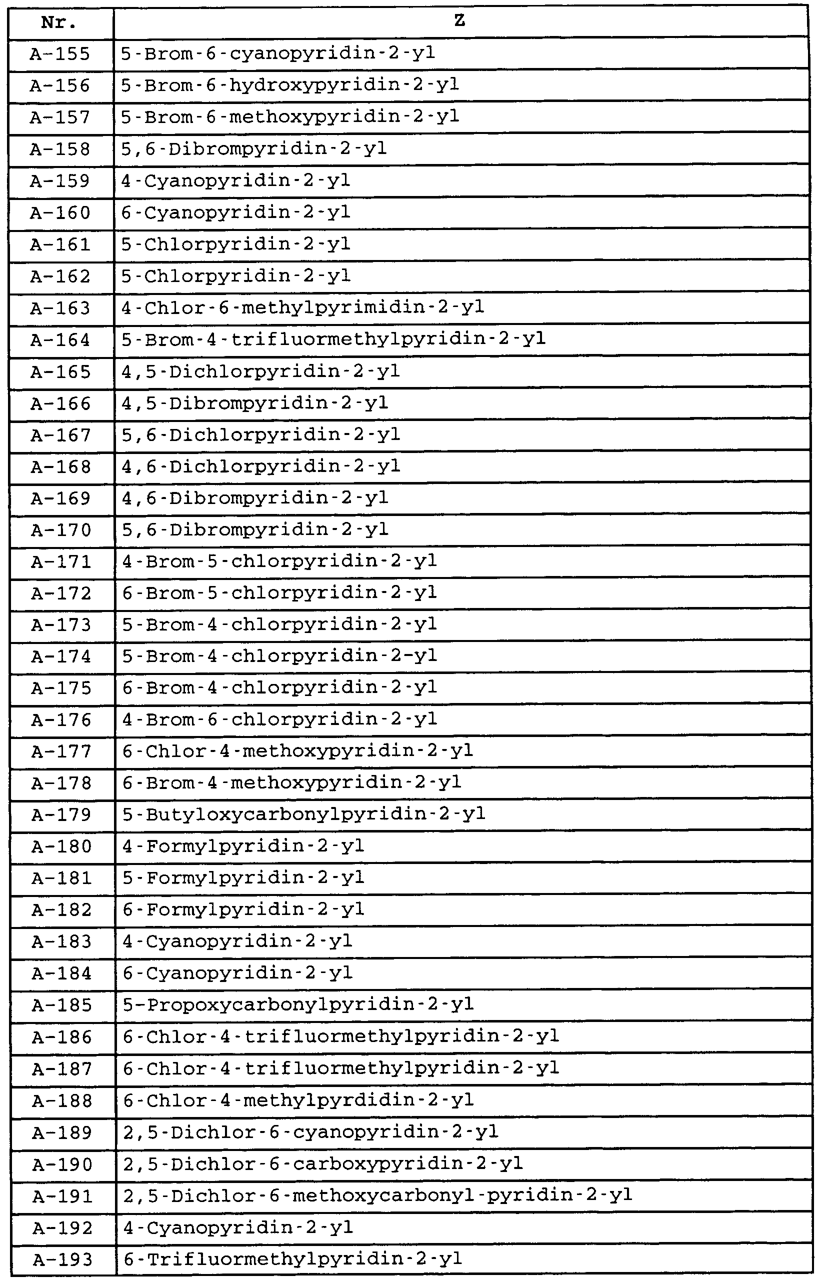

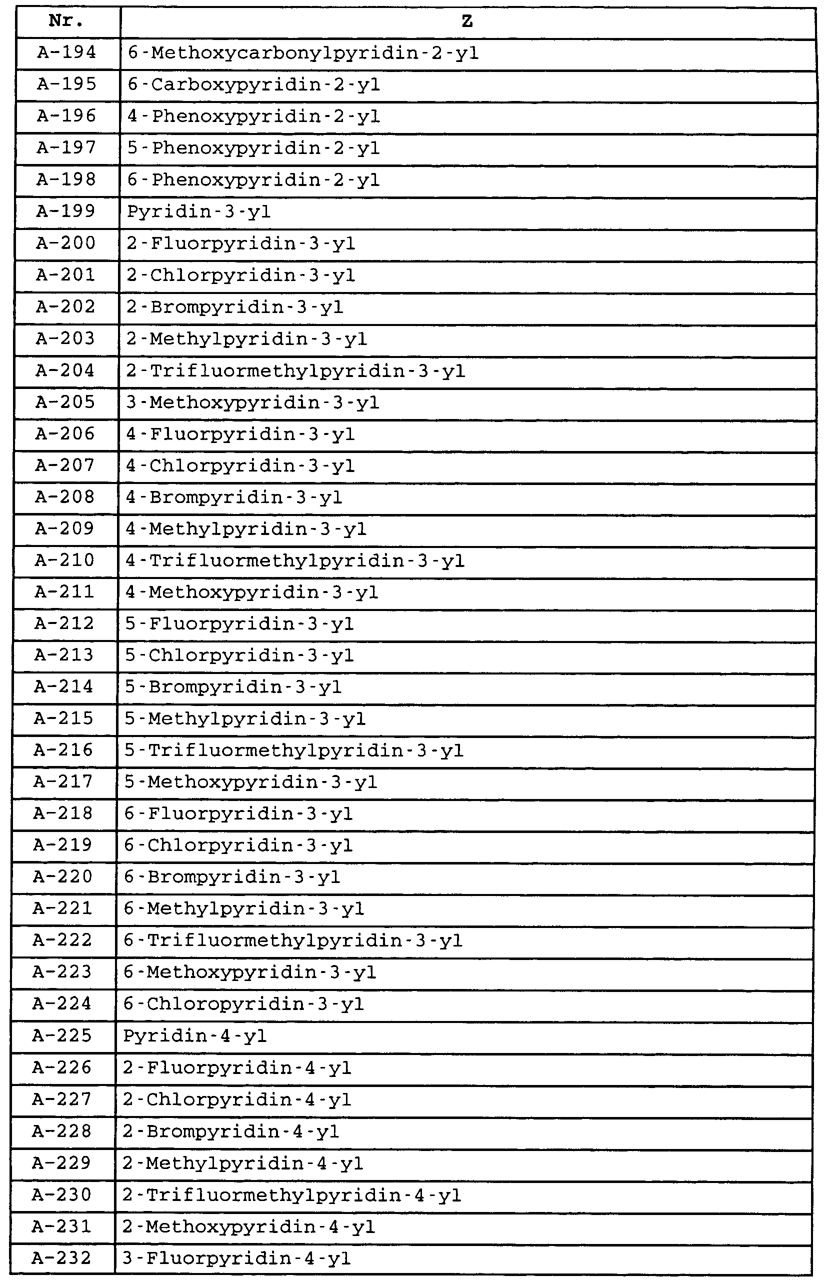

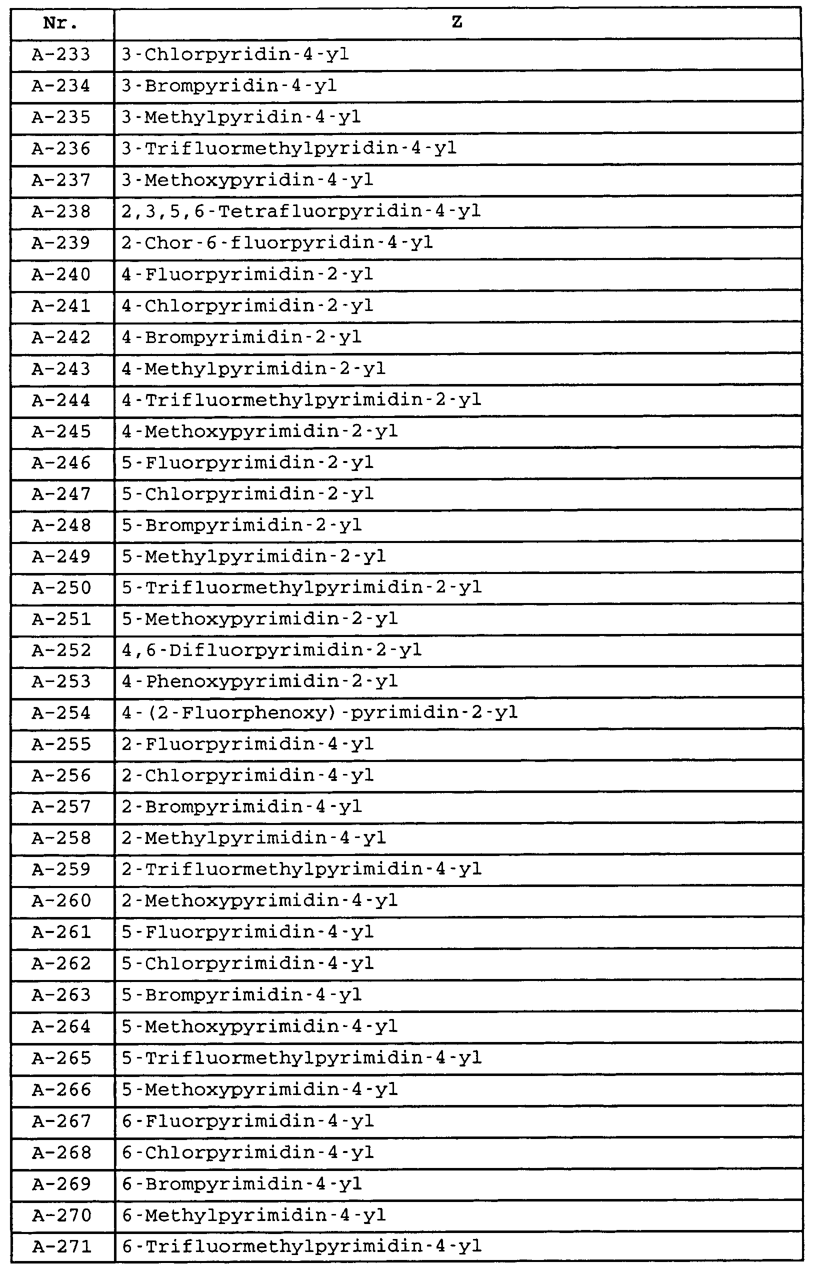

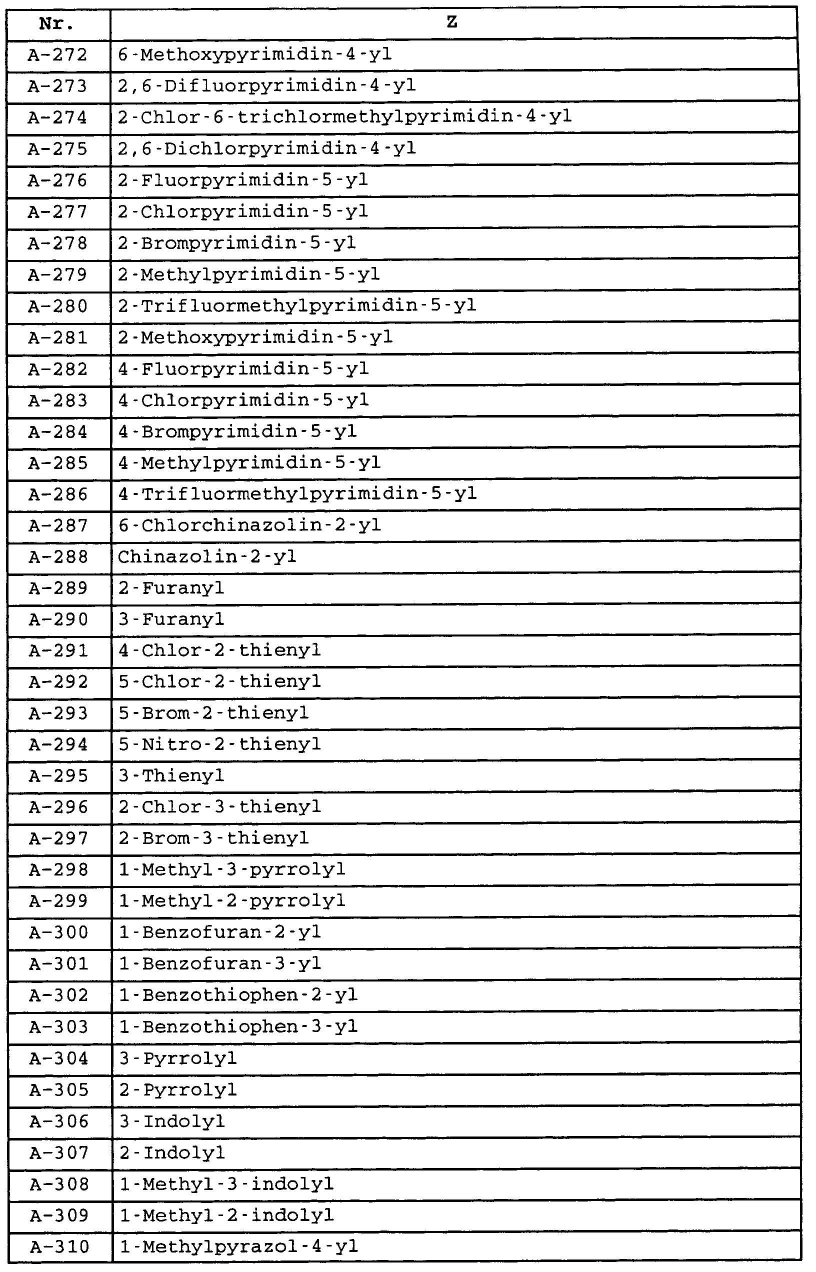

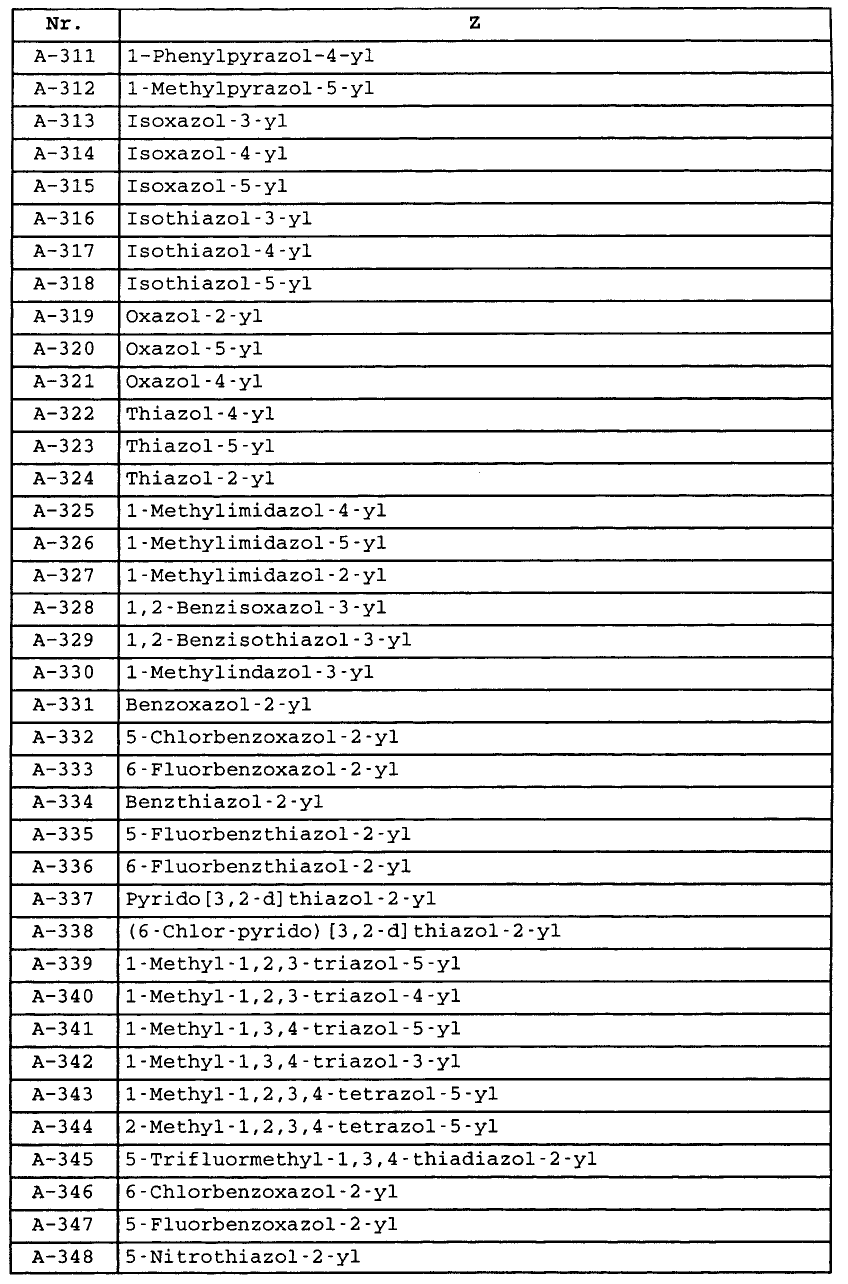

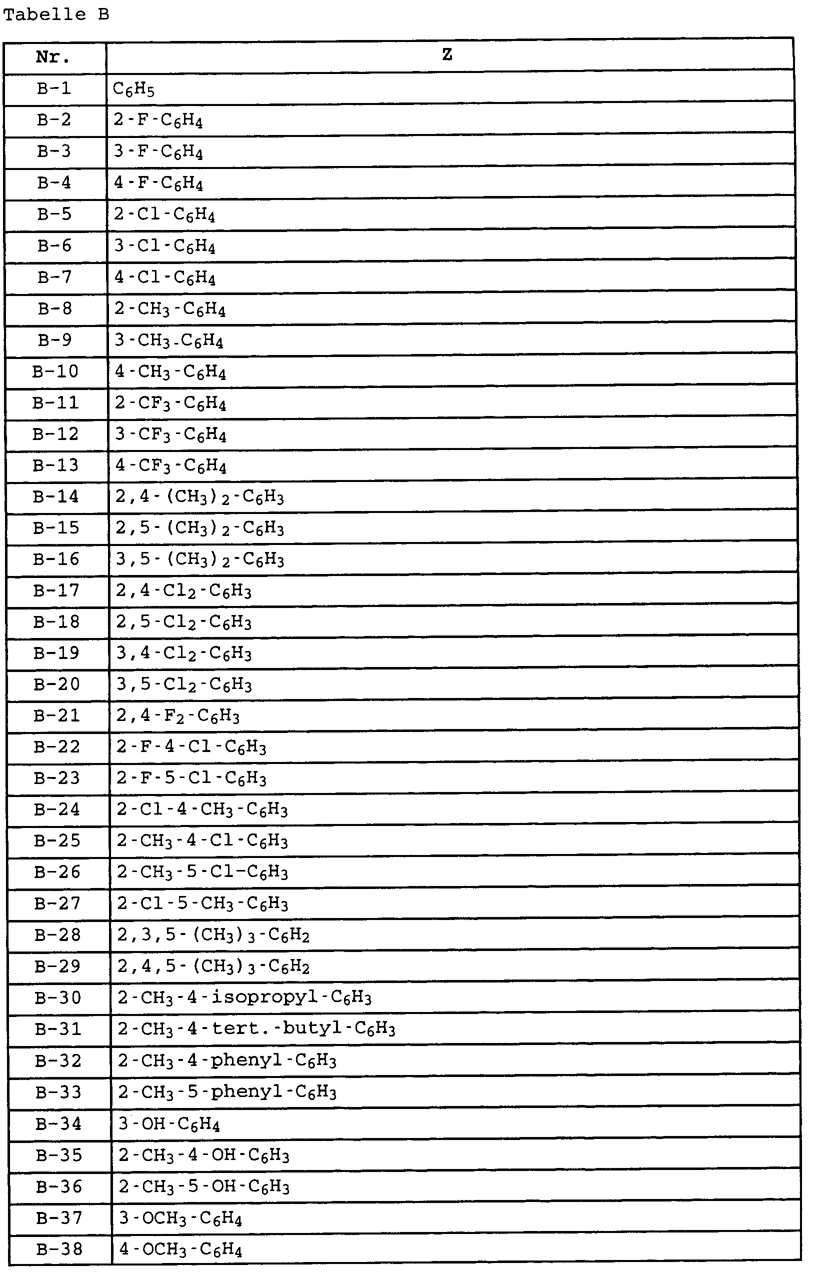

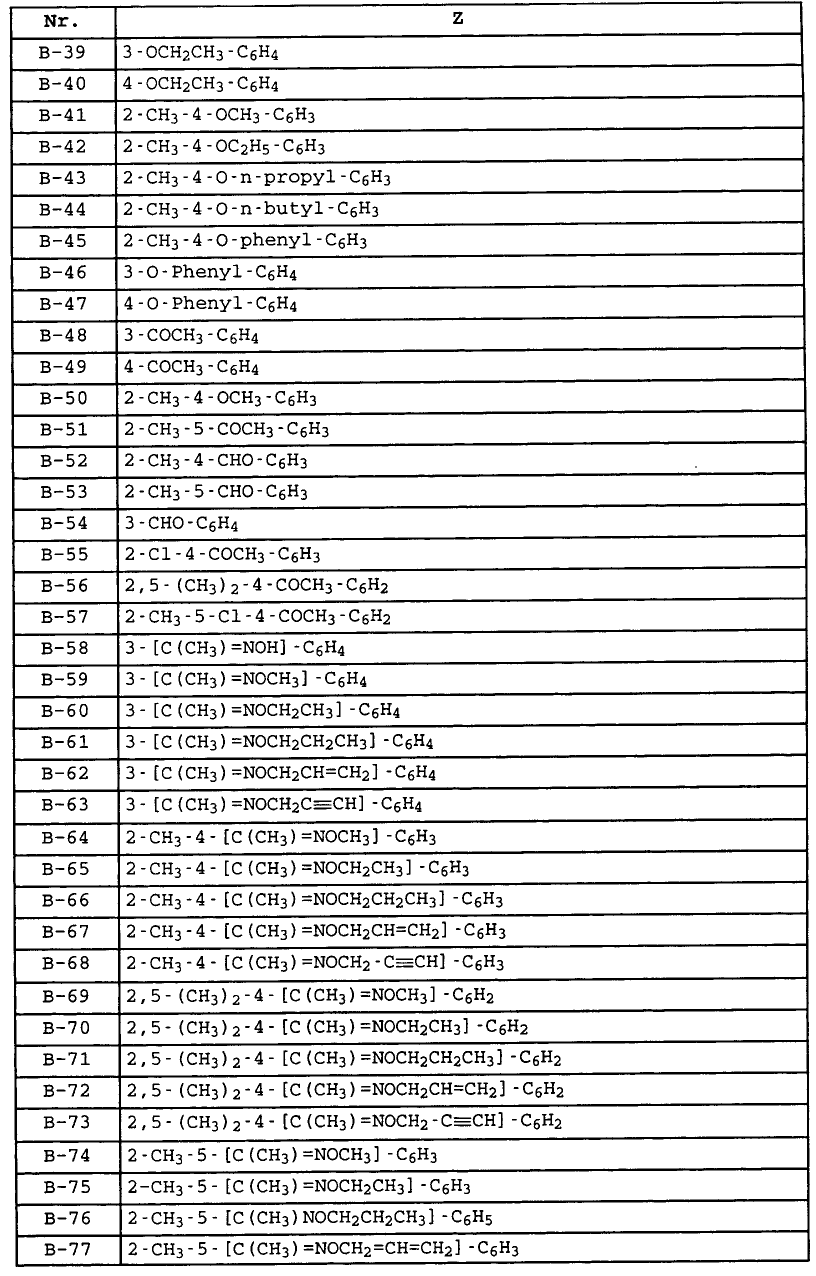

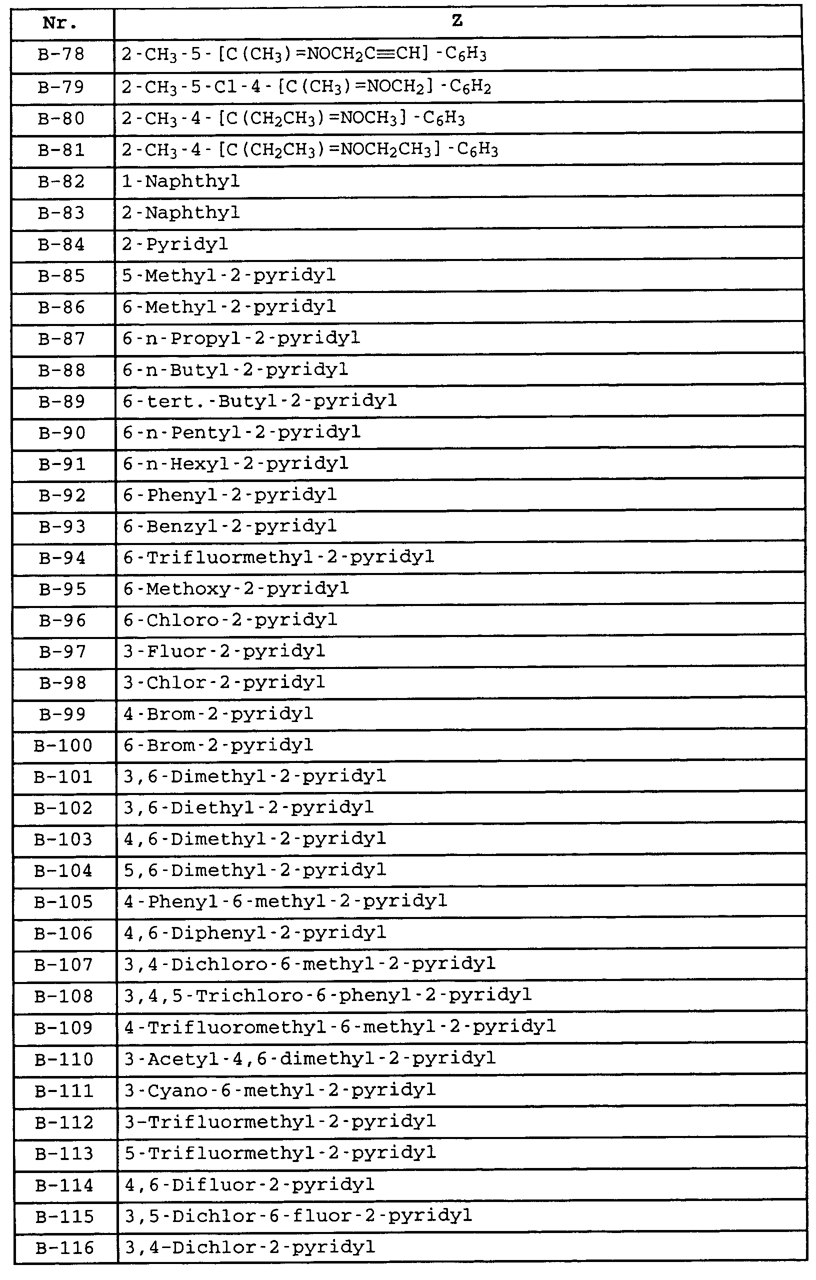

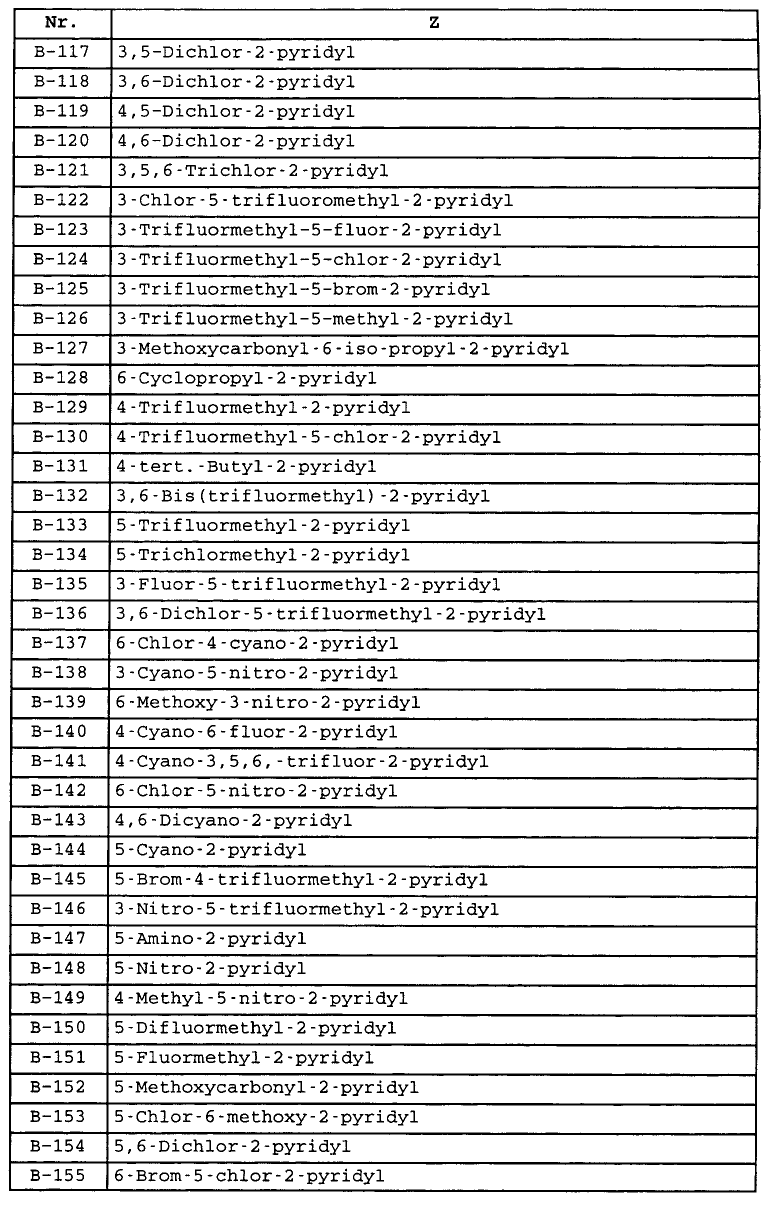

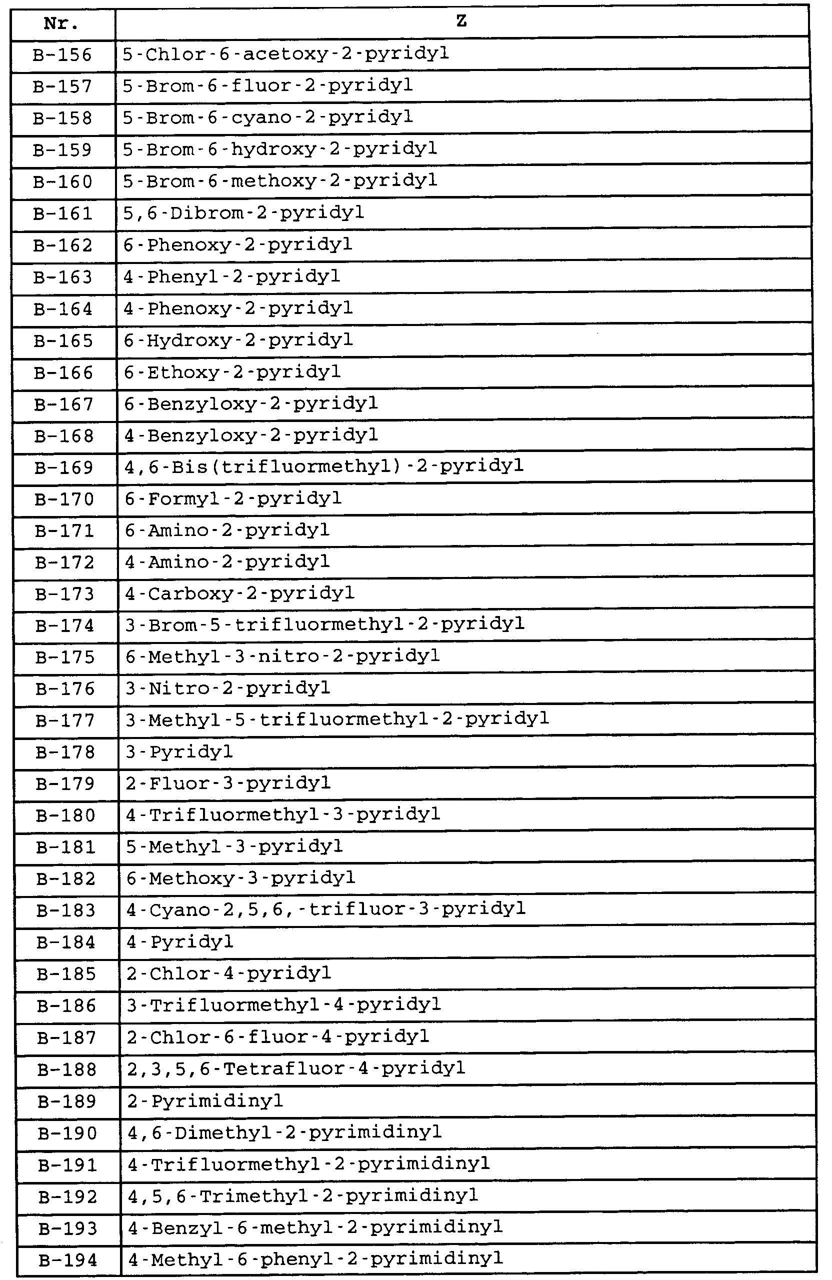

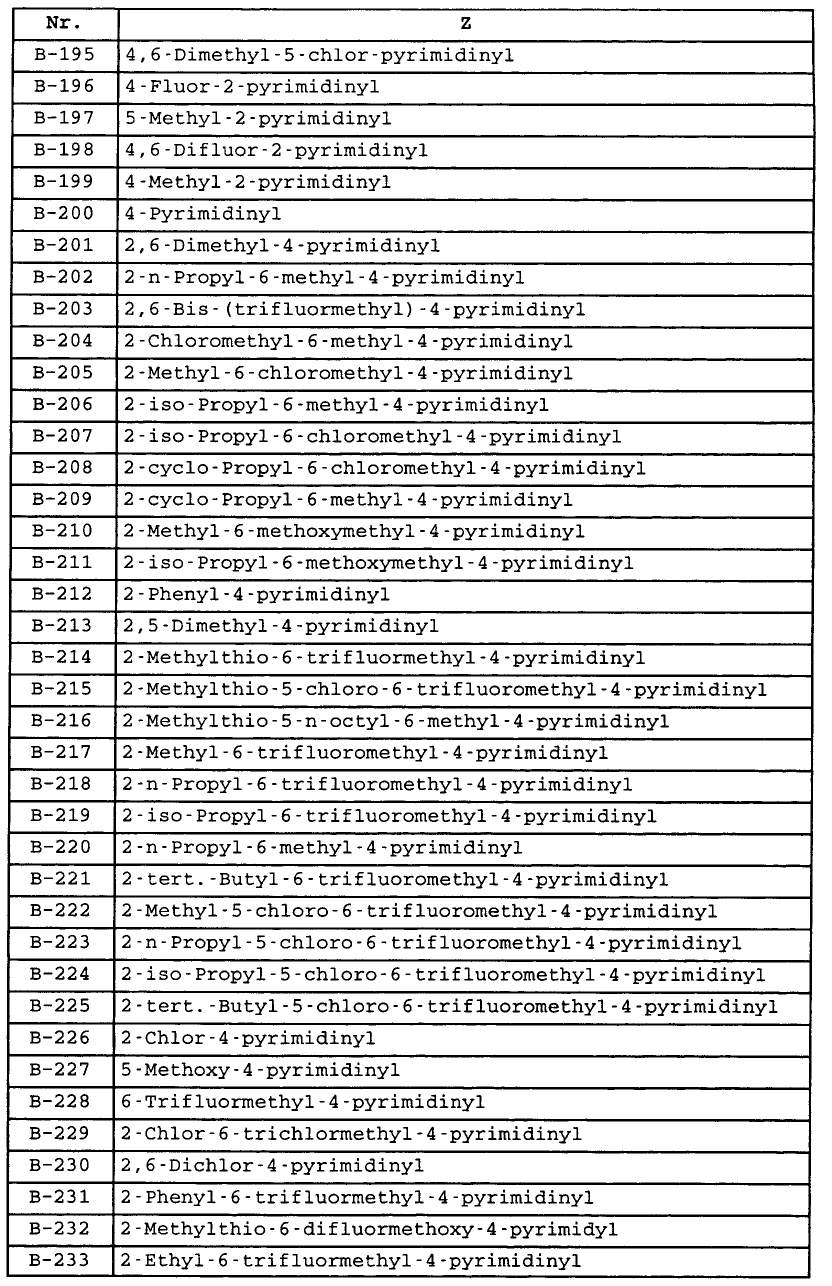

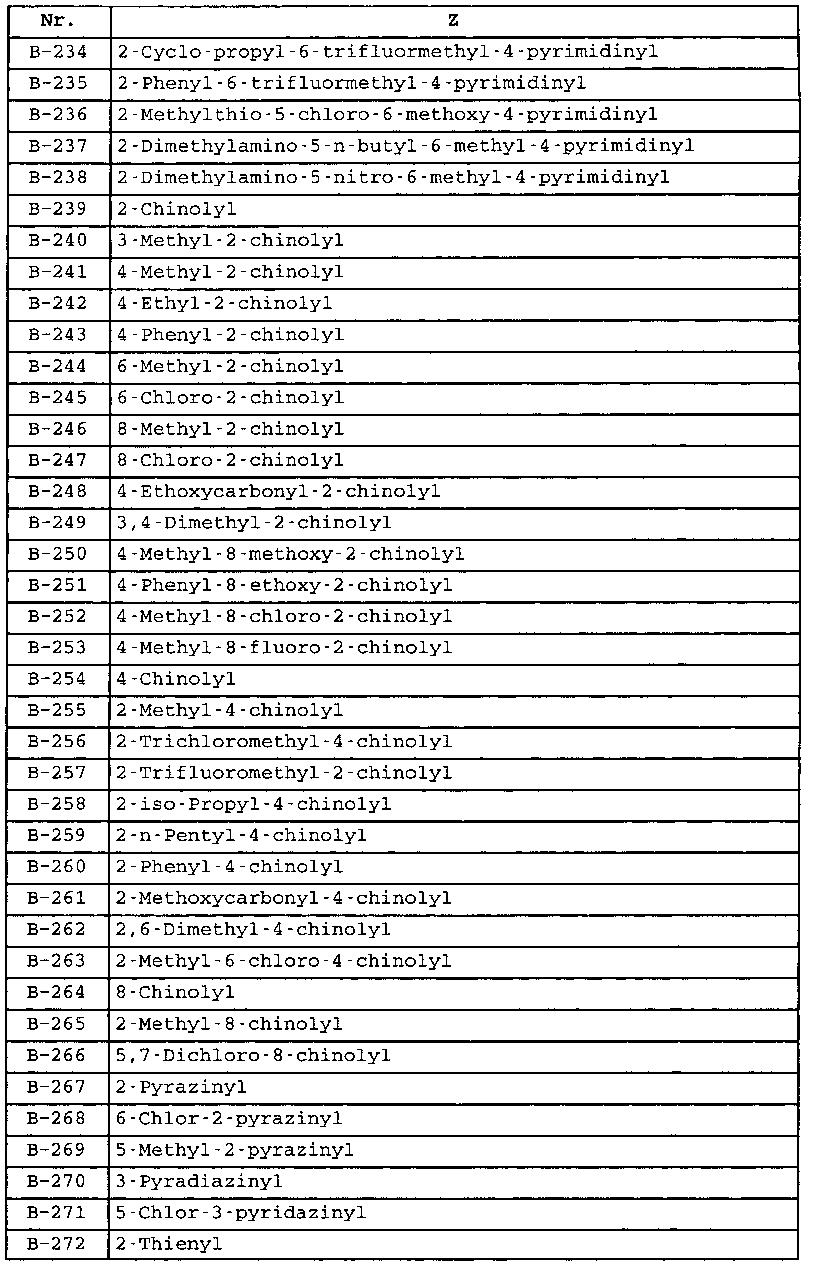

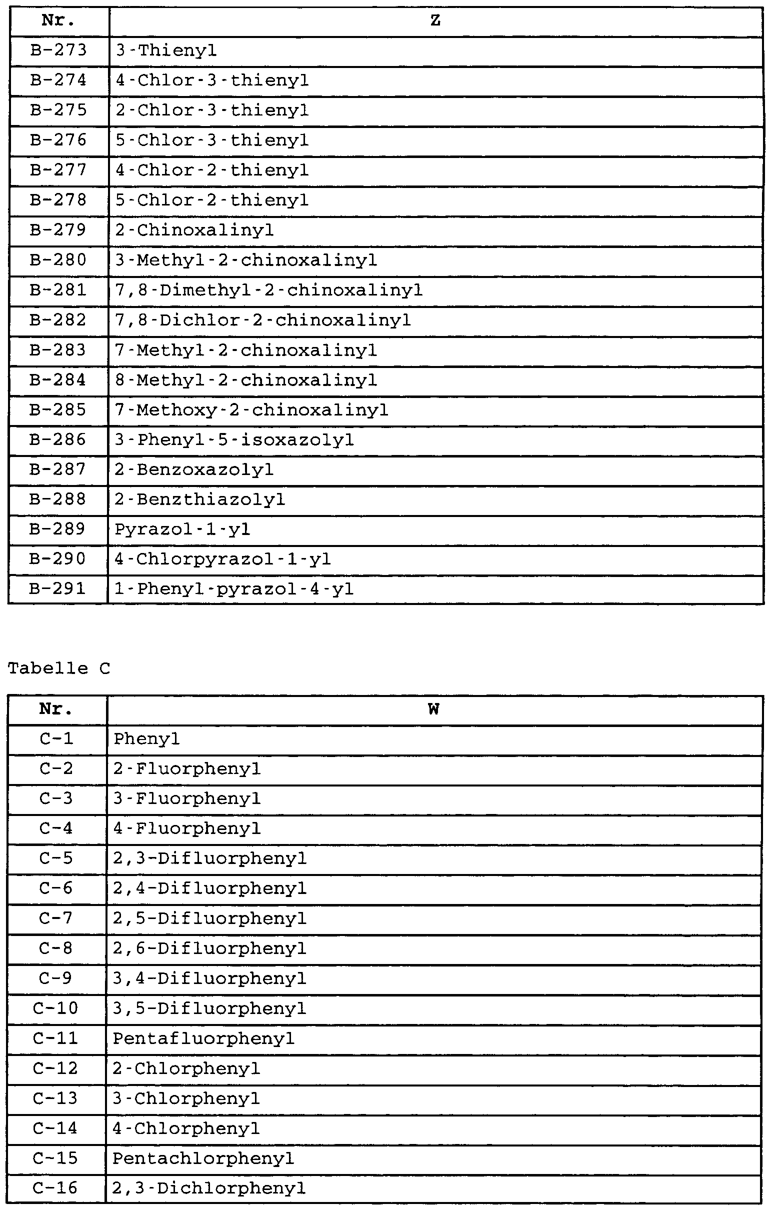

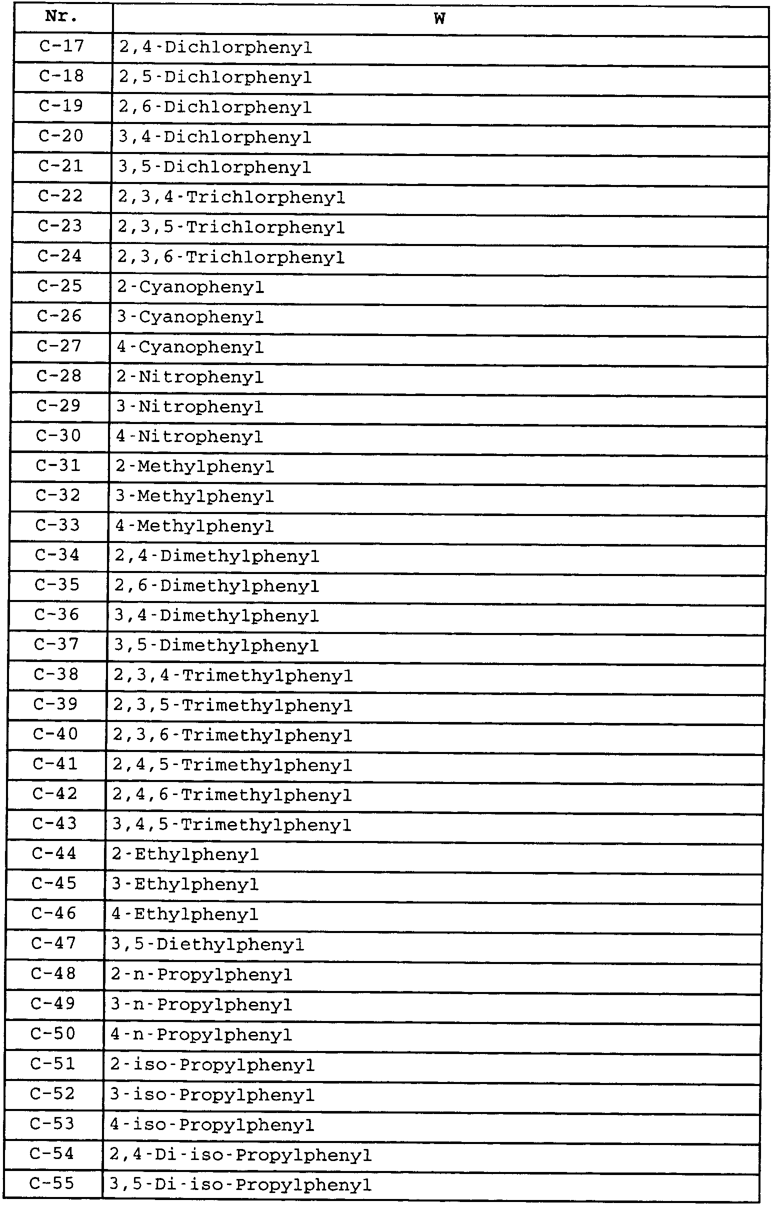

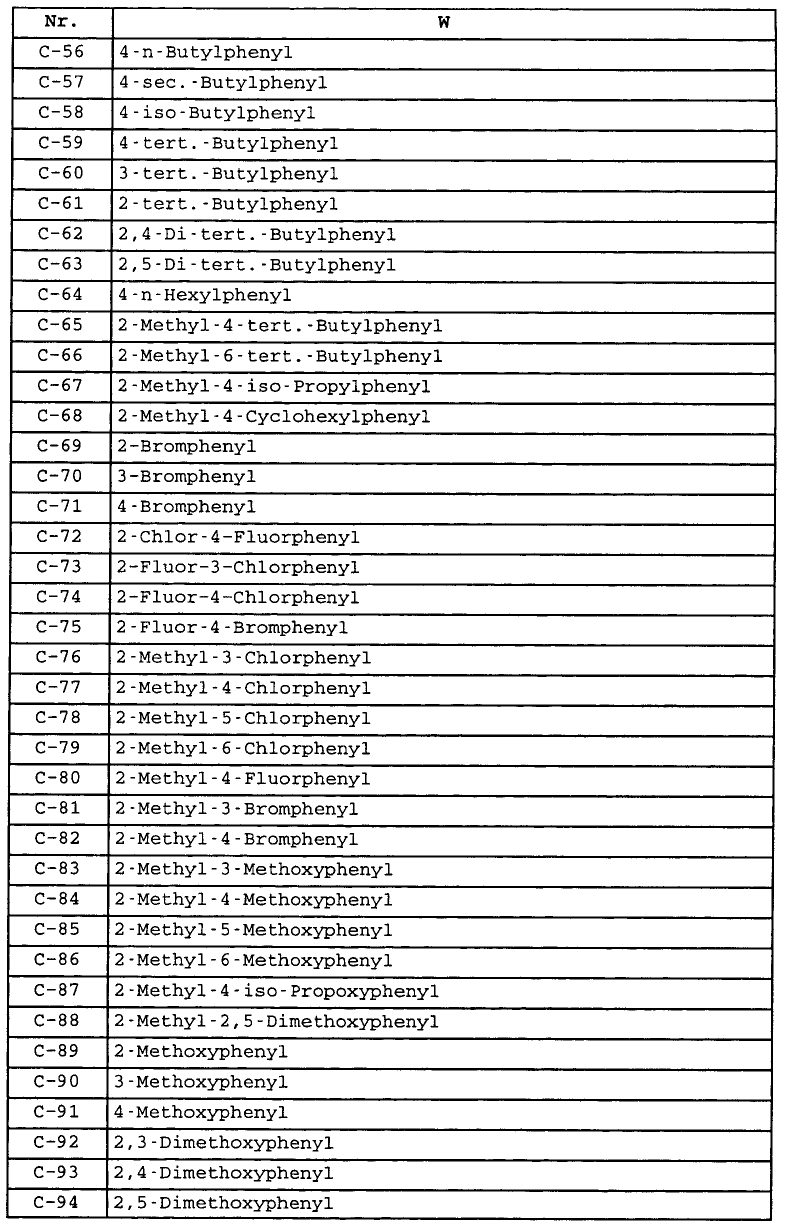

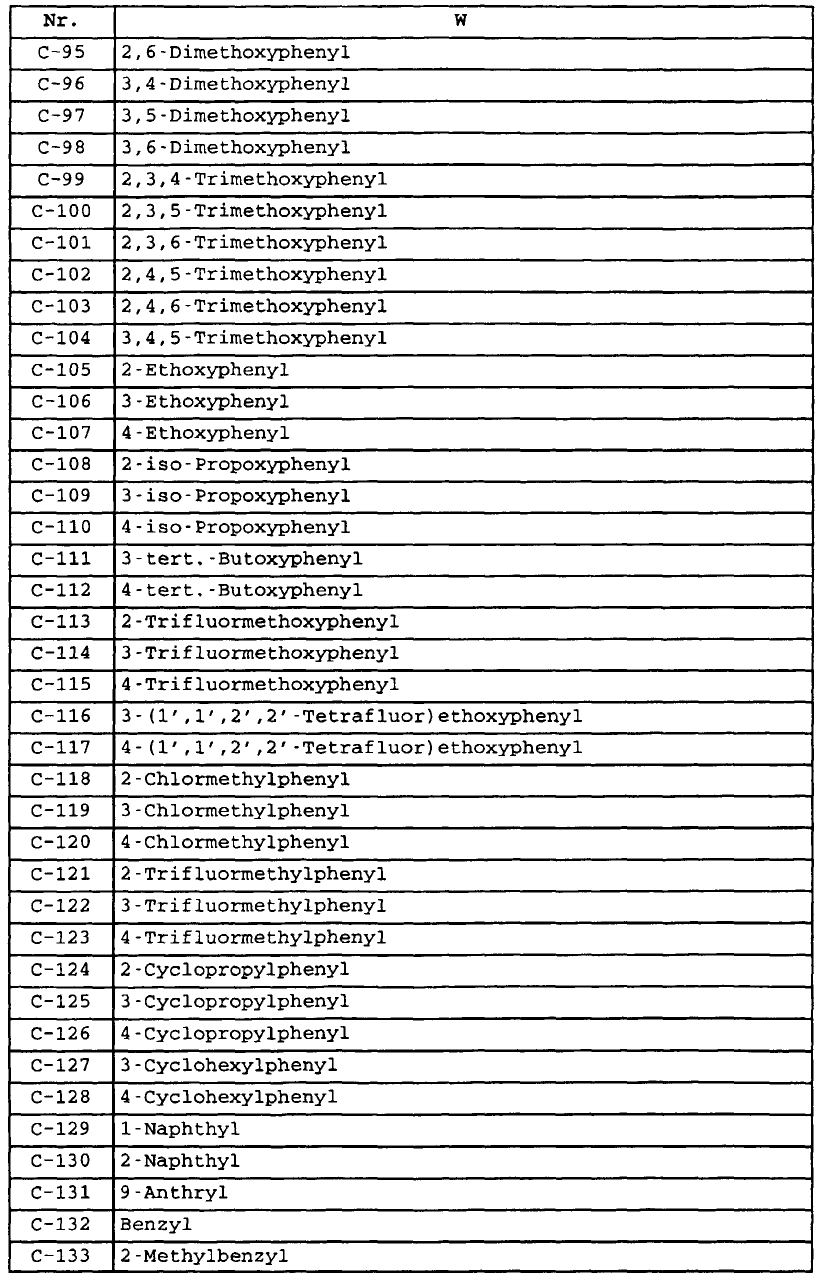

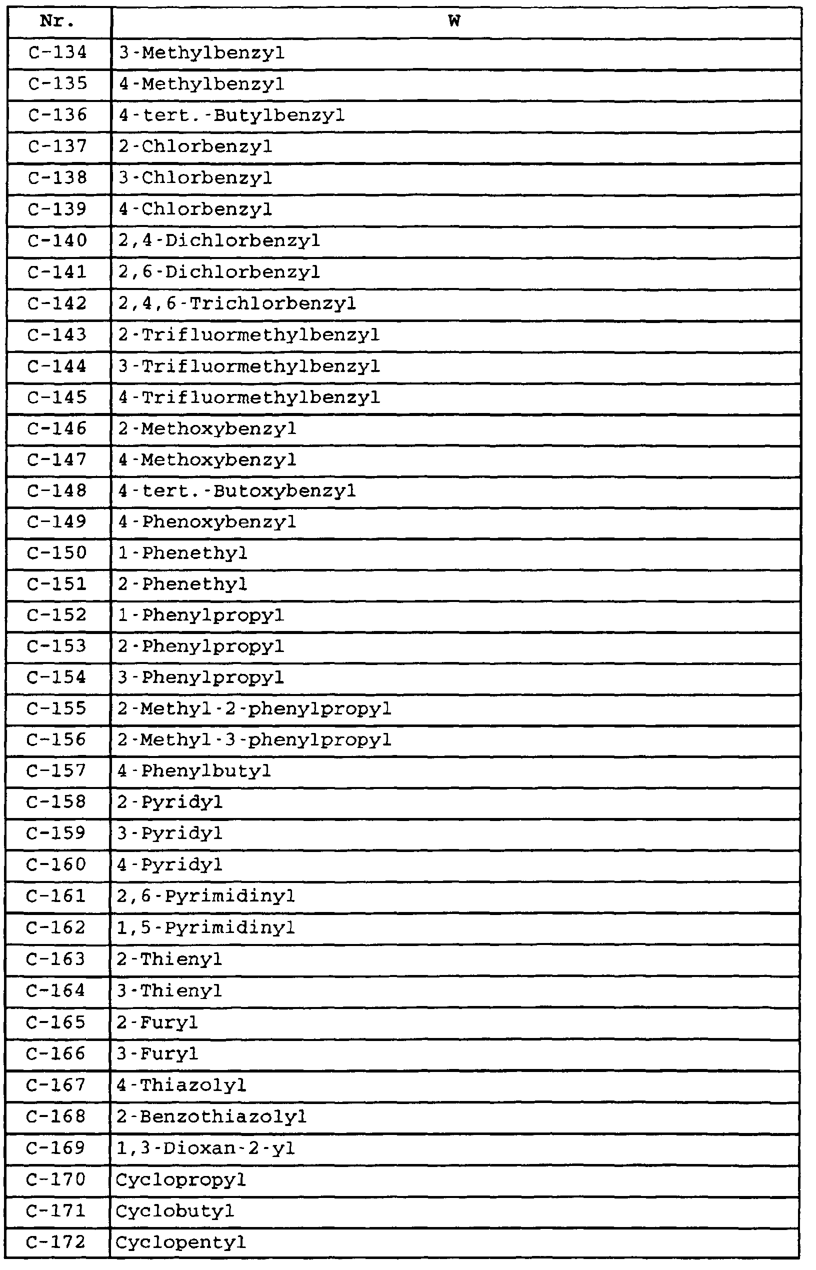

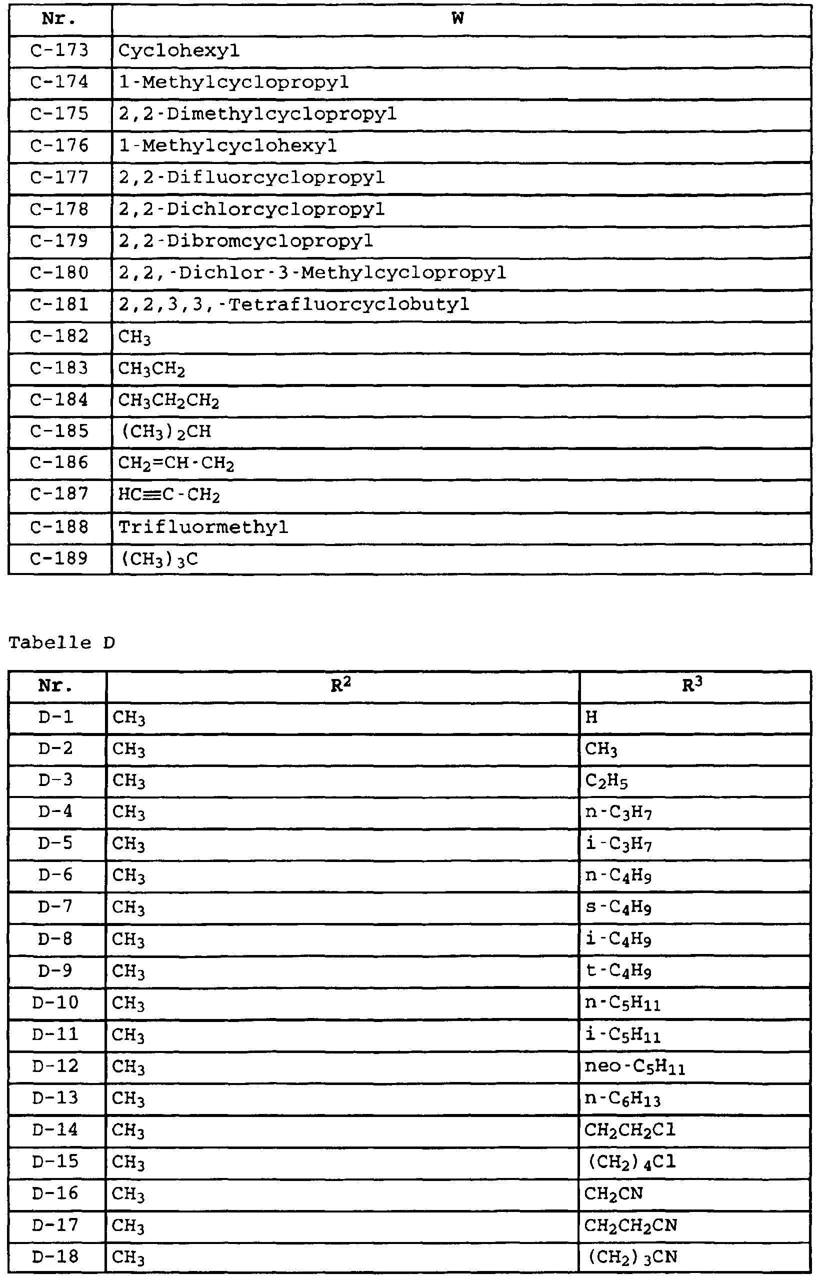

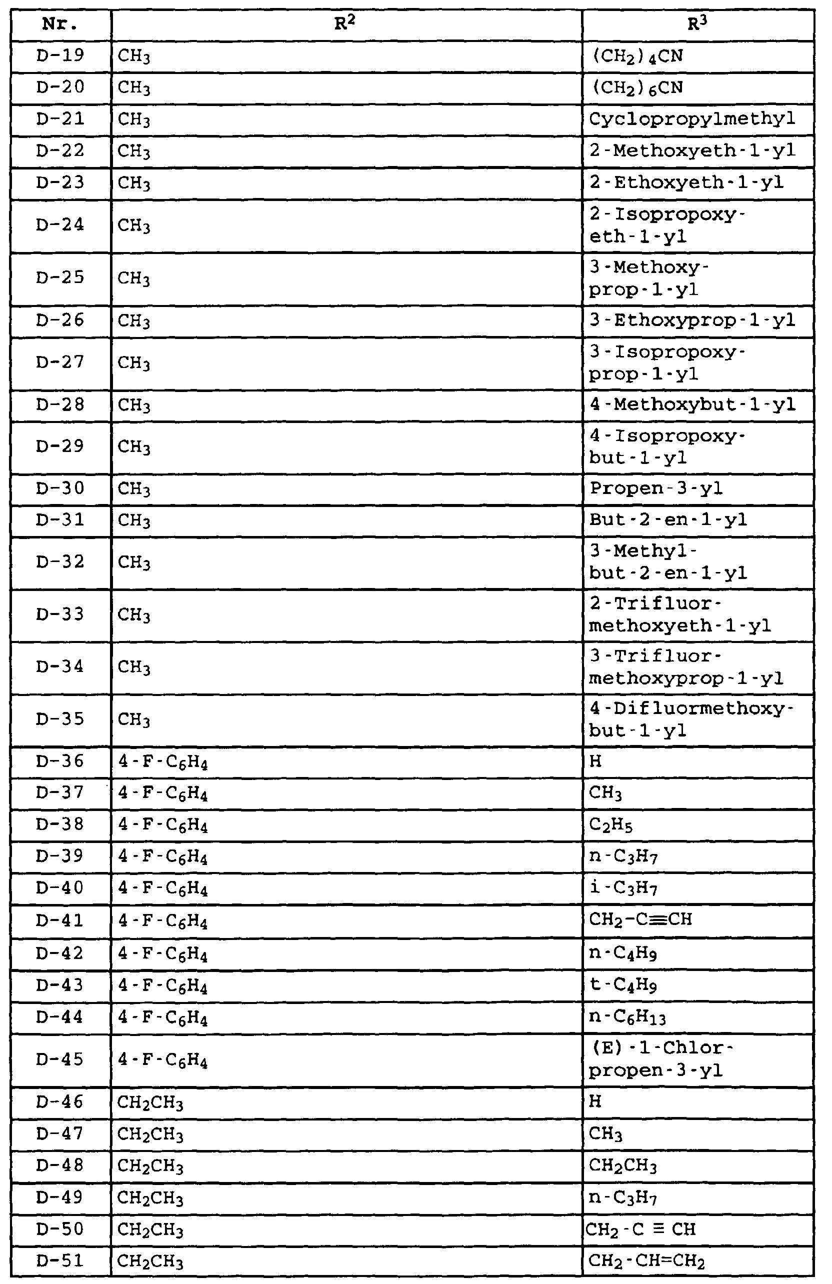

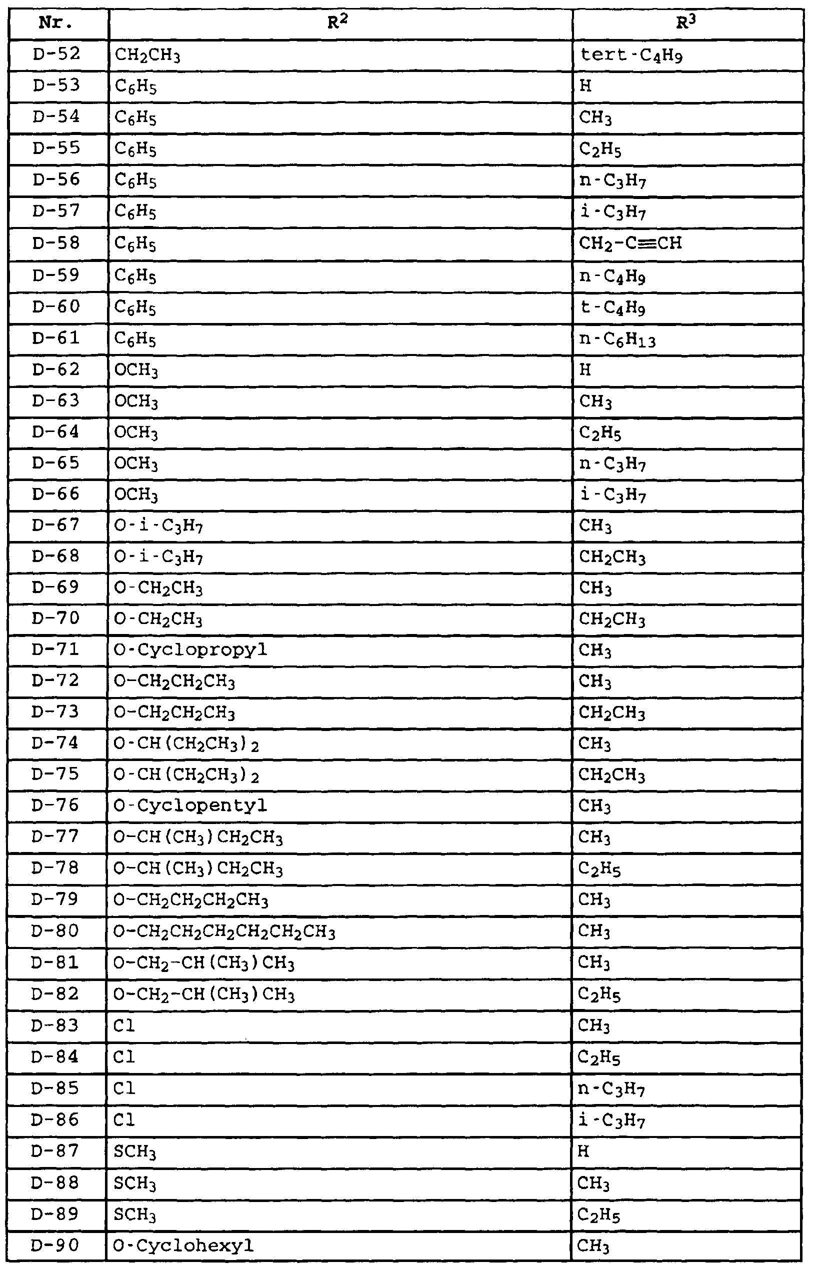

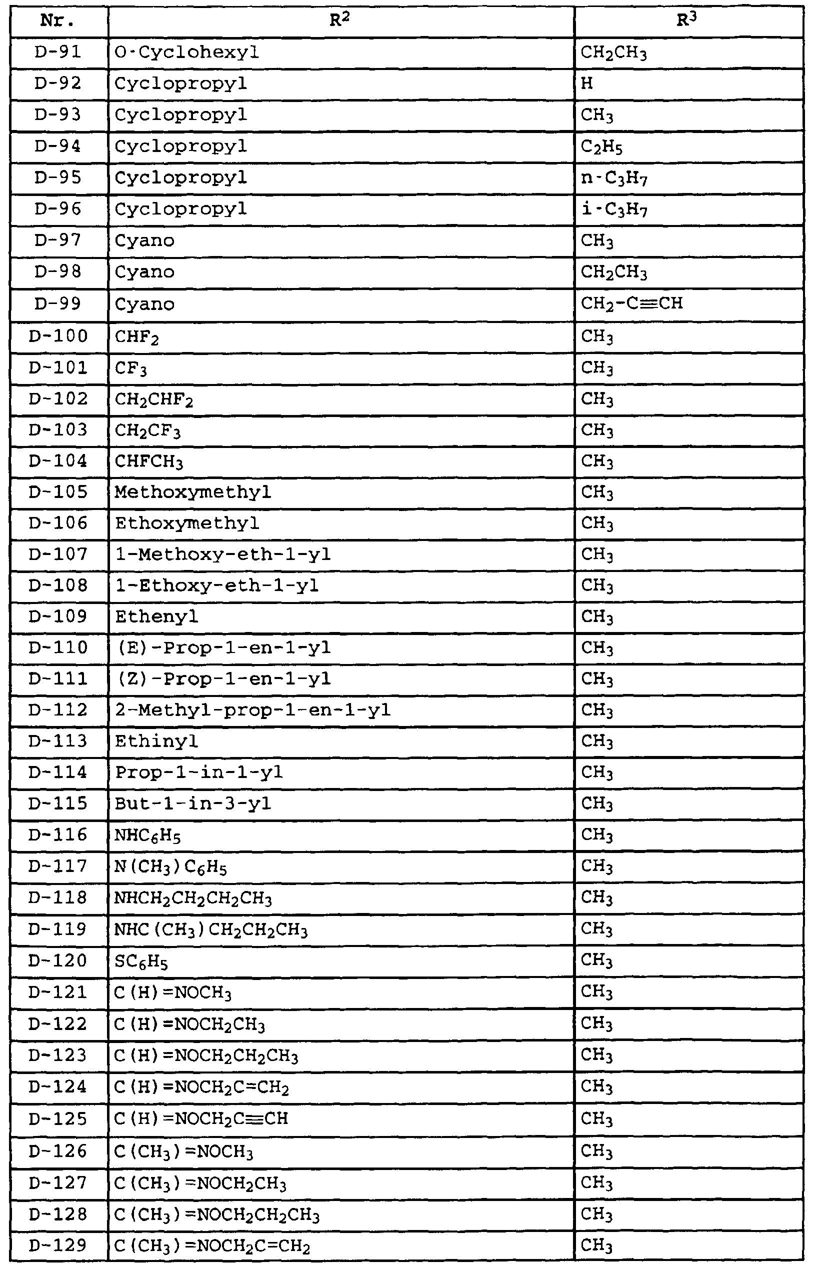

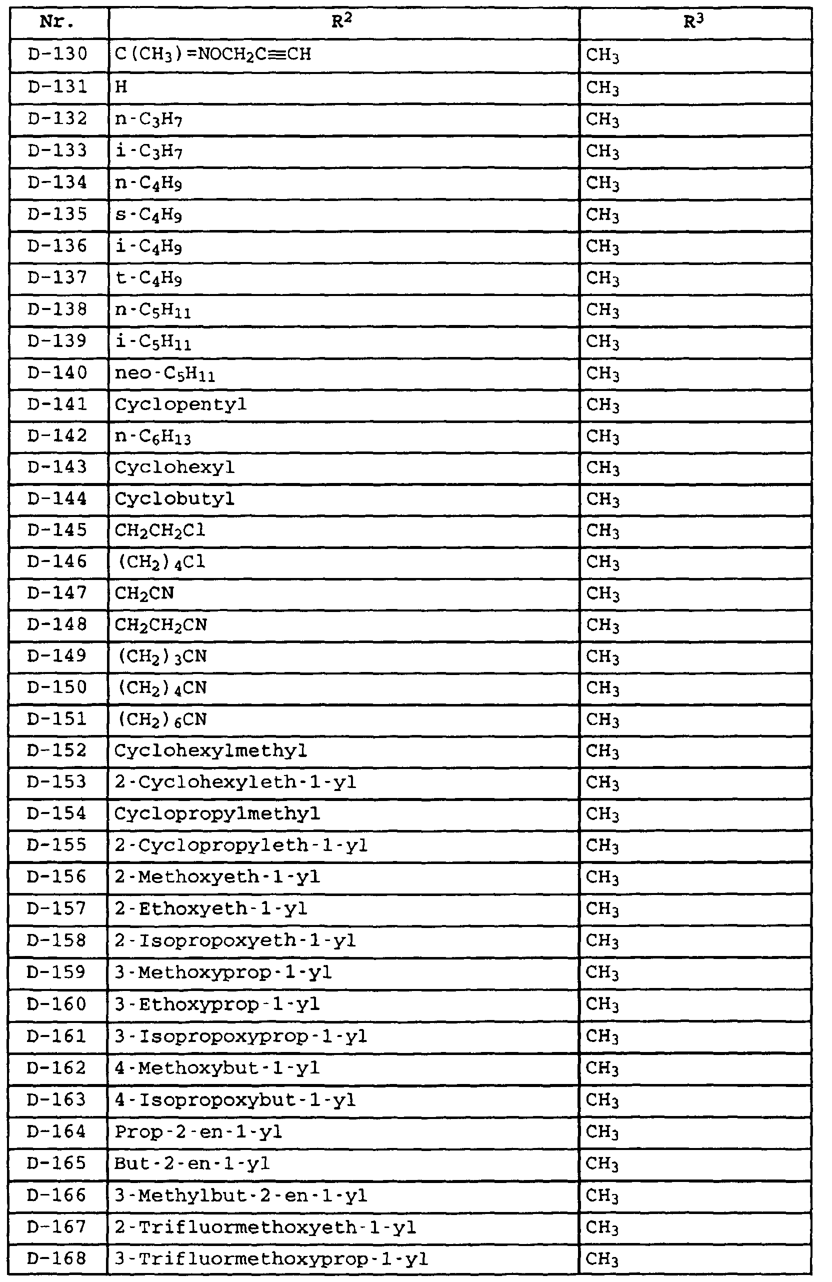

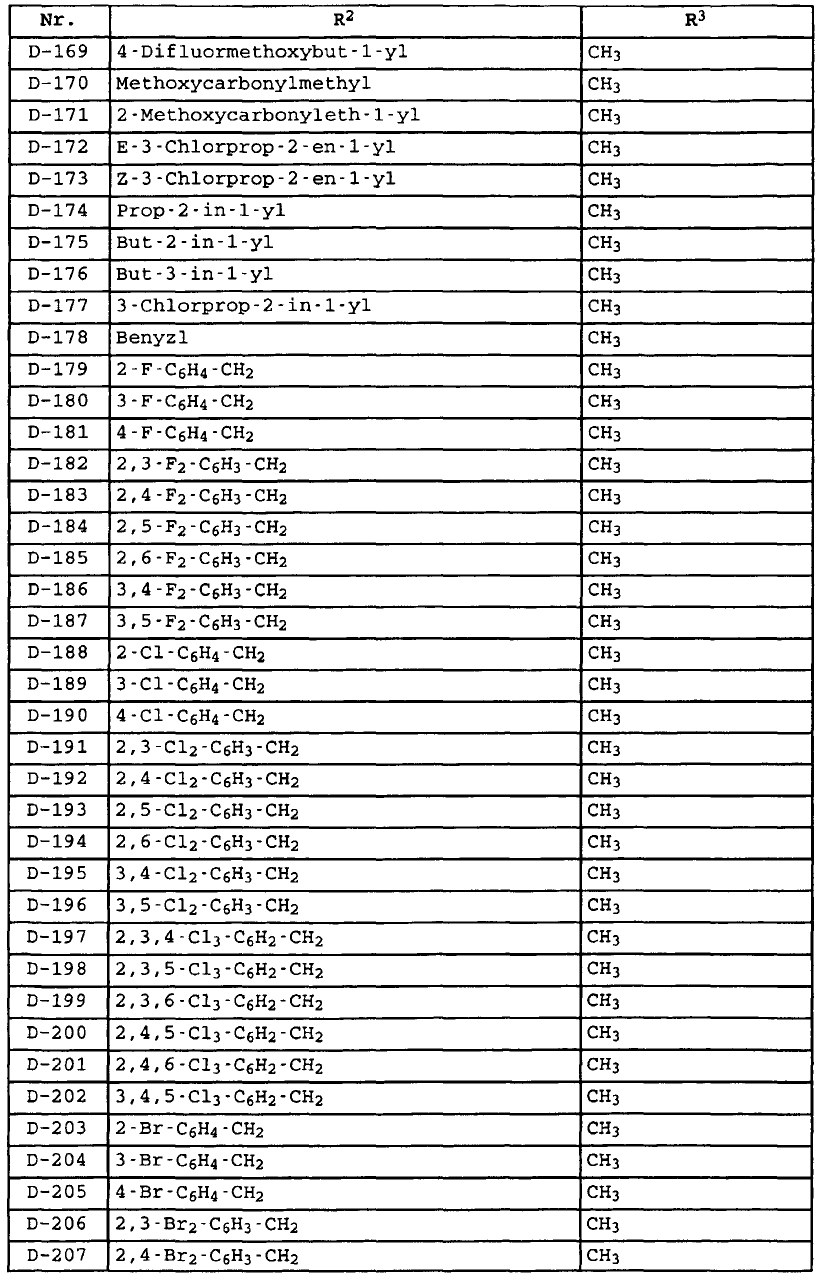

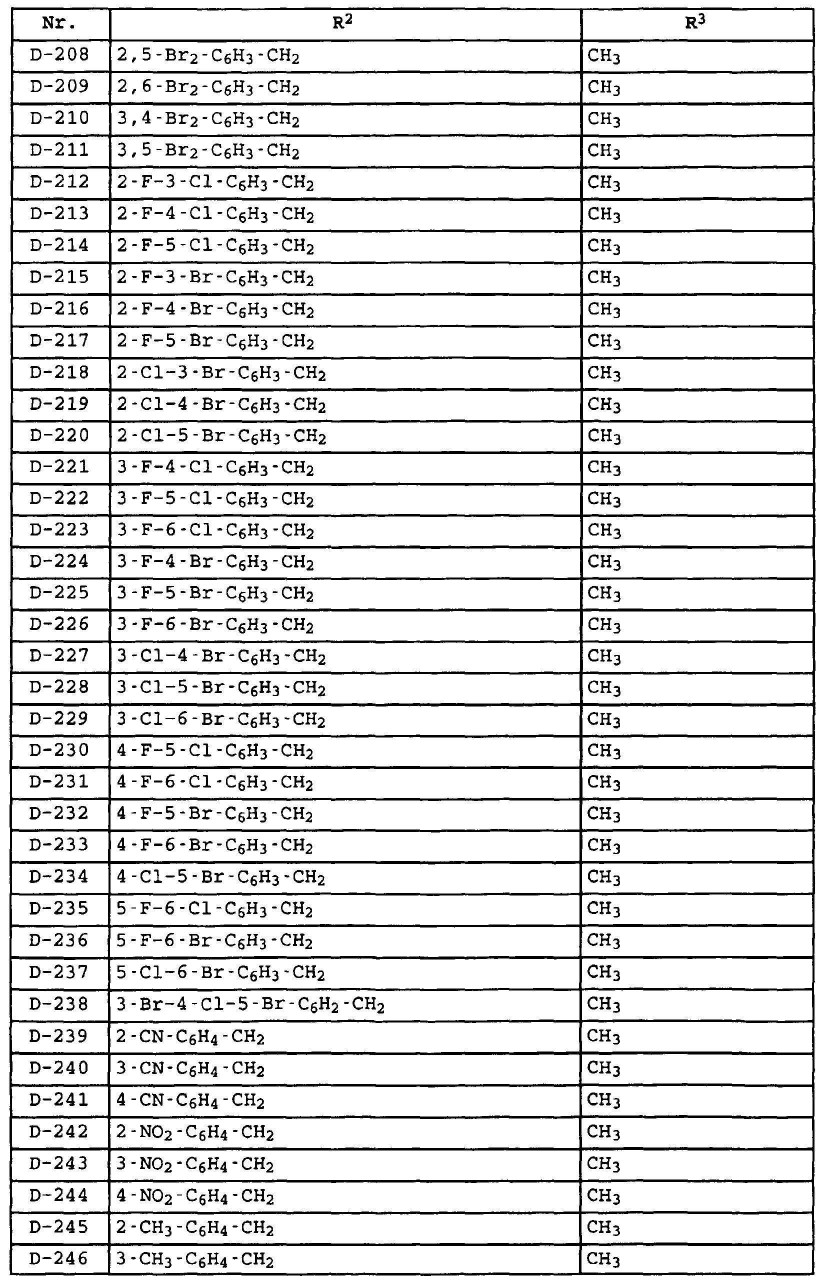

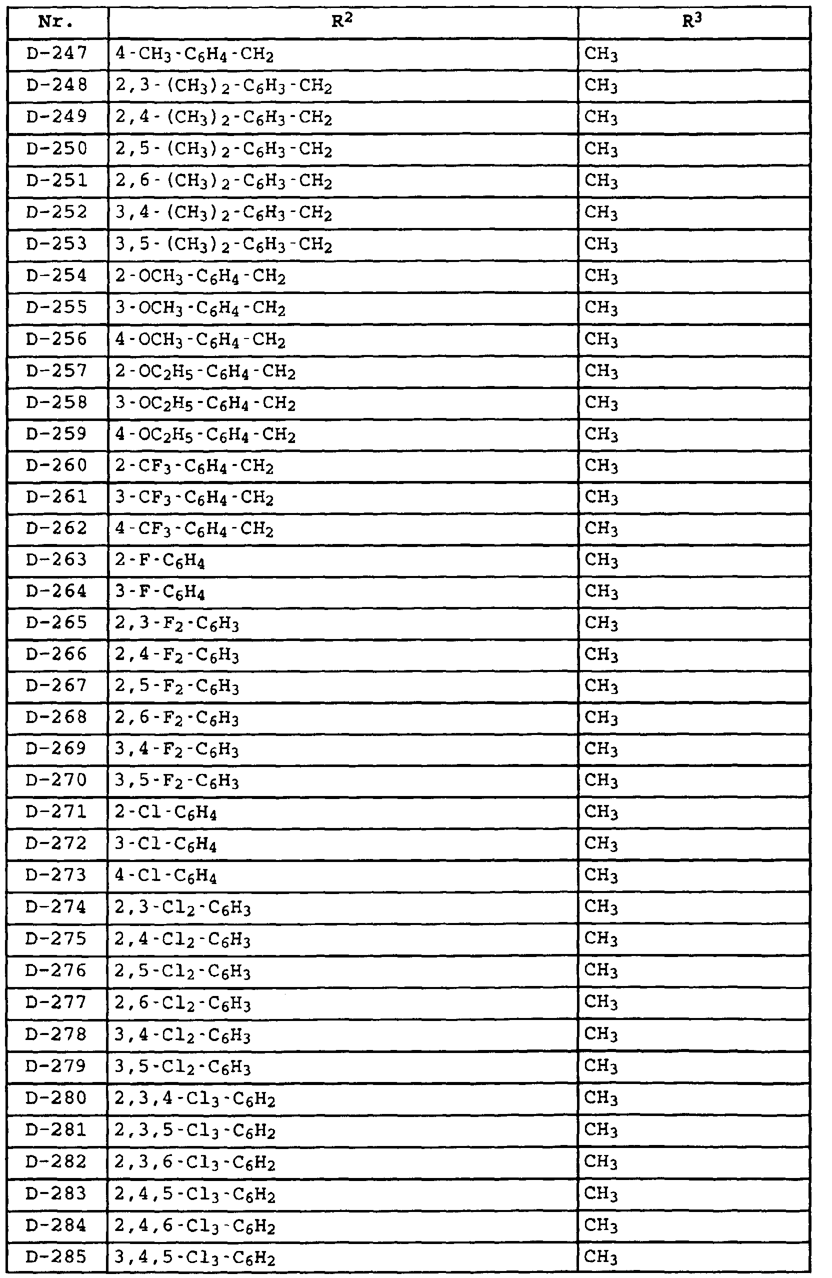

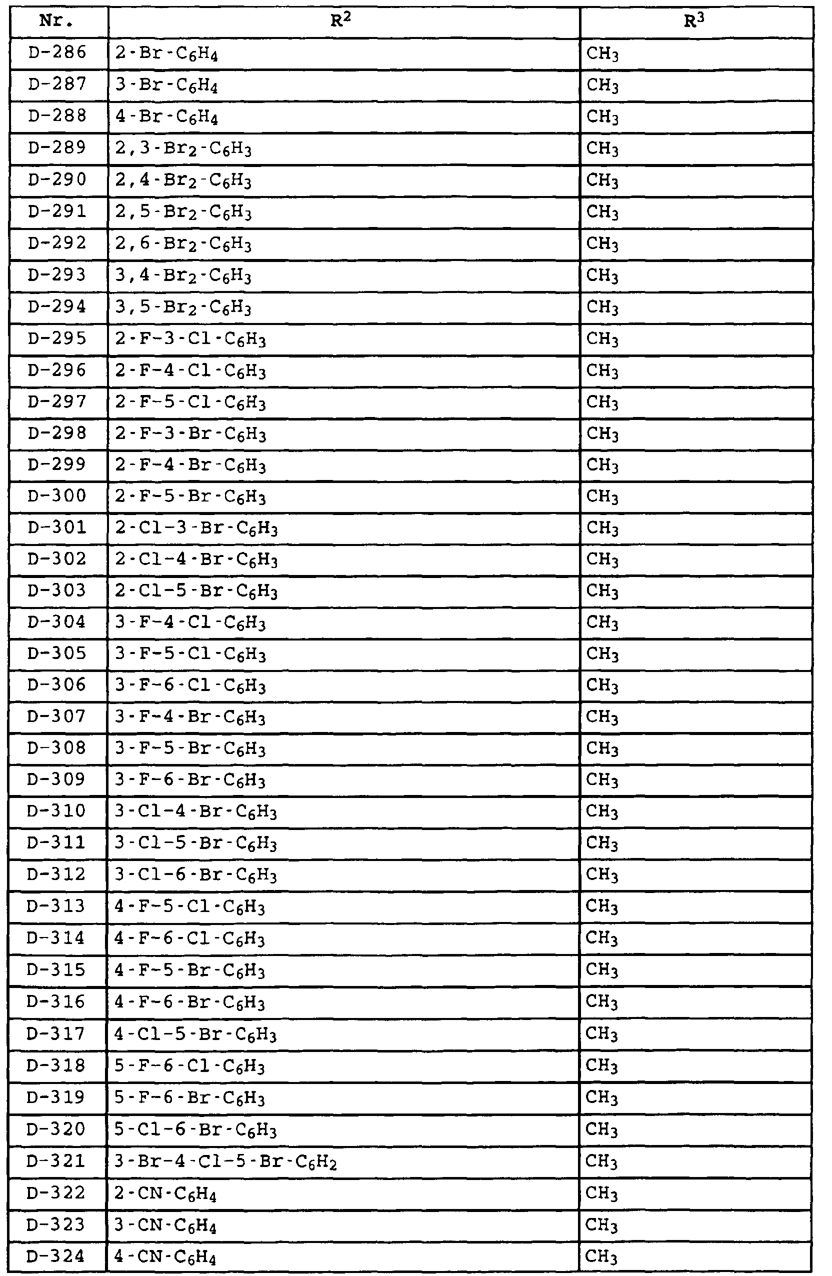

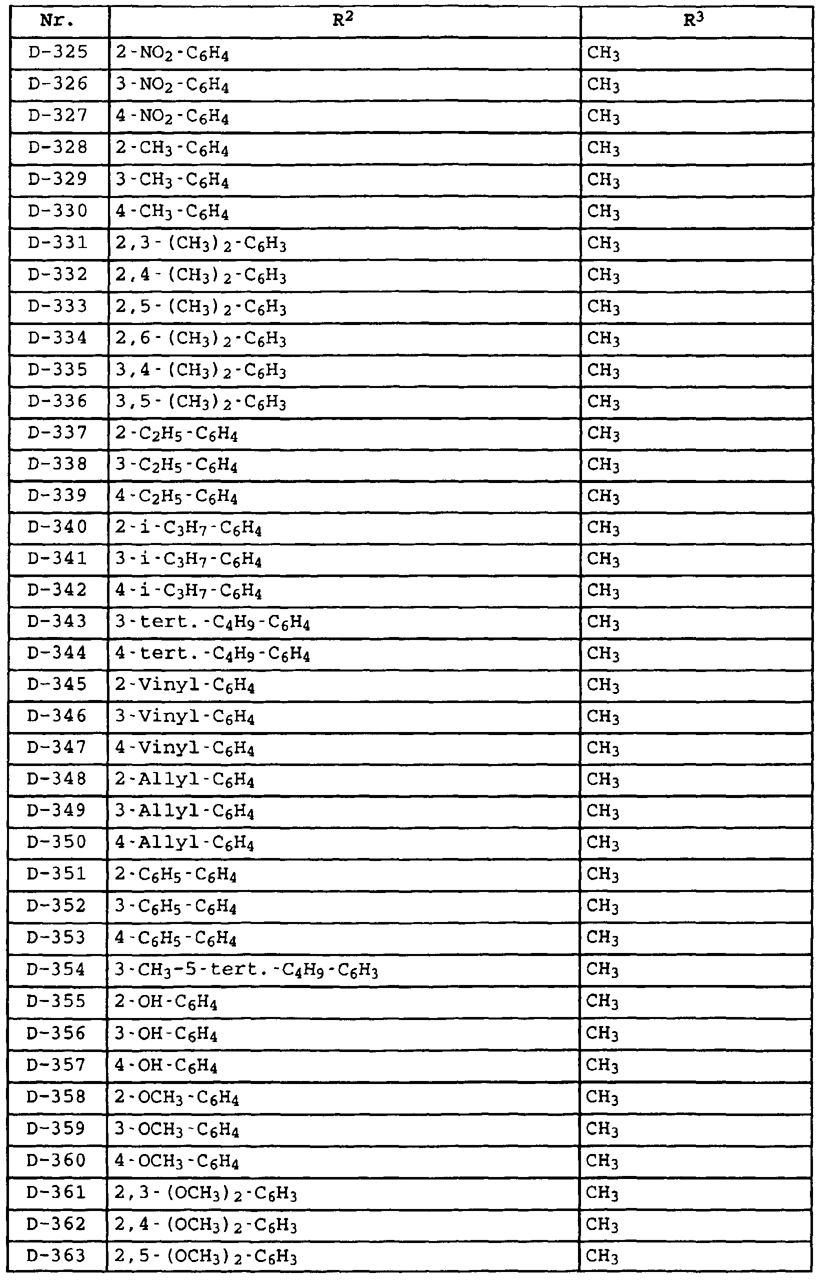

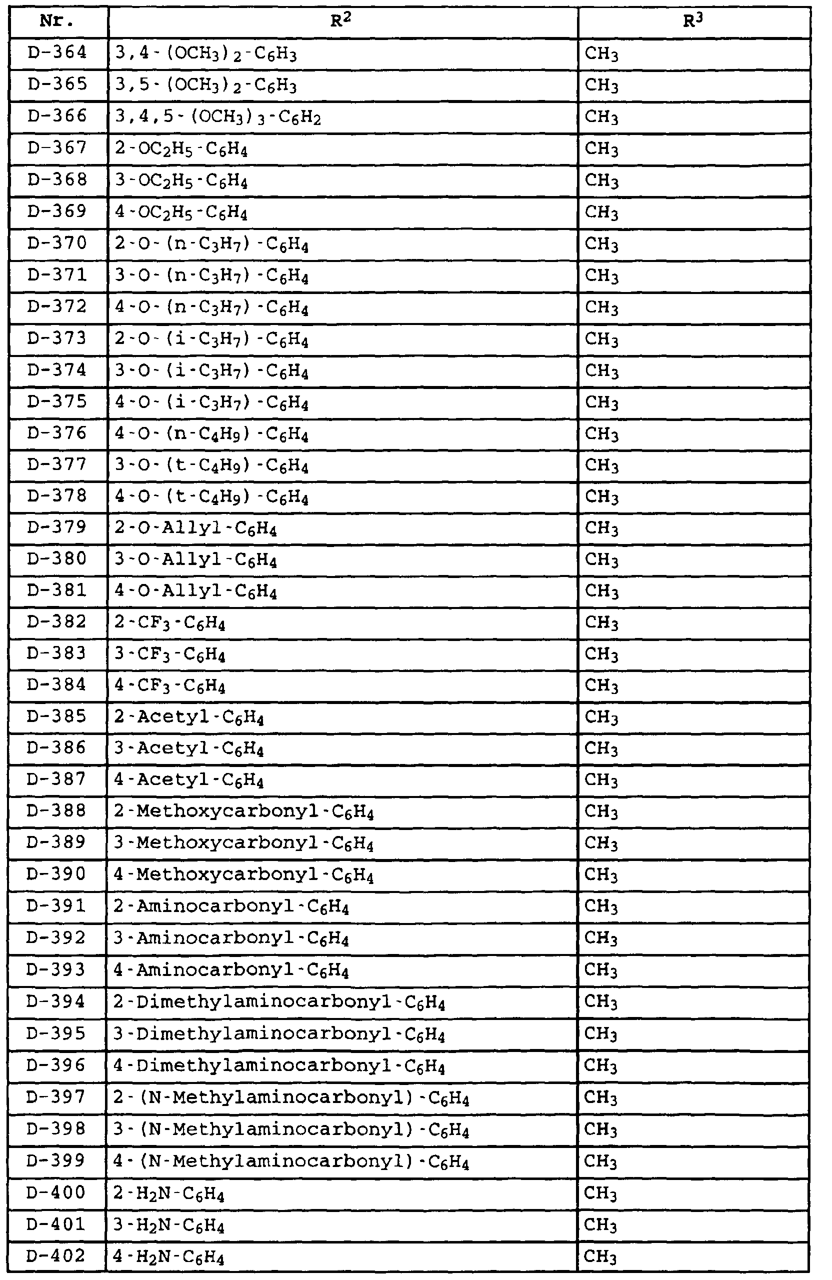

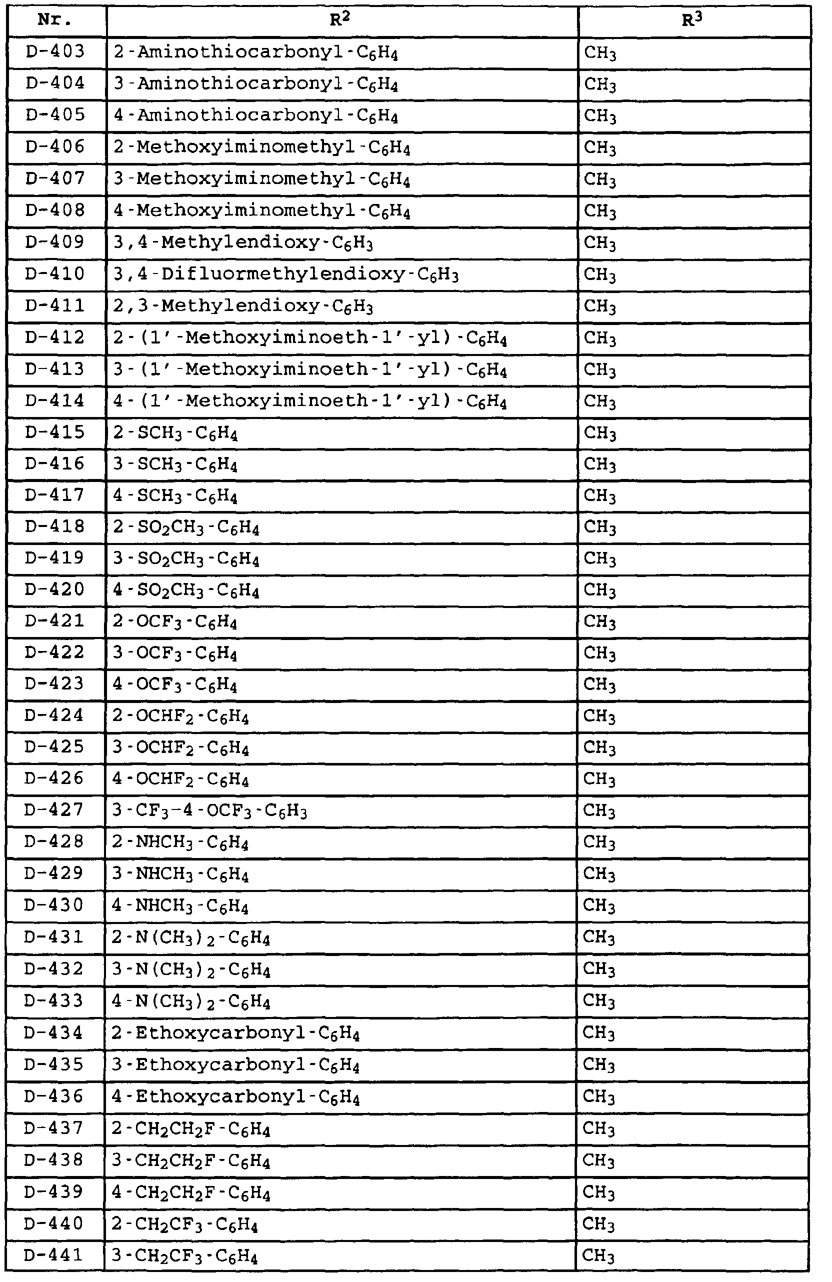

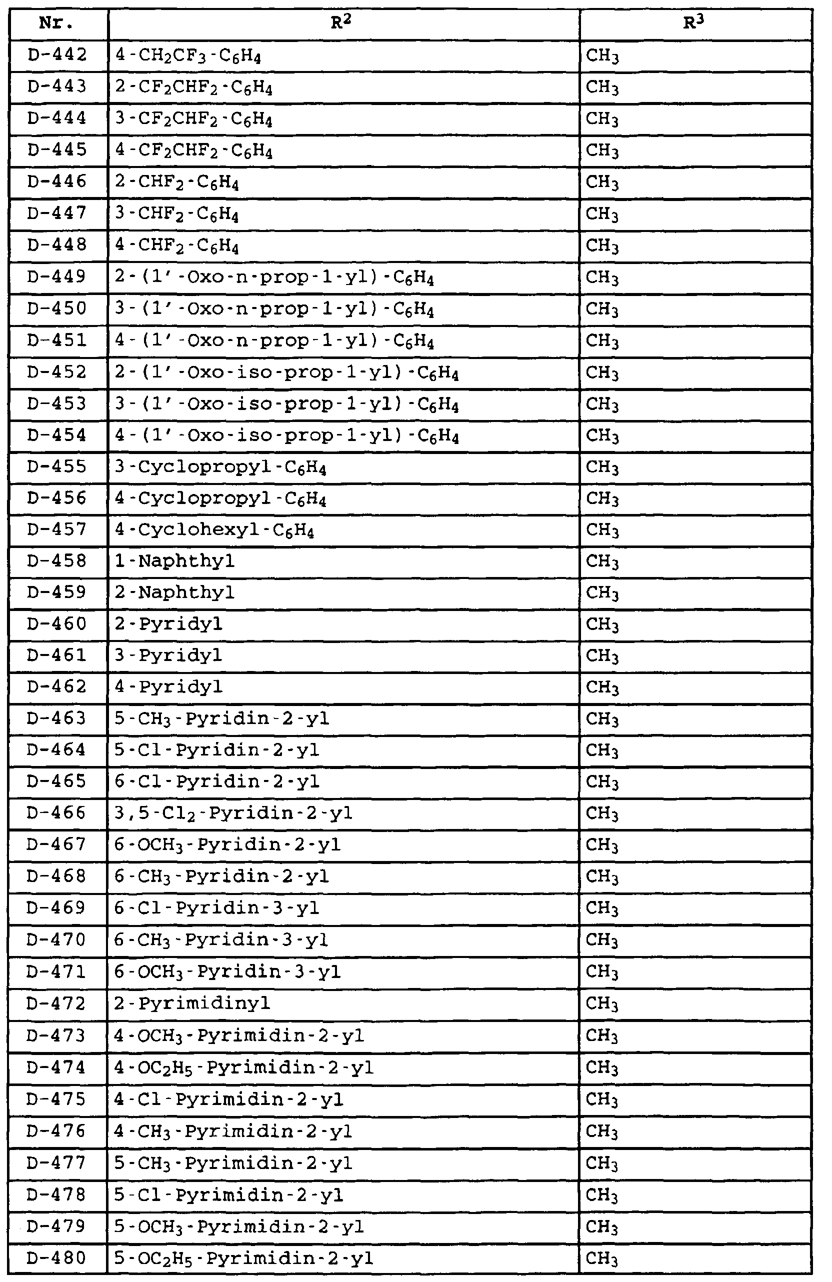

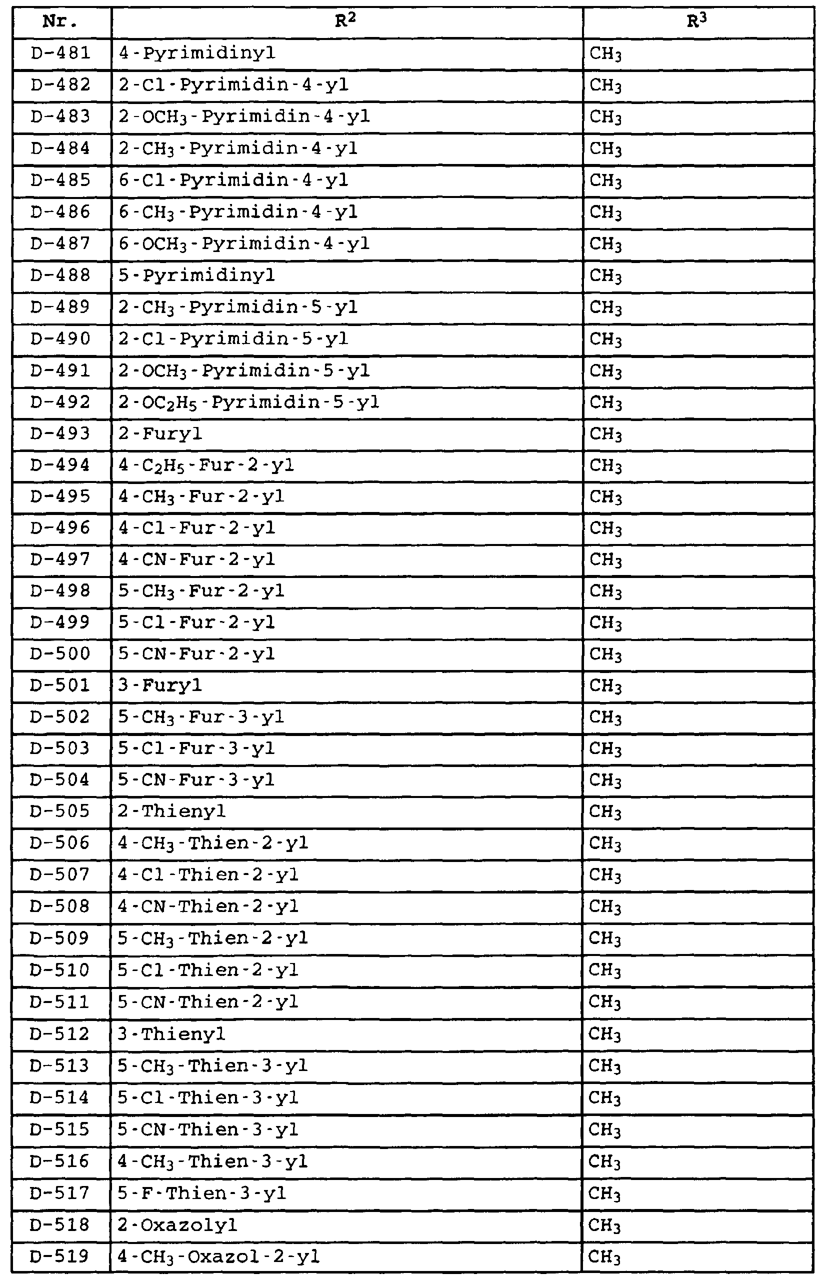

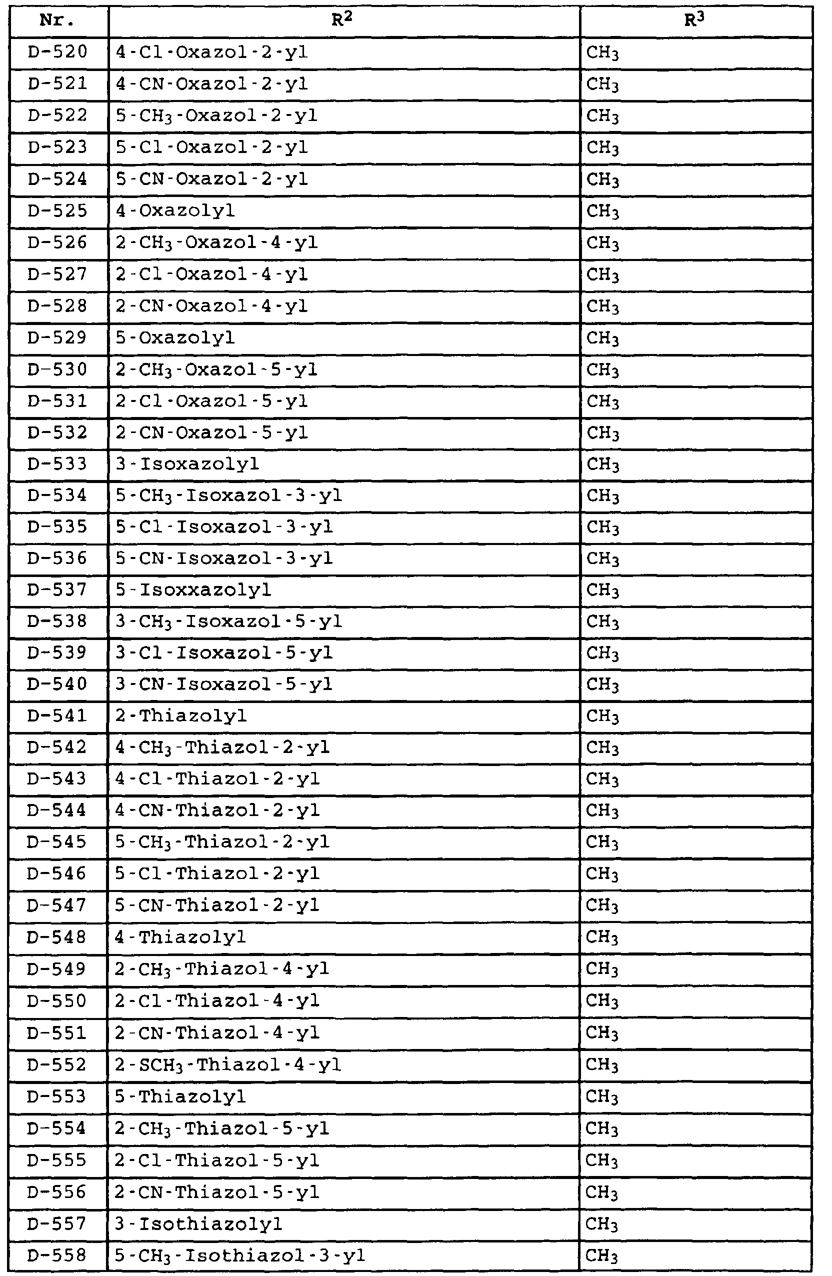

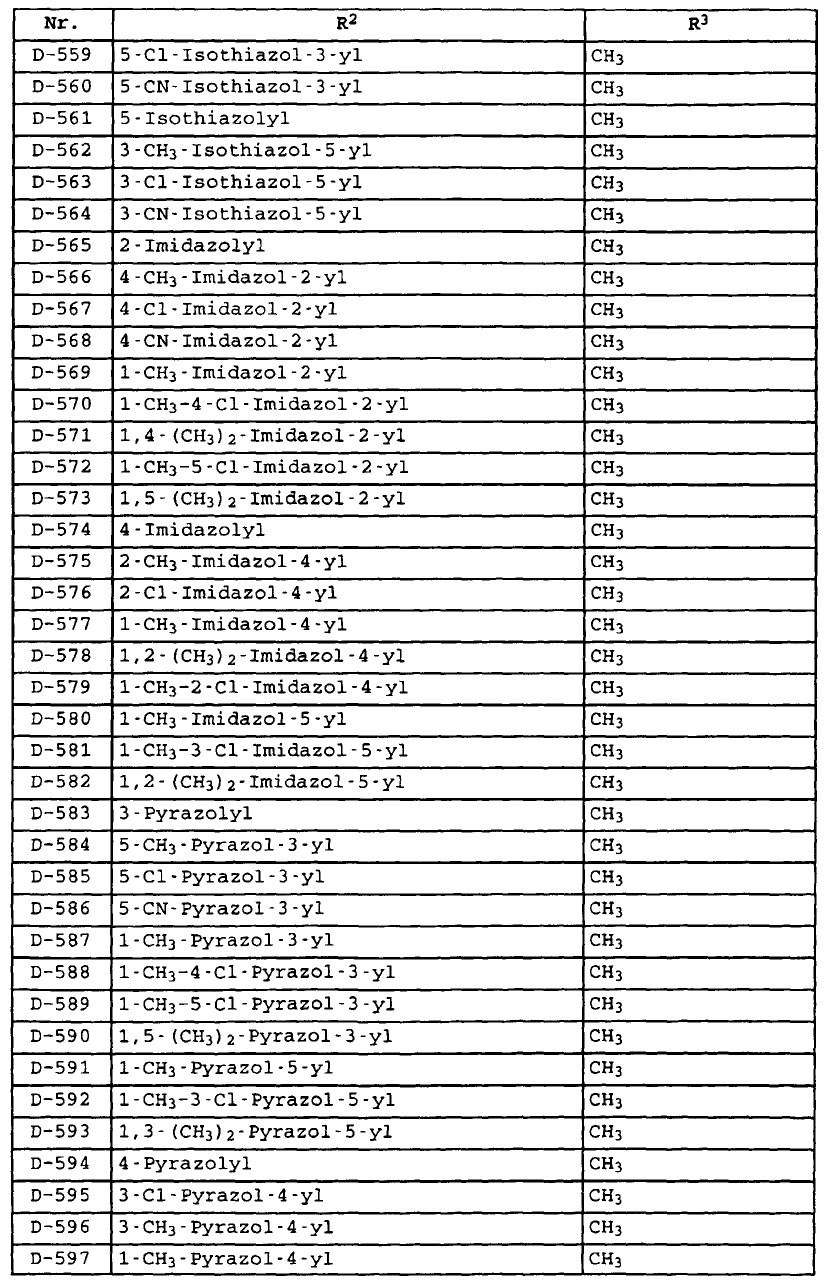

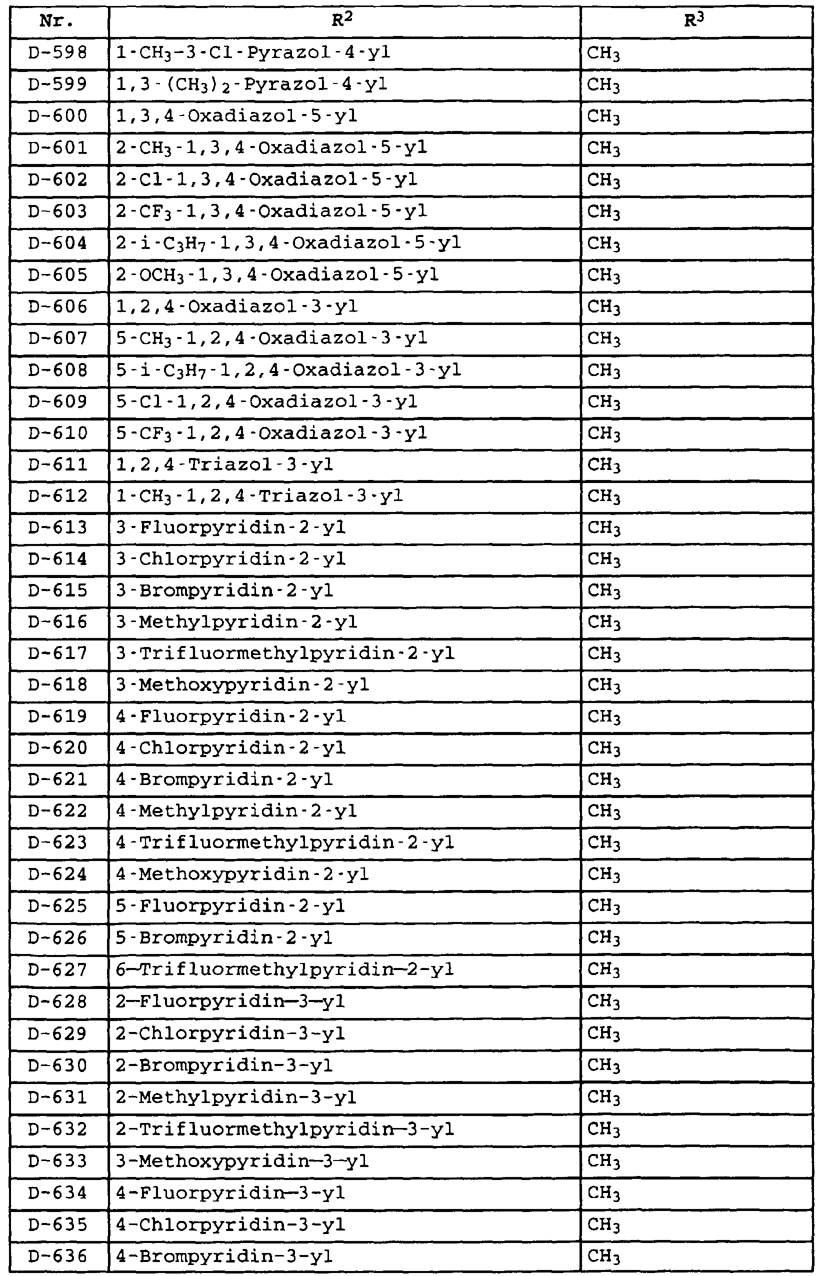

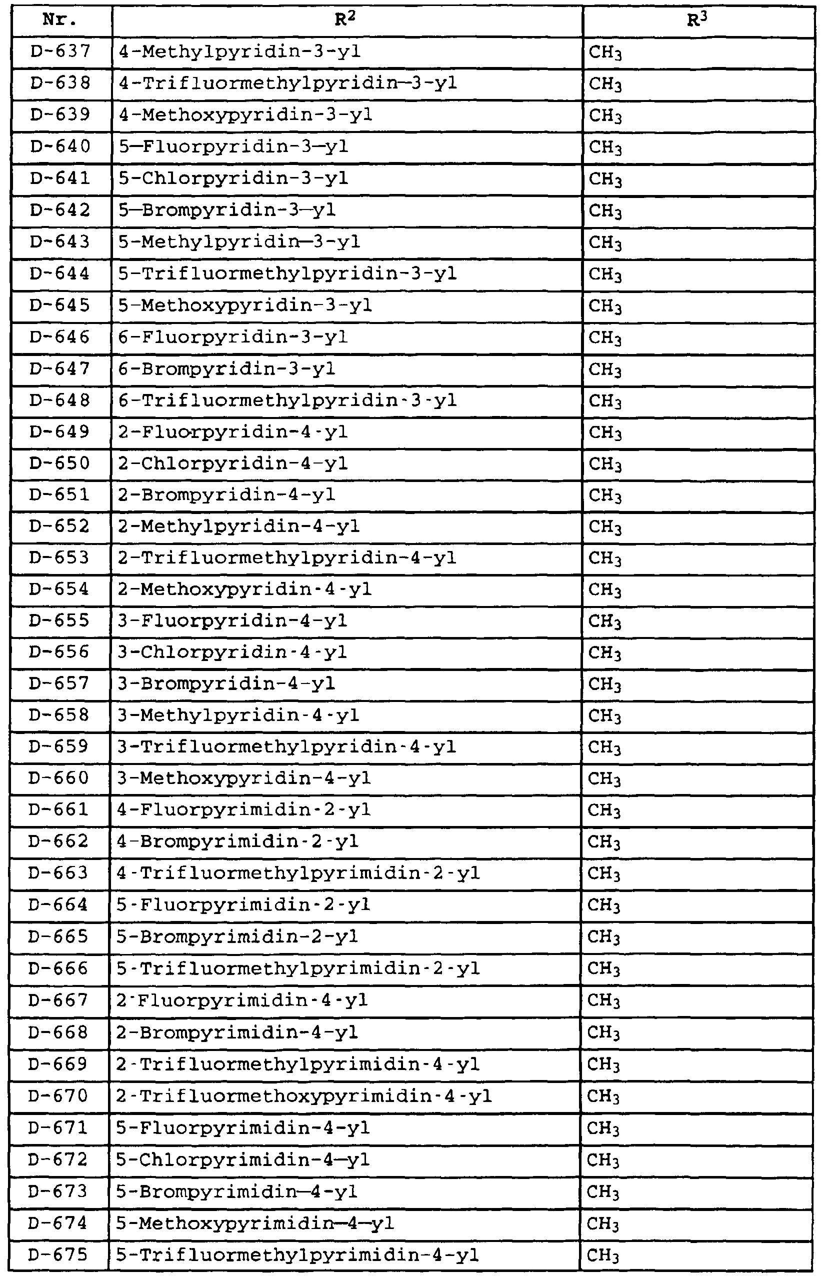

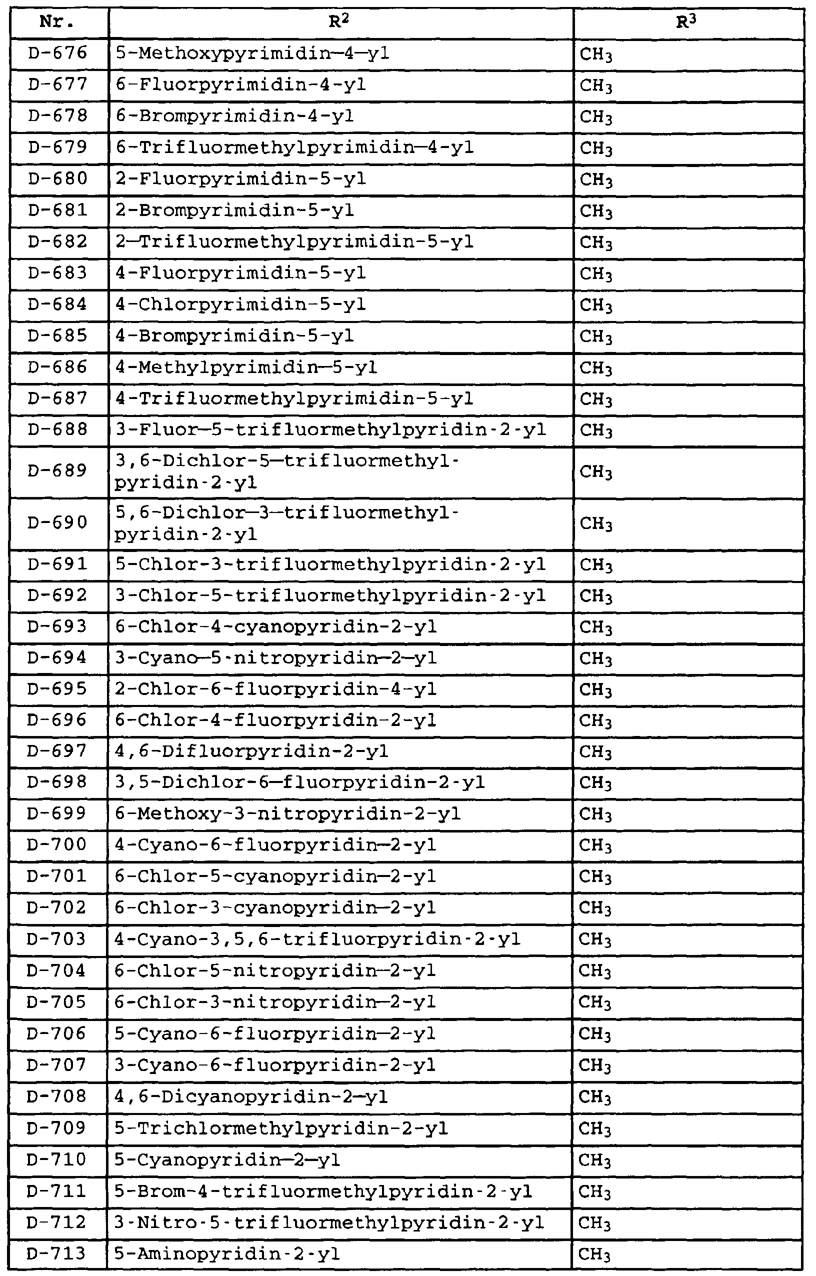

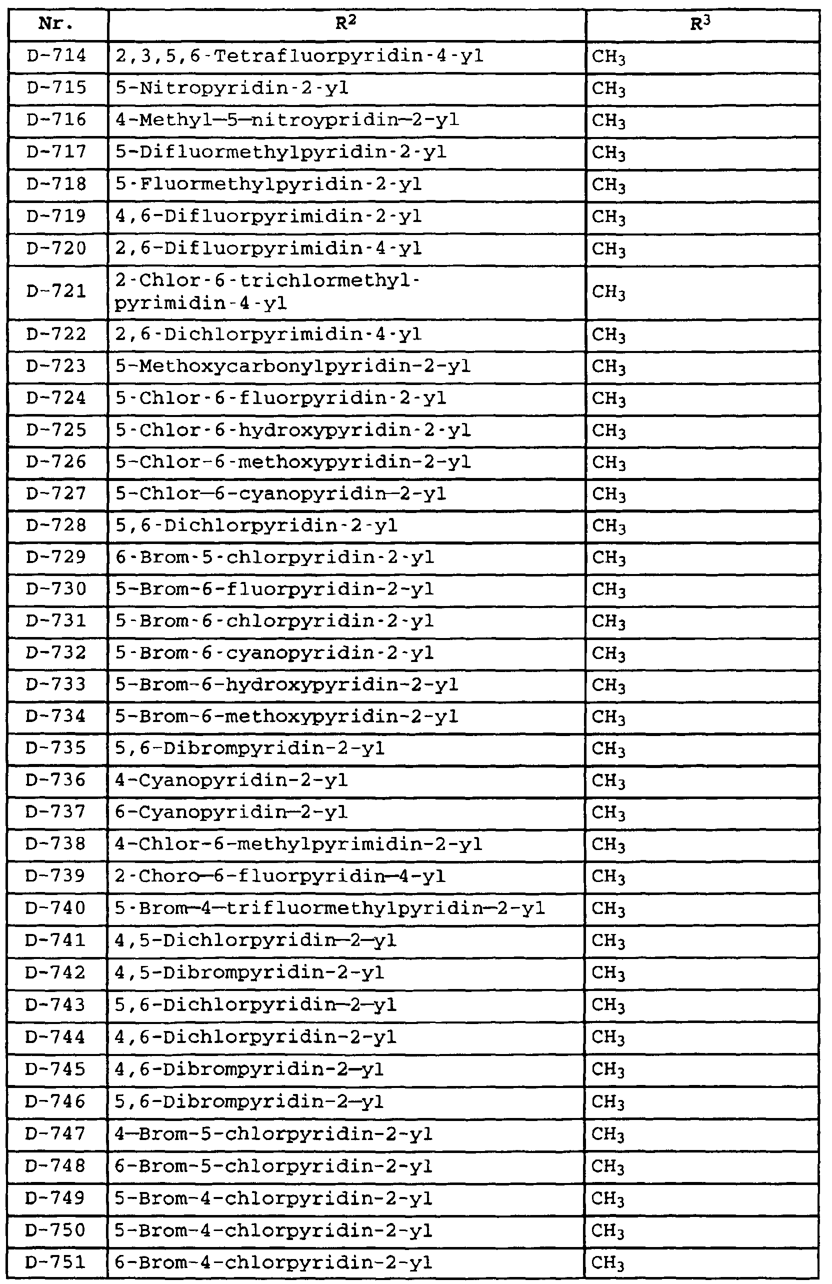

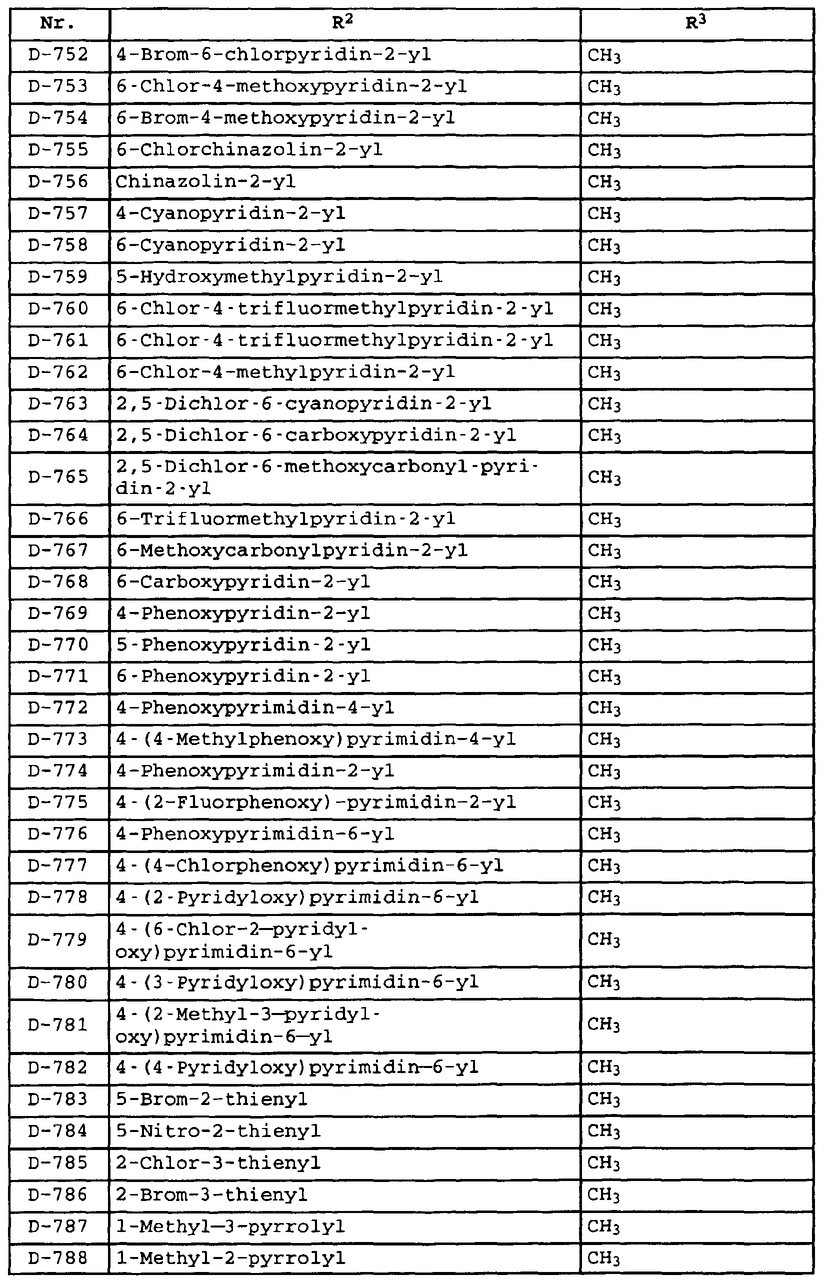

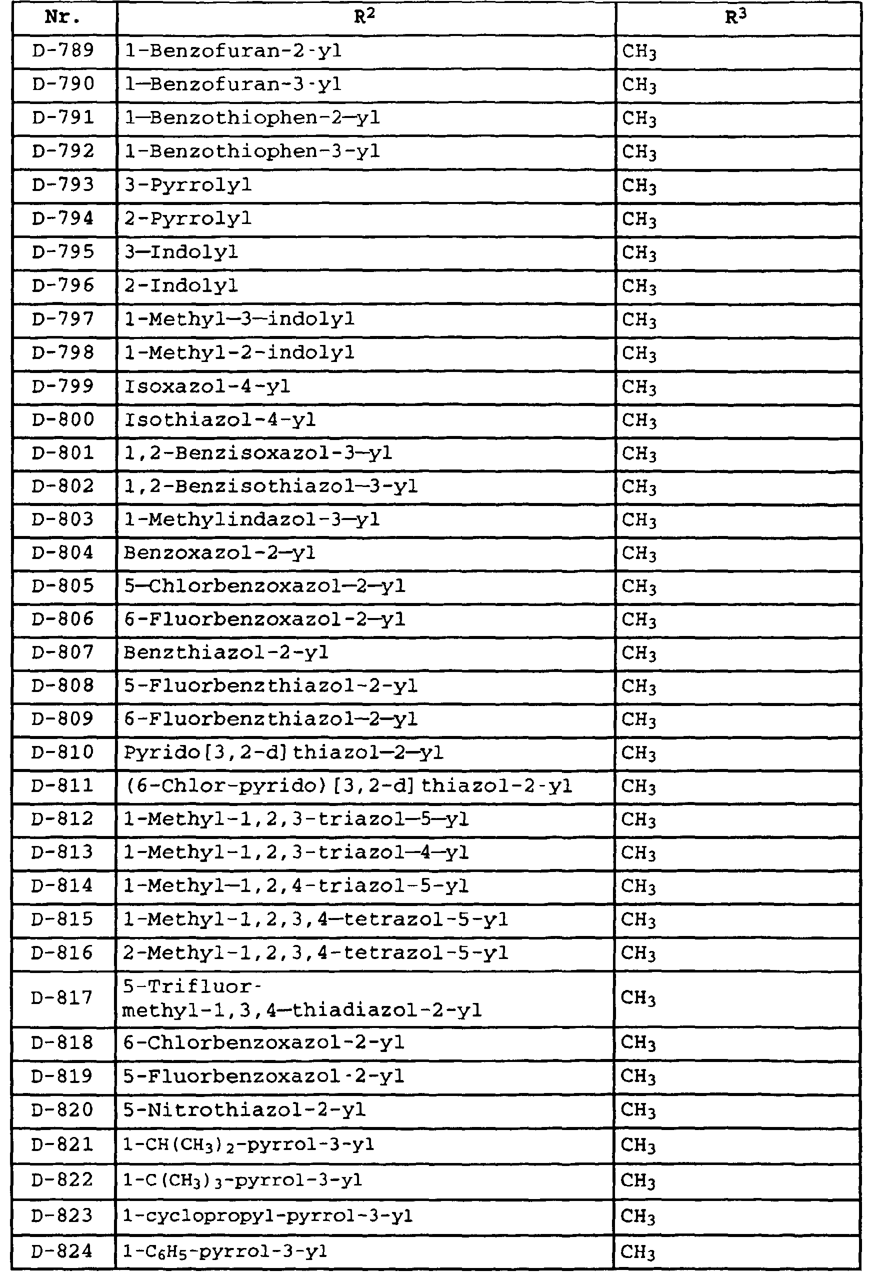

Die Verbindungen I eignen sich als Fungizide. Sie zeichnen sich durch eine hervorragende Wirksamkeit gegen ein breites Spektrum von pflanzenpathogenen Pilzen, insbesondere aus der Klasse der *Ascomyceten, Deuteromyceten, Phycomyceten* und *Basidiomyceten*, aus. Sie sind zum Teil systemisch wirksam und können im Pflanzenschutz als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Bananen, Baumwolle, Soja, Kaffee, Zuckerrohr, Wein, Obst- und Zierpflanzen und Gemüsepflanzen wie Gurken, Bohnen, Tomaten, Kartoffeln und Kürbisgewächsen, sowie an den Samen dieser Pflanzen.

Speziell eignen sie sich zur Bekämpfung folgender Pflanzenkrankheiten:
- Alternaria-Arten an Gemüse und Obst,
- Botrytis cinerea (Grauschimmel) an Erdbeeren, Gemüse, Zierpflanzen und Reben,
- Cercospora arachidicola an Erdnüssen,
- Erysiphe cichoracearum und Sphaerotheca fuliginea an Kurbisgewächsen,
- Erysiphe graminis (echter Mehltau) an Getreide,
- Fusarium- und Verticillium-Arten an verschiedenen Pflanzen,
- Helminthosporium-Arten an Getreide,
- Mycosphaerella-Arten an Bananen und Erdnüssen,
- Phytophthora infestans an Kartoffeln und Tomaten,
- Plasmopara viticola an Reben,
- Podosphaera leucotricha an Äpfeln,
- Pseudocercosporella herpotrichoides an Weizen und Gerste,
- Pseudoperonospora-Arten an Hopfen und Gurken,
- Puccinia-Arten an Getreide,
- Pyricularia oryzae an Reis,
- Rhizoctonia-Arten an Baumwolle, Reis und Rasen,
- Septoria nodorum an Weizen,
- Uncinula necator an Reben,
- Ustilago-Arten an Getreide und Zuckerrohr, sowie
- Venturia-Arten (Schorf) an Äpfeln und Birnen.

Die Verbindungen I eignen sich außerdem zur Bekämpfung von Schadpilzen wie Paecilomyces variotii im Materialschutz (z.B. Holz, Papier, Dispersionen für den Anstrich, Fasern bzw. Gewebe) und im Vorratsschutz.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung kann sowohl vor als auch nach der Infektion der Materialien, Pflanzen oder Samen durch die Pilze erfolgen.

Die fungiziden Mittel enthalten im allgemeinen zwischen 0,1 und 95, vorzugsweise zwischen 0,5 und 90 Gew.-% Wirkstoff.

Die Aufwandmengen liegen bei der Anwendung im Pflanzenschutz je nach Art des gewünschten Effektes zwischen 0,01 und 2,0 kg Wirkstoff pro ha.

Bei der Saatgutbehandlung werden im allgemeinen Wirkstoffmengen von 0,001 bis 0,1 g, vorzugsweise 0,01 bis 0,05 g je Kilogramm Saatgut benötigt.

Bei der Anwendung im Material- bzw. Vorratsschutz richtet sich die Aufwandmenge an Wirkstoff nach der Art des Einsatzgebietes und des gewünschten Effekts. Übliche Aufwandmengen sind im Materialschutz beispielsweise 0,001 g bis 2 kg, vorzugsweise 0,005 g bis 1 kg Wirkstoff pro Qubikmeter behandelten Materials.

Die Verbindungen der Formel I sind außerdem geeignet, tierische Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor zur Bekämpfung tierischer Schädlinge eingesetzt werden. Insbesondere eignen sie sich zur Bekämpfung der folgenden tierischen Schädlinge:
- Insekten aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grandiosella, Earias insulana, Elasmopalpus lignosellus, Eupoecilia ambiguella, Evetria bouliana, Feltia subterranea, Galleria mellonella, Grapholitha funebrana, Grapholitha molesta, Heliothis armigera, Heliothis virescens, Heliothis zea, Hellula undalis, Hibernia defoliaria, Hyphantria cunea, Hyponomeuta malinellus, Keiferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flammea, Pectinophora gossypiella, Peridroma saucia, Phalera bucephala, Phthorimaea operculella, Phyllocnistis citrella, Pieris brassicae, Plathypena scabra, Plutella xylostella, Pseudoplusia includens, Rhyacionia frustrana, Scrobipalpula absoluta, Sitotroga cerealella, Sparganothis pilleriana, Spodoptera frugiperda, Spodoptera littoralis, Spodoptera litura, Thaumatopoea pityocampa, Tortrix viridana, Trichoplusia ni und Zeiraphera canadensis,
- Käfer (Coleoptera), z.B. Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrhynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Oulema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus und Sitophilus granaria,
- Zweiflügler (Diptera), z.B. Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossina morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea und Tipula paludosa,
- Thripse (Thysanoptera), z.B. Frankliniella fusca, Frankliniella occidentalis, Frankliniella tritici, Scirtothrips citri, Thrips oryzae, Thrips palmi und Thrips tabaci,
- Hautflügler (Hymenoptera), z.B. Athalia rosae, Atta cephalotes, Atta sexdens, Atta texana, Hoplocampa minuta, Hoplocampa testudinea, Monomorium pharaonis, Solenopsis geminata und Solenopsis invicta,
- Wanzen (Heteroptera), z.B. Acrosternum hilare, Blissus leucopterus, Cyrtopeltis notatus, Dysdercus cingulatus, Dysdercus intermedius, Eurygaster integriceps, Euschistus impictiventris, Leptoglossus phyllopus, Lygus lineolaris, Lygus pratensis, Nezara viridula, Piesma quadrata, Solubea insularis und Thyanta perditor,
- Pflanzensauger (Homoptera), z.B. Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nordmannianae, Dreyfusia piceae, Dysaphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mala, Sappaphis mali, Schizaphis graminum, Schizoneura lanuginosa, Trialeurodes vaporariorum und Viteus vitifolii,
- Termiten (Isoptera), z.B. Calotermes flavicollis, Leucotermes flavipes, Reticulitermes lucifugus und Termes natalensis,
- Geradflügler (Orthoptera), z.B. Acheta domestica, Blatta orientalis, Blattella germanica, Forficula auricularia, Gryllotalpa gryllotalpa, Locusta migratoria, Melanoplus bivittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus und Tachycines asynamorus,
- Arachnoidea wie Spinnentiere (Acarina), z.B. Amblyomma americanum, Amblyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubata, Otobius megnini, Paratetranychus pilosus, Dermanyssus gallinae, Phyllocoptruta oleivora, Polyphagotarsonemus latus, Psoroptes ovis, Rhipicephalus appendiculatus, Rhipicephalus evertsi, Sarcoptes scabiei, Tetranychus cinnabarinus, Tetranychus kanzawai, Tetranychus pacificus, Tetranychus telarius und Tetranychus urticae,
- Nematoden wie Wurzelgallennematoden, z.B. Meloidogyne hapla, Meloidogyne incognita, Meloidogyne javanica, Zysten bildende Nematoden, z.B. Globodera rostochiensis, Heterodera avenae, Heterodera glycines, Heterodera schachtii, Heterodera trifolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratylenchus curvitatus und Pratylenchus goodeyi.

Die Aufwandmenge an Wirkstoff zur Bekämpfung von tierischen Schädlingen beträgt unter Freilandbedingungen 0,1 bis 2,0, vorzugsweise 0,2 bis 1,0 kg/ha.

Die Verbindungen I können in die üblichen Formulierungen überführt werden, z.B. Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung der erfindungsgemäßen Verbindung gewährleisten.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B.Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. PolyoxyethylenFettalkohol-Ether, Alkylsulfonate und Arylsulfonate) und Dispergiermittel wie Lignin-Sulfitablaugen und Methylcellulose.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxvethylenalkylether, ethoxyliertes Polyoxypropylen, Laurylalkoholpolyglykoletheracetal, Sorbitester, Ligninsulfitablaugen und Methylcellulose in Betracht.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Dungemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,01 und 95 Gew.-%, vorzugsweise zwischen 0,1 und 90 Gew.-% des Wirkstoffs. Die Wirkstoffe werden dabei in einer Reinheit von 90% bis 100%, vorzugsweise 95% bis 100% (nach NMR-Spektrum) eingesetzt.

Beispiele für Formulierungen sind:
I. 5 Gew.-Teile einer erfindungsgemäßen Verbindung werden mit 95 Gew.-Teilen feinteiligem Kaolin innig vermischt. Man erhält auf diese Weise ein Stäubemittel, das 5 Gew.-% des Wirkstoffs enthält.
II. 30 Gew.-Teile einer erfindungsgemäßen Verbindung werden mit einer Mischung aus 92 Gew.-Teilen pulverförmigem Kieselsäuregel und8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit (Wirkstoffgehalt 23 Gew.-%).
III. 10 Gew.-Teile einer erfindungsgemäßen Verbindung werden in einer Mischung gelöst, die aus 90 Gew.-Teilen Xylol, 6 Gew.-Teilen des Anlagerungsproduktes von 8 bis 10 Mol Ethylenoxid an 1Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.-%).
IV. 20 Gew.-Teile einer erfindungsgemäßen Verbindung werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5Gew.-Teilen des Anlagerungsproduktes von 40 Mol Ethylenoxid an 1 Mol Ricinusöl besteht (Wirkstoffgehalt 16 Gew.-%).
V. 80 Gew.-Teile einer erfindungsgemäßen Verbindung werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (Wirkstoffgehalt 80 Gew.-%).
VI. Man vermischt 90 Gew.-Teile einer erfindungsgemäßen Verbindung mit 10 Gew.-Teilen N-Methyl-α-pyrrolidon und erhält eine Lösung, die zur Anwendung in Form kleinster Tropfen geeignet ist (Wirkstoffgehalt 90 Gew.-%).
VII. 20 Gew.-Teile einer erfindungsgemäßen Verbindung werden in einer Mischung gelöst, die aus 40 Gew.-Teilen Cyclohexanon, 30Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.-% des Wirkstoffs enthält.
VIII. 20 Gew.-Teile einer erfindungsgemäßen Verbindung werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20000 Gew.-Teilen Wasser erhält man eine Spritzbrühe, die 0,1 Gew.-% des Wirkstoffs enthält.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergieroder Emulgiermittel und eventuell Lösungsmittel oder Öl bestehende Konzentrate hergestellt werden, die zur Verdünnung mit Wasser geeignet sind.

Die Wirkstoffkonzentrationen in den anwendungsfertigen Zubereitungen können in größeren Bereichen variiert werden. Im allgemeinen liegen sie zwischen 0,0001 und 10%, vorzugsweise zwischen 0,01 und 1%.

Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Die erfindungsgemäßen Mittel können in der Apwendungsform als Fungizide auch zusammen mit anderen Wirkstoffen vorliegen, der z.B. mit Herbiziden, Insektiziden, Wachstumsregulatoren, Fungiziden oder auch mit Düngemitteln. Beim Vermischen der Verbindungen I bzw. der sie enthaltenden Mittel in der Anwendungsform als Fungizide mit anderen Fungiziden erhält man in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
- Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zinkethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylenbis-dithiocarbamat), N,N'-Polypropylenbis-(thiocarbamoyl)disulfid;
- Nitroderivate, wie Dinitro-(1-methylheptyl)-phenylcrotonat, 2-sec-Butyl-4,6-dinitrophenyl-3,3-dimethylacrylat, 2-sec-Butyl-4,6-dinitrophenyl-isopropylcarbonat, 5-Nitro-isophthalsäure-di-isopropylester;
- heterocyclische Substanzen, wie 2-Heptadecyl-2-imidazolin-acetat, 2,4-Dichlor-6-(o-chloranilino)-s-triazin, O,O-Diethylphthalimidophosphonothioat, 5-Amino-1-[bis-(dimethylamino)-phosphinyl]-3-phenyl-1,2,4-triazol, 2,3-Dicyano-1,4-dithioanthrachinon, 2-Thio-1,3-dithiolo[4,5-b]chinoxalin, 1-(Butylcarbamoyl)-2-benzimidazol-carbaminsäuremethylester, 2-Methoxycarbonylamino-benzimidazol, 2-(Furyl-(2))-benzimidazol, 2-(Thiazolyl-(4))-benzimidazol, N-(1,1,2,2-Tetra-chlorethylthio)-tetrahydrophthalimid, N-Trichlormethylthio-tetrahydrophthalimid, N-Trichlormethylthio-phthalimid,
- N-Dichlorfluormethylthio-N',N'-dimethyl-N-phenyl-schwefelsaurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methylfuran-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-Iod-benzoesäure-anilid, H-Formyl-N-morpholin-2,2,2-trichlorethylacetal, Piperazin-1,4-diylbis-1-(2,2,2-trichlorethyl)-formamid, 1-(3,4-Dichloranilino)-1-formylamino-2,2,2-trichlorethan, 2,6-Dimethyl-N-tridecyl-morpholin bzw. dessen Salze, 2,6-Dimethyl-N-cyclododecyl-morpholin bzw. dessen Salze, N-[3-(p-tert.-Butylphenyl)-2-methylpropyl]-cis-2,6-dimethyl-morpholin, N-[3-(p-tert.-Butylphenyl2-methylpropyl]-piperidin, 1-[2-(2,4-Dichlorphenyl)-4-ethyl-1,3-dioxolan-2-yl-ethyl]-1H-1,2,4-triazol, 1-[2-(2,4-Dichlorphenyl)-4-n-propyl-1,3-dioxolan-2-yl-ethyl]-1H-1,2,4-triazol, N-(n-Propyl)-N-(2,4,6-trichlorphenoxyethyl)-N'-imidazol-yl-harnstoff, 1-(4-Chlorphenoxy)-3,3-dimethyl-1-(1H-1,2,4-triazol-1-yl)-2-butanon, 1-(4-Chlorphenoxy)-3,3-dimethyl-1-(1H-1,2,4-triazol-1-yl)-2-butanol, (2RS,3RS)-1-[3-(2-Chlorphenyl)-2-(4-fluorphenyl)-oxiran-2-ylmethyl]-1H-1,2,4-triazol, α-(2-Chlorphenyl)-α-(4-chlorphenyl)-5-pyrimidin-methanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol,
- Strobilurine wie Methyl-E-methoxyimino-[α-(o-tolyloxy)-o-tolyl]acetat, Methyl-E-2-{2-[6-(2-cyanophenoxy)-pyrimidin-4-yl-oxy]-phenyl}-3-methoxyacrylat, Methyl-E-methoxyimino-[α-(2-phenoxyphenyl)]-acetamid, Methyl-E-methoxyimino-[α-(2,5-dimethylphenoxy)-o-tolyl]-acetamid,
- Anilinopyrimidine wie N-(4,6-Dimethylpyrimidin-2-yl)-anilin, N-[4-Methyl-6-(1-propinyl)-pyrimidin-2-yl]-anilin, N-[4-Methyl-6-cyclopropyl-pyrimidin-2-yl]-anilin,
- Phenylpyrrole wie 4-(2,2-Difluor-1,3-benzodioxol-4-yl)-pyrrol-3-carbonitril,
- Zimtsäureamide wie 3-(4-Chlorphenyl)-3-(3,4-dimethoxyphenyl)-acrylsäuremorpholid,
- sowie verschiedene Fungizide, wie Dodecylguanidinacetat, 3-[3-(3,5-Dimethyl-2-oxycyclohexyl)-2-hydroxyethyl]-glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alanin-methyl- ester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-dioxo-1,3-oxazolidin, 3-[3,5-Dichlorphenyl(-5-methyl-5-methoxymethy]-1,3-oxazolidin- 2,4-dion, 3-(3,5-Dichlorphenyl)-1-isopropylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)-pentyl]-1H-1,2,4-triazol, 2,4-Difluor-α-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-1,2,4-triazol.

### Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in den anschließenden Tabellen mit physikalischen Angaben aufgeführt.

### Beispiel 1 Herstellung von N-Methyl-5-[2-(o-tolyloxymethylen)phenyl]-1,3-oxazolidin-2,4-dion

Zu einer Lösung von 5 g (17 mmol) α-Hydroxy-α-2-(o-tolyloxymethylen)-phenyl-essigsäuremethylester [vgl. WO-A 96/07,633] in 80 ml wasserfreiem Toluol wurden bei etwa 70°C eine Lösung von 1,8 g (6 mmol) Triphosgen in 15 ml wasserfreiem Toluol zugetropft. Die Lösung wurde noch drei Std. bei 70°C und 14 Std. bei etwa 20-25°C gerührt, dann wurde das Lösungsmittel bei etwa 250 mbar abdestilliert. Zu der Lösung des Rückstandes in 40 ml wasserfreiem Tetrahydrofuran (THF) wurden bei etwa 20-25°C 8 ml einer 2 N Methylamin-Lösung in THF zugetropft. Die Lösung wurde auf Eis gegossen, dann mit Methyl-tert.-Butylether extrahiert. Aus den organischen Phasen wurde nach Trocknen und Abdestillieren des Lösungsmittels 3,0 g der Titelverbindung als hellgelbes Öl erhalten.
¹H-NMR (CDCl₃, δ in ppm): 2,3 (s, 3H); 3,3 (s, 3H), 5,2 (s, 2H); 5,7 (s, 1H); 6,8 - 8,5 (m, br, 8H).

### Beispiele für die Wirkung gegen Schadpilze

Die fungizide Wirkung der Verbindungen der allgemeinen Formel I ließ sich durch die folgenden Versuche zeigen:

Die Wirkstoffe wurden getrennt oder gemeinsam als 10%ige Emulsion in einem Gemisch aus 70 Gew.-% Cyclohexanon, 20 Gew.-% Nekanil® LN (Lutensol® AP6, Netzmittel mit Emulgier- und Dispergiervirkung auf der Basis ethoxylierter Alkylphenole) und 10 Gew.-% Wettol® EM (nichtionischer Emulgator auf der Basis von ethoxyliertem Ricinusöl) aufbereitet und entsprechend der gewünschten Konzentration mit Wasser verdünnt.

### Anwendungsbeispiel 1 - Wirksamkeit gegen Weizenmehltau

Blätter von in Töpfen gewachsenen Weizenkeimlingen der Sorte "Frühgold" wurden mit wäßriger Wirkstoffaufbereitung, die aus einer Stammlösung bestehend aus 10 % Wirkstoff, 63 % Cyclohexanon und 27 % Emulgiermittel angesetzt wurde, bis zur Tropfnässe besprüht und 24 Stunden nach dem Antrocknen des Spritzbelages mit Sporen des Weizenmehltaus (Erysiphe graminis forma specialis tritici) bestäubt. Die Versuchspflanzen wurden anschließend im Gewächshaus bei Temperaturen zwischen 20 und 24°C und 60 bis 90 % relativer Luftfeuchtigkeit aufgestellt. Nach 7 Tagen wurde das Ausmaß der Mehltauentwicklung visuell in % Befall der gesamten Blattfläche ermittelt.

In diesem Test zeigten die mit 250 ppm der Verbindung aus Beispiel 1 behandelten Pflanzen 15 % Befall, während die unbehandelten zu 80 % befallen waren.

### Beispiele für die Wirkung gegen tierische Schädlinge

Die Wirkung der Verbindungen der allgemeinen Formel I gegen tierische Schädlinge ließ sich durch folgende Versuche zeigen:

Die Wirkstoffe wurden
a. als 0,1%-ige Lösung in Aceton oder
b. als 10%-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Wettol® EM (nichtionischer Emulgator auf der Basis von ethoxyliertem Ricinusöl)
aufbereitet und entsprechend der gewünschten Konzentration mit Aceton im Fall von a. bzw. mit Wasser im Fall von b. verdünnt.

Nach Abschluß der Versuche wurde die jeweils niedrigste Konzentration ermittelt, bei der die Verbindungen im Vergleich zu unbehandelten Kontrollversuchen noch eine 80 bis 100%-ige Hemmung bzw. Mortalität hervorriefen (Wirkschwelle bzw. Minimalkonzentration).

## Patentansprüche

1. Heterocyclylsubstituierte Phenylverbindungen der Formel I, 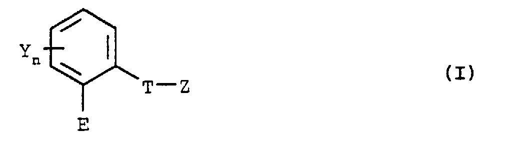 in der die Substituenten folgende Bedeutung haben:
Y Halogen, C₁-C₄-Alkyl, C₁-C₄-Halogenalkyl oder C₁-C₄-Alkoxy;
n 0, 1 oder 2, wobei die Reste Y verschieden sein können, wenn n = 2 ist;
E eine Gruppe A oder B, 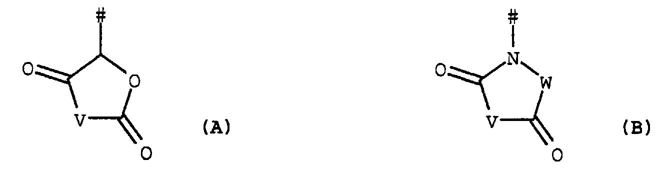 wobei # die Bindung mit dem Phenylring kennzeichnet;
V Sauerstoff oder N-R^{α}, und
G N-R^{β}, wobei
R^{α} C₁-C₄-Alkyl, C₁-C₄-Halogenalkyl, C₃-C₆-Cycloalkyl oder ggf. subst. Arylmethylen;
R^{β} Wasserstoff oder einen Rest R^{α};
T Sauerstoff oder Oxymethylen;
Z eine Gruppe X, N=CWR¹ oder N=C(R¹)-C(R²)=NOR³;
X ggf. subst. Heterocyclyl, ggf. subst. Aryl, ggf. subst. Hetaryl, ggf. subst. Arylmethylen oder ggf. subst. Hetarylmethylen;
W ggf. subst. C₁-C₆-Alkyl, ggf. subst. C₂-C₆-Alkenyl, ggf. subst. C₂-C₆-Alkinyl, ggf. subst. C₃-C₆-Cycloalkyl, ggf. subst. C₃-C₆-Cycloalkenyl, ggf. subst. Heterocyclyl, ggf. subst. Aryl oder ggf. subst. Hetaryl;
R¹ Wasserstoff, Cyano, C₁-C₄-Alkyl, C₁-C₄-Halogenalkyl, C₁-C₄-Alkoxy, C₁-C₄-Alkoxy-C₁-C₄-alkyl, C₃-C₆-Cycloalkyl;
R² Wasserstoff, Cyano, Halogen, C(R^{d})=NOR³ oder W, OW, SW oder NR^{c}W, wobei R^{c} Wasserstoff, C₁-C₆-Alkyl, C₂-C₆-Alkenyl oder C₂-C₆-Alkinyl und R^{d} Wasserstoff oder C₁-C₆-Alkyl;
R³ Wasserstoff, ggf. subst. C₁-C₆-Alkyl, ggf. subst. C₂-C₆-Alkenyl oder ggf. subst. C₂-C₆-Alkinyl bedeuten.

2. Verfahren zur Herstellung von Verbindungen der Formel IAa, 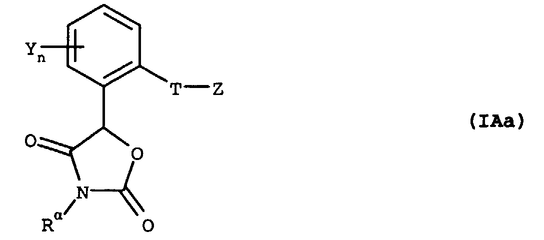 in denen R^{α}, Y, n, T und Z die in Anspruch 1 gegebene Bedeutung haben,
dadurch gekennzeichnet, daß man α-Hydroxy-α-Phenylessigsäurealkylester der Formel IIA, 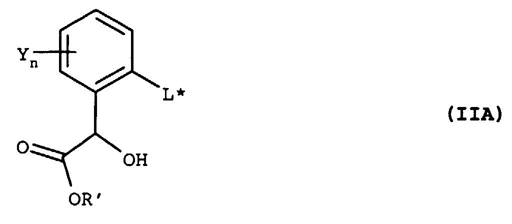 in der
L* CH₂L' oder eine Gruppe L', wobei
L' eine nucleophil abspaltbare Gruppe und
R' C₁-C₄-Alkyl bedeutet,
mit Phosgen oder einem Phosgenäquivalent zu Verbindungen der Formel IIIA 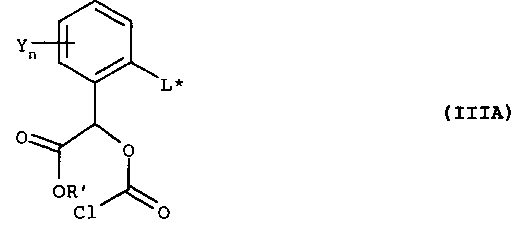 acyliert, die durch Umsetzung mit primären Aminen der Formel IVa 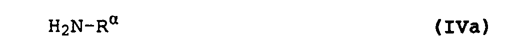 und gegebenenfalls, sofern die Cyclisierung zu Oxazolidindionen der Formel VAa 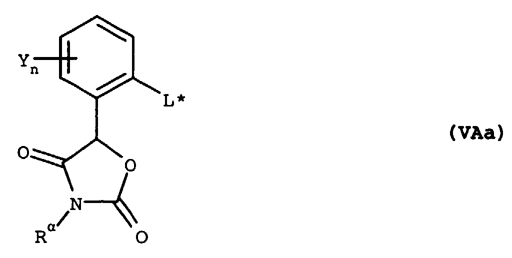 nicht von selbst abläuft, Verseifung des entstandenen Esteramids der Formel VIA 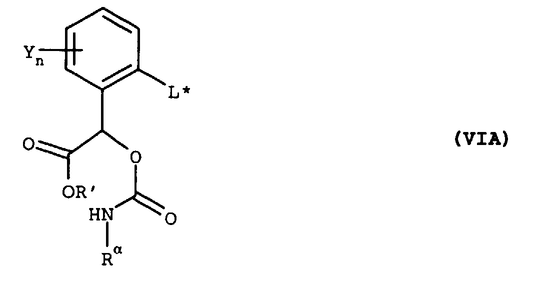 und anschließender Säurebehandlung cyclisiert werden und VAa durch Umsetzung mit Verbindungen Z-OH in IAa überführt werden.

3. Verfahren zur Herstellung von Triazolidindionen der Formel IBa, 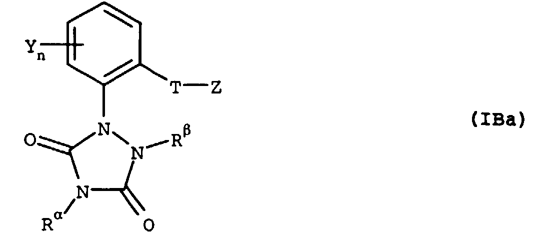 dadurch gekennzeichnet, daß man Phenylhydrazine der Formel IIB 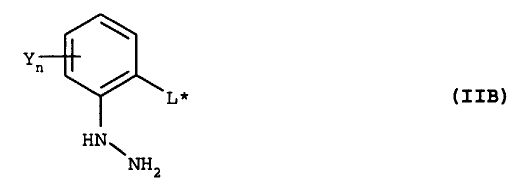 durch Umsetzung mit Chlorameisensäurealkylestern in Carbamate der Formel IIIB, 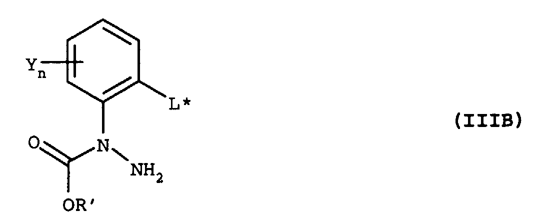 in der R' für C₁-C₄-Alkyl steht, überführt, die mit Phosgen oder einem Phosgenäquivalent zu Verbindungen der Formel VB 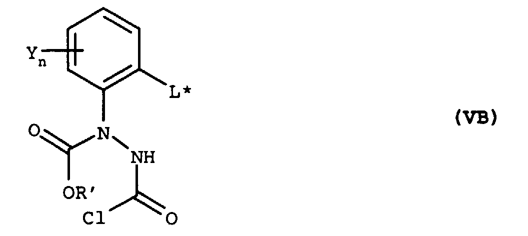 acyliert, die durch Umsetzung mit primären Aminen der Formel IVa nach Anspruch 3 und gegebenenfalls, sofern die Cyclisierung zu Triazolidindionen der Formel VIBa 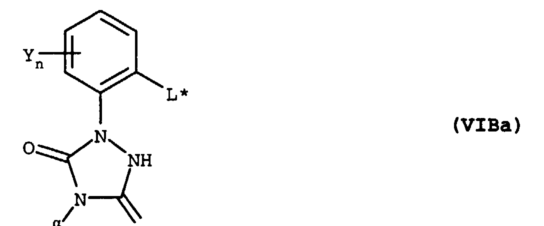 nicht von selbst abläuft, Verseifung des entstandenen Esteramids der Formel VIIB 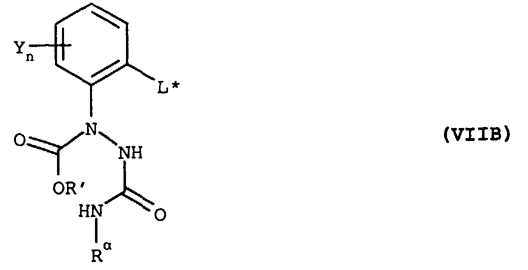 und anschließender Säurebehandlung cyclisiert und, sofern R^{β} in Formel IBa nicht für Wasserstoff steht, VIBa zu Verbindungen VIBa', 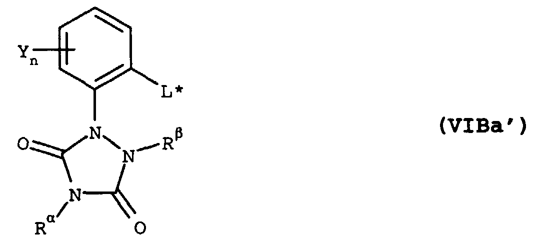 in denen R^{β} nicht für Wasserstoff steht, alkyliert und
VIBa oder VIBa' durch Umsetzung mit Verbindungen Z-OH in IBa überführt werden.

4. Zwischenprodukte der Formel 1, 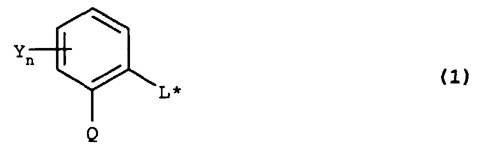 in der
Q eine Gruppe A nach Anspruch 1 oder
CH(COOR')OCOCl, CH(COOH)OCOCl, CH(COOR')OCONHR^{α} oder CH(COOH)OCONHR^{α}, wobei
R' C₁-C₄-Alkyl bedeutet;
L* Hydroxy, CH₃, CH₂L' oder eine Gruppe L', wobei
L' eine nucleophil abspaltbare Gruppe bedeutet und
Y und n die in Anspruch 1 gegebene Bedeutung haben.

5. Zwischenprodukte der Formel 2, 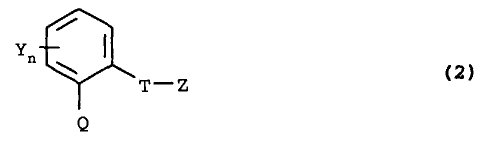 in der
Q N(NH₂)COOR', N(NHCOCl)COOR' oder N(NHCONHR^{α})COOR' bedeuten, wobei
R' C₁-C₄-Alkyl bedeutet und
R^{α}, Y, n, T und Z die in Anspruch 1 gegebene Bedeutung haben.

6. Zwischenprodukte der Formel VIBa, 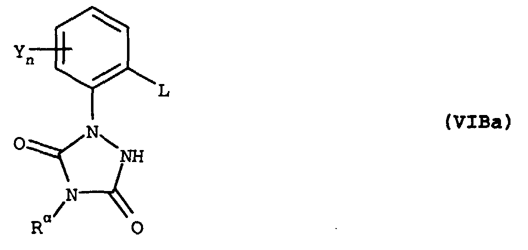 in der
L CH₃, CH₂L', Nitro oder Alkylsulfonat oder Arylsulfonat, wobei
L' eine nucleophil abspaltbare Gruppe bedeutet und
Y, n und R^{α} die in Anspruch 1 gegebene Bedeutung haben.

7. Zur Bekämpfung von tierischen Schädlingen oder Schadpilzen geeignetes Mittel, enthaltend einen festen oder flüssigen Trägerstoff und eine Verbindung der Formel I gemaß Anspruch 1.

8. Verwendung der Verbindungen I gemäß Anspruch 1 zur Herstellung eines zur Bekämpfung von tierischen Schädlingen oder Schadpilzen geeigneten Mittels.

9. Verfahren zur Bekämpfung von Schadpilzen, dadurch gekennzeichnet, daß man die Pilze oder die vor Pilzbefall zu schützenden Materialien, Pflanzen, den Boden oder Saatgüter mit einer wirksamen Menge einer Verbindung der Formel I gemäß Anspruch 1 behandelt.

10. Verfahren zur Bekämpfung von tierischen Schädlingen, dadurch gekennzeichnet, daß man die tierischen Schädlinge oder die vor ihnen zu schützenden Materialien, Pflanzen, den Boden oder Saatgüter mit einer wirksamen Menge einer Verbindung der allgemeinen Formel I gemäß Anspruch 1 behandelt.
